# FASCICULE DE BREVET EUROPEEN

(11) **EP 0 348 271 B1**
(45) Date de publication et mention de la délivrance du brevet: **30.03.1994**
(21) Numéro de dépôt: 89401641.9
(22) Date de dépôt: 13.06.1989
(51) Int. Cl.: A61M 5/14, A61B 5/07, A61B 5/05, A61N 1/36, A61N 1/38

(54) **Dispositif de protection contre les affections liées au sang, notamment thromboses, embolies, spasmes vasculaires, hémorragies, hémopathies et la présence d'éléments anormaux dans le sang**
Schutzeinrichtung gegen gesundheitliche Schädigung des Blutes, besonders Thrombosen, Embolien, Gefässpasmen, Hämorrhagien, Hämopathien und das Vorhandensein von anormalen Elementen im Blut
Protection means against ailment of the blood, particularly thrombosis, embolism, vascular spasm, haemorrhage, haemopathy and the presence of abnormal elements in the blood

(30) Priorité: 13.06.1988 FR 8807852; 14.10.1988 FR 8813523
(43) Date de publication de la demande: 27.12.1989
(73) Titulaire: Zacouto, Fred, F-75015 Paris (FR)
(72) Inventeur: Zacouto, Fred, F-75015 Paris (FR)
(74) Mandataire: Bernasconi, Jean

(56) Documents cités:
- EP-A- 0 009 255
- DE-A- 2 204 433
- DE-B- 2 758 039
- FR-A- 1 514 319
- FR-A- 2 255 872
- FR-A- 2 257 312
- FR-A- 2 403 775
- FR-A- 2 596 950
- US-A- 3 805 795
- US-A- 4 146 029
- US-A- 4 754 753
- US-A- 4 787 389
- BIOLOGIE MEDICALE vol. 60, no. 1, 1971, pages 14-34; F. ZACOUTO et al.: "Sur l'évolution des circulations assistées" * pages 18,19 *

## Description

La présente invention décrit un dispositif implantable chez l'homme et les mammifères capable d'analyser automatiquement la constitution biologique et chimique du sang et/ou son hémodynamique, et d'intervenir sélectivement en cas de constatation d'anomalies, ainsi que les procédés d'analyse et de traitement mis en oeuvre.

L'invention a trait plus précisément à un dispositif implanté de protection contre les affections liées au sang. Par affections liées au sang, on entend non seulement les affections propres au sang et de ses divers constituants, mais également les affections du coeur, des artères ou des veines qui, quelles que soient les origines, ont des effets sur l'état local ou général de la circulation.

Un but de l'invention est notamment de fournir un dispositif de protection contre les thromboses, embolies, et rétrécissements athéromateux, infectieux ou spastiques des vaisseaux et cavités sanguines, et notamment, les artères irriguant le myocarde ou le cerveau.

L'invention a également pour but de lutter contre les hémorragies vasculaires lorsque celles-ci résultent d'un trouble de la coagulation sanguine ou d'une hypertension artérielle excessive ou d'une perméabilité anormale des petits vaisseaux ou des endothéliums des capillaires.

L'invention a également pour but de surveiller sélectivement dans les vaisseaux sanguins certaines compositions et présences de molécules chimiques, biochimiques et ioniques ainsi que les numération et formule sanguines, fonctions qui permettent la détection précoce et le traitement immédiat et constamment contrôler des hémopathies même autonomes, et de certains cancers dont l'apparition et le traitement peuvent être observés par l'apparition de molécules nouvelles ou par une concentration différente de certaines molécules existantes.

L'invention a également pour but de protéger contre les pollutions de l'air, de l'eau et des aliments en surveillant automatiquement dans le sang et dans la moelle osseuse, les molécules relatives à l'agent polluant, l'hématopoïèse et les numération et formule sanguines.

L'invention est notamment destinée à amplifier et compléter le dispositif de protection du coeur faisant l'objet des brevets Zacouto français FR-A- 2.082.703 et des brevets US-A-3.857.399 et 4.052.991.

La protection de l'organisme humain contre les thromboses, spasmes et embolies artérielles et veineuses est souvent peu efficace, coûteuse et n'est pas exempte d'effets secondaires.

En dehors des traitements médicaux permanents, notamment anticoagulants chimiques ou biochimiques à doses invariables entre les intervalles de contrôle de l'hémostase, qui sont longs, ou des traitements chirurgicaux de réparation, la protection contre les thromboses consiste, d'une part, à tenter de traiter les thromboses et embolies intervenues, par administration immédiate de très courte durée et, de préférence in situ au voisinage du coagulat sanguin, de thrombolytiques tels que streptokinase ou urokinase et dérivés, activateurs tissulaires du plasminogène (TPA) et dérivés.

Les traitements intravasculaires doivent être pratiqués le plus rapidement possible pour diminuer la dose nécessaire et augmenter l'efficacité et la bonne tolérance contre les risques hémorragiques.

Il est préférable de pouvoir simultanément protéger le coeur contre les tachycardies, les bradyarythmies et/ou tachyarythmies graves, les fibrillations et les insuffisances et défaillances mécaniques du coeur.

Il existe déjà différents type de dispositifs de stimulation électrique du coeur contre les bradycardies et les tachycardies, notamment les dispositifs décrits dans les brevets US-A-3 857 339 et 4 052 991 de l'inventeur qui décrivent un pacemaker, à large utilisation clinique actuelle, capable de protéger le coeur à la fois contre les bradycardies et contre les tachycardies et désigné ci-après "stimulateur orthorythmique" en abréviation "POR".

On connaît également des dispositifs de défibrillation. L'inventeur a déjà déposé un brevet français n^{o} FR-A-1 237 702 le 11 juillet 1953 décrivant un dispositif externe fixé sur une ceinture thoracique, capable, en cas de syncope, de détecter la nature de la syncope, arrêt ou fibrillation ventriculaire et, suivant le cas, de déclencher automatiquement soit un stimulateur cardiaque à la demande, soit un défibrillateur par choc électrique externe ou interne capable de détecter également l'efficacité de l'intervention automatique déclenchée et de réagir automatiquement selon le résultat clinique constaté. Les appareils selon cette invention furent fabriqués sous la direction de l'inventeur de 1961 à 1965 par la société Cotelec, filiale de Thompson à Paris, et vendus à plus de 100 hopitaux français et allemands. Il a ensuite été développé, par M. Miroyski, un défibrillateur implantable faisant l'objet du brevet US-Re-27 757 (1970). Voir également le brevet français de l'inventeur FR-A-74 01383.

Ces dispositifs de défibrillation interne implantables avec électrodes épi- et/ou intracardiaques, actuellement commercialisés, présentent un certain nombre d'inconvénients. Ils sont extrêmement onéreux et ont un délai d'attente, avant intervention, de 20 sec. environ et une durée de vie réduite, n'étant guère fiables au-delà de deux années. Ils sont capables d'inhiber l'envoi d'un choc électrique lorsque le coeur rétablit son fonctionnement normal quelques secondes avant la fin du délai d'attente. Ils sont incapables de réduire les tachycardies et les bradycardies, de même qu'un arrêt cardiaque non fibrillatoire. En outre, la nécessité de disposer d'une énergie de l'ordre de 30 joules pour chaque choc, entraîne un encombrement et un poids importants.

D'autres moyens de détecter l'activité mécanique cardiaque associés à un défibrillateur ont été décrits dans le brevet EP-A-0 009 255. Ce dispositif détecte la variation du volume sanguin cardiaque par mesure de la modification d'impédance entre les électrodes dans un ventricule.

On a déjà proposé, par exemple dans le brevet US-A-4 146 029, des dispositifs de traitement automatique implantables comprenant un ou plusieurs capteurs d'un paramètre physiologique dans le corps, des moyens électroniques de traitement de l'information provenant du capteur, et des moyens de délivrance d'un médicament dans le corps, par exemple à partir d'une pompe implantée, ces moyens de délivrance étant contrôlés par les moyens de traitement électronique en réponse à la détection, par exemple, lorsque la valeur du paramètre détecté franchit un seuil. Ces dispositifs n'étaient cependant pas capables de détecter des paramètres biologiques susceptibles de précéder ou d'accompagner une affection liée à la coagulation du sang, tels que thromboses, embolies, coagulopathies, hémorragies, hémopathies et présence d'éléments cellulaires anormaux.

D'autre part si ces dispositifs, dans leur fonction propre, associée à la détection d'un paramètre biologique tel que par exemple rythme cardiaque, pH, glucose, sont capables de remédier à un désordre établi constaté par la variation du paramètre, ils ne permettent pas de prévenir un désordre dont la survenue est imminente, mais qui ne s'est pas encore produit. On comprend qu'une telle prévention est particulièrement importante dans des affections pouvant se traduire par la survenue d'un désordre brusque comme cela est le cas lorsque les facteurs de la coagulation sanguine se trouvent affectés.

Un objectif de l'invention est donc de fournir un dispositif de protection contre les thromboses, ischémies et embolies vasculaires installées ou en voie de formation, qui permette un traitement immédiat, efficace, rapide et sélectif.

Un autre objectif de l'invention est de fournir un traitement préventif à la demande, s'adaptant rapidement et automatiquement aux variations spontanées physiologique et pathologique plurijournalières de la crase sanguine et présentant des risques d'effets secondaires réduits.

Un autre objectif de l'invention est de fournir un dispositif de protection contre les thromboses des artères notamment coronaires cardiaques et artères carotides, voire cérébrales, susceptible de traiter, de préférence in situ, une thrombose ou embolie débutante notamment pulmonaire, et de prévenir son apparition. En particulier, le dispositif selon l'invention permet d'empêcher la réobstruction après pontage aorto-coronarien ou angioplastie coronarienne, carotidienne ou cérébrale endoartérielle.

Un autre objectif de l'invention est de fournir un dispositif de protection contre les coagulopathies de consommation (CIVD).

Un autre objectif de l'invention est de fournir un dispositif de protection contre les hémorragies et de moduler un traitement anticoagulant selon les variations spontanées et induites de la crase sanguine.

Un autre objectif de l'invention est d'assurer la défibrillation très rapidement après la survenue d'une fibrillation, ou d'une tachycardie très rapide ou malaise , et de préférence avant la disparition de la conscience du sujet.

Un autre objectif de l'invention est de fournir un dispositif qui n'envoie pas de décharge de défibrillation si le coeur retrouve, au dernier moment, une activité satisfaisante.

Un autre objectif de l'invention est de fournir un dispositif qui n'intervient qu'en cas de fibrillation ventriculaire ou tachycardie très grave résistante se traduisant par un arrêt ou une défaillance très grave de la fonction mécanique vitale du coeur.

Un autre objectif encore de l'invention est de fournir un dispositif qui assure, en plus de la défibrillation, une assistance mécanique respiratoire.

Un autre autre objectif de l'invention est de fournir un dispositif qui puisse alerter le sujet ou l'entourage en cas de danger et de dysfonctionnement grave du coeur et/ou de la circulation artérielle.

Un autre objectif encore de l'invention est de fournir un dispositif qui puisse préventivement intervenir et/ou alerter le sujet ou l'entourage avant la survenue effective d'une tachycardie ou d'une fibrillation ou d'une défaillance mécanique du coeur.

Un autre objectif encore de l'invention est de fournir un tel dispositif dans lequel il soit possible d'obtenir, à l'extérieur du sujet, les paramètres électriques et mécaniques des cycles ou activités cardiaques et de l'hémodynamique artérielle.

Un autre objectif de l'invention est de fournir un tel dispositif susceptible de protéger le coeur et d'assurer la circulation sanguine en cas de défibrillation inefficace ou de défaillance grave subite ou progressive de la contraction cardiaque.

L'invention a pour objet un dispositif de protection contre les affections liées à la coagulation sanguine, et notamment thromboses, embolies, coagulopathies, hémorragies, hémopathies et présence d'éléments cellulaires anormaux dans le sang, caractérisé en ce qu'il comprend :
- des premiers moyens implantables pour mesurer, en continu ou périodiquement, au moins un paramètre biologique susceptible de précéder ou d'accompagner une affection lieé à la coagulation sanguine comprenant au moins un moyen de présentation susceptible d'être placé dans une cavité ou un vaisseau sanguin ou lymphathique, pour présenter au sang, ou à la lymphe, un élément agencé pour faciliter une variation locale dudit au moins un paramètre biologique, et des moyens de capteur associés audit élément et sensibles à ladite variation,
- des seconds moyens implantables déterminant un ou plusieurs seuils auquel ledit paramètre est comparé,
- et des troisième moyens implantables sensibles aux dépassements de ce(s) seuil(s) pour délivrer, dans la circulation, une dose d'une ou plusieurs substances actives sur la coagulation.

Les agents thérapeuthiques qui peuvent être libérés peuvent être, par exemple des thrombolytiques et/ou anticoagulants et/ou vasodilatateurs et/ou béta-bloquants et/ou diurétiques et/ou inhibiteurs calciques et/ou prostaglandines PGI2 et PG12 et/ou facteurs de la coagulation.

Les paramètres peuvent être, par exemple, des valeurs mesurées de phénomènes biologiques ou bien des valeurs détectées ou calculées de vitesse ou de sens ou de décours de variations de phénomènes biologiques.

Les paramètres mesurés peuvent être des paramètres généraux, c'est-à-dire des paramètres liés à des facteurs généraux susceptibles d'entraîner dans un délai plus ou moins bref, un accident tel que thrombose, par exemple coronaire, carotidienne ou cérébrale ou embolie, par exemple pulmonaire, ou bien traduisant dans la circulation générale, les conséquences de la survenue d'une telle embolie ou thrombose, ou d'une hémorragie ou d'une hémopathie. De tels paramètres généraux peuvent être, par exemple, des paramètres liés à la coagulabilité du sang, la survenue d'une hypertension ou hypotension artérielle, la libération de facteurs susceptibles d'influer sur l'état circulatoire tels que catécholamines, hypoxie ou hypercapnie, déséquilibres ioniques et pH, surcharge en chylomicrons, glucose, créatinine, volhémies, etc.

On peut, en outre, détecter le rythme et l'amplitude respiratoires, notamment en mesurant, d'une manière connue, l'impédance électrique thoracique.

Le dispositif selon l'invention peut être prévu pour détecter de tels paramètres généraux en un endroit quelconque de la circulation, mais on peut avantageusement prévoir de disposer ces moyens de mesure dans une cavité cardiaque ou encore à proximité immédiate d'une partie d'une voie vasculaire, lymphatique, intraosseuse ou cardiaque présentant un risque ou un intérêt diagnostique particuliers.

Dans une autre forme de réalisation de l'invention, les paramètres mesurés peuvent être des paramètres locaux, les moyens de mesure étant alors disposés à proximité ou même au niveau d'une voie vasculaire sanguine ou lymphatique ou d'un carrefour présentant des risques particuliers. Ces paramètres peuvent alors, par exemple, traduire une anomalie locale résultant de l'imminence ou de la survenue d'une embolie, thrombose ou spasme artériel dans la zone surveillée, ces paramètres pouvant être, par exemple, une concentration en ions ou autres éléments libérés lors d'une ischémie tissulaire, ou encore des anomalies électriques de genèse, de propagation ou d'absorption d'un courant électrique spontané ou artificiel de mesure liées à un risque ou une survenue d'une telle ischémie, ou d'autres paramètres traduisant l'effet d'une ischémie sur un tissu, tels que par exemple, hypoxie tissulaire, modification de bruits myocardiques, variations fines de température locale, élévations de transaminases, etc.

On peut également détecter, par des mesures sur l'électrocardiogramme, des paramètres électriques relatifs aux troubles du rythme qui entraînent une diminution du débit cardiaque et coronaire, notamment en cas de troubles du rythme cardiaque tels que tachycardies ventriculaires, tachyarythmies par fibrillation auriculaire, extrasystolies ventriculaires, ou fibrillations ventriculaires. Les moyens de détection de tels troubles, par exemple décrits dans les brevets US Zacouto 3 857 399 et 4 052 991, peuvent alors provoquer, en plus du traitement électrique automatique décrit, l'administration d'anticoagulants et/ou fibrinolytiques pour diminuer ou éliminer les risques de thrombose qui sont souvent la cause ou un facteur d'aggravation de troubles du rythme.

Cependant, de préférence, le dispositif selon l'invention comportera des moyens de détection et de mesure sensibles, de façon directe ou indirecte, à la qualité de la perfusion telle que vitesse ou débit de pointe, moyenne ou minimale, débit, variations géométriques, pression de perfusion ou de contraction active de la voie vasculaire. Un paramètre préféré est constitué par une impédance électrique représentative de ces caractéristiques mais on peut également utiliser d'autres paramètres, par exemple pressions, déformations géométriques, mesures de débit, absorptions optiques (spectrométrie), fréquences de résonance propres de l'arbre artériel, vélocité de l'onde de la pulsabilité artérielle, etc.

Pour la prévention des embolies, on préférera également mesurer des paramètres locaux liés à l'imminence ou à la survenue d'une coagulation dans une voie particulièrement adéquate, telle que, par exemple, une oreillette ou une cavité ventriculaire.

L'un des paramètres préférés est constitué par l'électrocardiogramme (ECG) dont les variations sont analysées par des moyens électroniques connus d'analyse de l'ECG. De préférence ces moyens sont notamment sensibles au décalage du segment ST, de l'intervalle RR du cycle cardiaque, une ischémie myocardique donnant un décalage, décours, orientation ou vitesse de propagation du segment ST par rapport au tracé normal d'autant plus grand que l'ischémie est plus sévère et le volume myocardique concerné plus grand.

De préférence, l'ECG est acquis le long de deux ou plusieurs axes, permettant la localisation vectocardiographique de l'ischémie et la détection de la vitesse de propagation locale des dépolarisation et repolarisation. Ces électrodes de détection de l'ECG peuvent être endocavitaires ou intramyocardiaques, ou encore épicardiques, mais on préfère la combinaison d'un ensemble d'électrodes endocavitaires et/ou intramyocardiques et d'un ensemble épicardique ou juxtacardiaque (pouvant aussi être extracardiaque voire extrathoracique).

En cas de détection vectographique d'une modification, le dispositif vérifie tout d'abord que la modification est répétée sur plusieurs cycles consécutifs, et qu'il ne s'agit pas d'extrasystoles ou d'une tachycardie.

De préférence, le dispositif peut comparer le décalage ST d'un cycle à l'autre et détecter une modification progressive significative du segment ST.

Après le délai d'analyse et de contrôle, le dispositif actionne les moyens, de préférence programmés, de délivrance de doses médicamenteuses, et de préférence de thrombolytiques et/ou de vasodilatateurs coronariens, antispasmodiques, à action rapide.

De préférence, le dispositif mesure le nombre de cycles, ou la durée d'établissement de la déformation maximale du segment ST obtenue. Si cette durée est rapide, par example de l'ordre de quelques cycles cardiaques (par exemple moins de 15), le dispositif délivre un antispasmodique, par exemple trinitrine. Si la durée est plus longue, il délivre un thrombolytique de préférence. Cependant, dans une forme de réalisation simplifiée, il peut délivrer chaque fois un mélange de vasodilatateur et de thrombolytique, et éventuellement un antiarythmique ou un béta bloquant à faible dose.

Ce dispositif est avantageusement de préférence agencé pour comparer chaque cycle au cycle précédent. Si la durée de chacun des deux cycles consécutifs est peu différente (par exemple inférieure à 10 %) toute modification dans le même sens du décalage ST d'un cycle à l'autre est comptée comme significative et comptabilisée pour intervenir sur le programme de diagnostic et de traitement. Le dispositif peut varier le diagnostic et le traitement et notamment la dose selon la localisation et l'extension de l'ischémie détectée. Si la durée du cycle varie de plus de cette valeur, par exemple 10 %, le dispositif analyse, de préférence, la forme des complexes QRS pour détecter, d'une façon en soi connue, extrasystoles, arythmies ou tachycardies. Dans ce cas, le dispositif précise le diagnostic et peut déclencher la délivrance d'une dose d'antiarythmique.

De préférence, le dispositif comporte simultanément un capteur de pression ou autre moyen d'acquisition du mécanogramme cyclique du muscle cardiaque. Les moyens électroniques sont alors également sensibles aux déformations de la courbe de pression, par exemple à la pente d'attaque de la contraction systolique et/ou la durée de contraction isométrique. En cas d'affaiblissement de l'énergie de contraction, compte tenu des éventuelles modifications du rythme cardiaque, le dispositif est sensible à l'ischémie, et délivre une dose de médicament anti-ischémie et/ou délivre un signal destiné à un récepteur extérieur.

De préférence, le dispositif selon l'invention comporte simultanément des moyens d'acquisition des rythmes en rapport avec l'hémodynamique, notamment du rythme cardiaque et éventuellement du rythme respiratoire, par exemple électrocardiogramme, variations des impédances électriques, plethysmogramme, phonogramme, mécanogramme ou autres, cette détection pouvant être alors utilisée soit pour définir ou interpréter les mesures de paramètres variant à l'intérieur d'un cycle, par exemple dans le cas de l'impédance, des pressions ou des déformations géométriques ou de l'absorption optique, soit pour interpréter les résultats de la mesure en fonction des cycles cardiovasculaires détectés et ainsi modifier les seuils ou inhiber des fonctionnements.

Dans une forme de réalisation préférée, le dispositif est pourvu de moyens permettant de mesurer l'impédance ou conductance électrique, acoustique ou optique dans une ou plusieurs zones sensibles de la voie vasculaire à protéger.

On peut également disposer, dans un emplacement dans ou au bord de la circulation sanguine, ou même à l'extérieur, par exemple dans la moelle osseuse, par exemple sternale, une optique de type endoscopique à fibres de quartz transmettant l'image en couleurs et éventuellement avec angle de vision orientable, vers un analyseur d'images miniaturisé, d'un type également connu, où l'image est analysée, digitalisée, ordonnée et stockée sur une mémoire, cet ensemble étant, par exemple, situé dans un boîtier implanté contenant également les autres moyens du dispositif selon l'invention. Les images détectées peuvent être, d'une part, comparées, par des moyens de comparaison électroniques connus, à des images types mémorisées, par exemple de globules sanguins ou de cellules anormales dans leur formes, dans leurs contenus, dans leur localisation ou dans leurs quantités, et d'autre part, transmises à l'extérieur par émission radio-fréquence à la demande. Le dispositif peut ainsi déclencher, par exemple, une alarme extérieure par radio-fréquence pour signaler la vision d'une image anormale. Il peut également provoquer la délivrance automatique de doses de médicament adapté programmées à cet effet. On peut exécuter un prélèvement ponctionnable par aiguille transcutanée, et un nettoyage de l'optique par ultrasons, selon un procédé connu.

De façon préférée, les mesures d'impédance électriques se font simultanément sur au moins deux fréquences différentes, ce qui permet des mesures plus précises et mieux adaptées aux variations conductrices sélectives du milieu tissulaire ou liquide étudié. Un dispositif de mesure des impédances par modulation de fréquence d'émission sur au moins une gamme de variation de fréquence, et éventuellement plusieurs gammes de fréquence, peut être effectué en utilisant les mêmes électrodes d'émission et de réception ou en utilisant, par exemple, plusieurs électrodes, notamment des électrodes placées sur deux axes géométriques définissant un plan géométrique, axes de préférence perpendiculaires, pour permettre la détection des "vecteurs" d'impédance (courant maximal) dans ce plan. De préférence, on effectue également et simultanément la mesure dans un axe situé hors de ce plan, de préférence perpendiculaire à ce plan, afin d'acquérir les vectogrammes tridimensionnels des impédances. Les courants de mesure peuvent être, par exemple, émis en impulsions de balayage de fréquences simultanées ou décalées d'un axe de mesure à l'autre.

Le décalage temporel de certaines parties du spectre d'impédance électrique peut également être utilisé.

Ces moyens peuvent comporter des électrodes disposées autour des voies vasculaires à protéger, par exemple une artère coronaire, ou dans la voie vasculaire même, notamment dans une oreillette ou artère, ces électrodes étant reliées à des moyens permettant d'y adresser des courants de mesure d'impédance électrique.

Des électrodes locales ou régionales pour la mesure de l'impédance peuvent également être insérées dans le tissu myocardique, notamment apical, septal ou pariétal, de façon à mesurer l'impédance locale dans une partie du myocarde surveillée par les électrodes. La distance entre les électrodes peut être, par exemple, de quelques millimètres ou centimètres.

On peut ainsi mesurer les valeurs de l'impédance ou, ce qui revient au même, de la conductance soit de façon intermittente, soit de façon continue.

On peut notamment mesurer l'écart maximal d'impédance électrique traduisant, par exemple, des variations du volume sanguin dans la voie considérée ou celles de la circulation capillaire et/ou de la contraction musculaire dans le myocarde par exemple, à chaque cycle cardiaque.

On peut également mesurer, sur les décours de la courbe d'impédance acquise, les formes, pics et autres caractéristiques, y compris différentielles ou intégrales, à chaque cycle cardiaque.

Le courant utilisé pour la mesure des impédances peut être un courant continu en impulsions, par exemple dans le cas où l'on mesure l'impédance de zones très petites et assez homogènes, telles que par exemple une fine couche artificielle ou artificiellement provoquée de fibrine monomère ou polymère ou de précurseurs de la fibrine, fibrinogène, fibrinopeptides, en mélange ou isolés, ou de prothrombine mélangée à de la prothrombinase pour tester la tendance à la production de thrombine, facteur de la formation de fibrine tout en tenant compte, de préférence, de l'accumulation locale des ions calcium ou magnésium par rapport aux mêmes ions mesurés dans la circulation générale. Ce courant peut également être, de préférence, variable et l'on peut, le cas échéant, utiliser un ou des courants de formes et de fréquences variables ou différentes en fonction du type de variation d'impédance à mesurer. Pour la mesure d'une impédance d'un volume sanguin, par exemple dans une artère coronaire ou dans une cavité cardiaque, on peut utiliser des fréquences relativement basses, par exemple de l'ordre de quelques kilohertz. On préférera utiliser les fréquences plus élevées pour la mesure d'impédances tissulaires, par exemple de l'ordre de 100 kHz à une centaine de mégahertz. Les intensités de courant peuvent avantageusement être de l'ordre de 25 microampères.

On peut également enregistrer simultanément le long de deux axes, par exemple entre une électrode intraauriculaire et une électrode portée par le boîtier d'un stimulateur, d'une part, et une électrode intraventriculaire ou intramyocardique et la même électrode sur le boîtier d'autre part. On obtient ainsi une courbe de type Lissajoux dans le plan formé par les deux axes et l'on peut reconstituer le vectogramme tridimensionnel des variations des impédances électriques du coeur si l'on ajoute une mesure selon au moins un axe supplémentaire extérieur au plan. On peut également utiliser des électrodes cutanées ou sous-cutanées dans ce but pour reconstituer l'image tridimensionnelle de la variation des impédances dans le coeur et éventuellement pour émettre des signaux correspondants hors du corps vers un récepteur de visualisation tridimensionnelle.

De façon préférée, on peut superposer l'électrocardiogramme mono-, bi- ou, de préférence, tridimensionnel au vectogramme correspondant des variations des impédances électriques du coeur mono-, bi- ou, de préférence tri-dimensionnel. En cas de thrombose coronarienne qui provoque une modification nette de l'électrovectocardiogramme, le vectogramme des impédances électriques du coeur correspondant est nettement modifié et permet, d'une part, de valider la variation électrocardiographique et, d'autre part, de préciser les répercussions sur les contractions du myocarde.

Comme autre paramètre biologique pour la protection des artères ou cavités du coeur, pris isolément ou combiné à la mesure de l'impédance, on peut utiliser des modifications de l'électrocardiogramme est notamment les modifications du vectocardiogramme détecté par des jeux d'électrodes disposées convenablement dans l'espace, soit épicardiaques, soit simplement endocavitaires, soit en position monopolaire seule, soit avec ajout d'électrodes bipolaires, de préférence sur un cathéter endocavitaire d'un stimulateur cardiaque. On peut ainsi détecter des modifications significatives et sélectives de la propagation de l'électrogénèse cardiaque dans se dépolarisation et sa repolarisation par rapport aux cycles précédents.

A la place ou en plus des mesures du ou des paramètres électriques précités, on peut également prévoir des moyens sensibles à des phénomènes liés à la teneur en oxygène et/ou en CO² et/ou CO et/ou au pH et/ou au rH (coefficient d'oxydo-réduction) et/ou aux ions sodium, chlore ou phosphore pour mesurer la pression osmotique et/ou à une ischémie du tissue irrigué par la branche artérielle à protéger, et notamment un capteur sensible à des variations ioniques telles que celles de l'ion potassium ou de l'ion H⁺ dans ledit territoire ou au voisinage ou drainage de celui-ci et/ou un capteur sensible à la teneur en oxygène dans, ou près de, ce territoire, par exemple un capteur optique sensible à l'oxyhémoglobine à une longueur d'onde de 660 nm ou à la méthémoglobine. (Karl STANGL et al. A new Multisensor Pacing System ..., Pace, Vol. 11 Juin 1988 : 712-724).

Un paramètre particulièrement avantageux de l'invention est la concentration en nicotinamide adénine-dinucléotide réduit (NAOH) dans le myocarde. Cette concentration augmente notablement pendant l'ischémie puis est abaissée en cas de reperfusion coronarienne. Le dispositif selon l'invention comporte, par exemple, une fibre optique dont l'extrémité, avec son optique, est en contact ou enfoncée dans le myocarde, l'autre extrémité étant reliée à une source de lumière d'excitation, telle qu'un laser à azote à 338 nm et un laser à rhodamine à 586 nm (voir M. Toussaint et al., Fluorimétrie laser du NADH, Arch Mal. Coeur 1988 ; 81 (11):47-51). Ce microprocesseur compare en permanence le niveau de NADH à une valeur de seuil détectée compte tenu du rythme et de l'hémodynamique.

Dans une autre forme de réalisation, on peut également, pour détecter une menace survenue d'une hémorragie interne, placer un détecteur optique, par exemple un détecteur de lumière rouge ou autre sensible à la longueur d'ondes absorbée par l'hémoglobine et/ou autres substances colorées, par exemple bilirubine, au niveau des lymphatiques drainant la lymphe du corps ou du cerveau dans le canal lymphatique. On peut également ajouter à ce dispositif de dosage sensible à de faibles quantités d'hémoglobine dans les vaisseaux lymphatiques, au moins un microcompteur optique de type connu permettant de compter les globules rouges ou plaquettes et capable de quantifier un passage de sang dans la lymphe et, le cas échéant, de distinguer entre la provenance encéphalique ou non de ces éléments. Ainsi, une tendance hémorragique par hypocoagulabilité ou fragilité vasculaire, toxique ou infectieuse ou causée par une tension artérielle excessive, pourra être détectée et au besoin traitée automatiquement par la délivrance d'un médicament recoagulant tel que, par exemple, prothrombine en équilibre avec facteur Stuart et ions calcium ou hypotenseurs. Cette perfusion de recoagulation sera automatiquement stoppée dès que ledit détecteur de trace de sang dans la lymphe indique une disparition du saignement, ou bien dès qu'une mesure d'un facteur de la crase sanguine indique une tendance vers un début d'hypercoagulabilité ou de normalisation.

Il est également possible de détecter une hémorragie ou oedème intra-myocardique ou intra-musculaire par l'utilisation d'un détecteur optique sélectif discriminant entre l'hémoglobine ou ses dérivés et la myoglobine, une telle cellule optique, du type à fibre de silice endoscopique, permettant use analyse de l'image locale et l'observation d'une ischémie et le suivi de son évolution au repos et à l' effort, et pouvant être placée dans une partie intra-myocardique d'une sonde reliée à un stimulateur cardiaque. Des moyens de détection de couleurs par fibres sont, par exemple, décrits par G. Boisdé et J.J. Perez, Opto 48, p. 36-44, janvier-février 1989.

L'utilisation d'une cellule optique à fibres de quartz placée, par exemple, dans le cathéter, par exemple dans les cavités du coeur droit permet également de mesurer la concentration sanguine en globules rouges, plaquettes et globules blancs et de mesurer l'hématocrite dont une baisse accompagne une hémorragie, ou mesurer une baisse isolée des globules rouges ou des thrombocytes en rapport ou non avec une hémorragie.

On peut aussi utiliser des moyens sensibles au rayonnement lumineux, y compris IR ou UV, réfléchi ou absorbé par le sang et/ou le myocarde ou une paroi vasculaire lors d'une émission convenablement orientée de lumière monochromatique ou non, cohérente ou non, en continu ou en impulsions, par exemple en utilisant un conducteur optique très mince, par exemple fibre de silice d'un diamètre inférieur ou égal à 1 mm, dont une extrémité optique regarde la couleur du sang, ou du myocarde dans lequel elle est placée, des moyens associés à la fibre étant agencés pour analyser le spectre lumineux qui reflète l'oxygénation ou un autre paramètre métabolique du sang ou des tissus par exemple séparément.

Comme autre paramètre, on peut aussi mesurer la déformation géométrique de la voie vasculaire, myocarde ou cavité cardiaque, par exemple par jauge de contrainte, ou mesure de temps de transit d'ultrasons, par exemple du type TRITON-sonomicrometer, SL 5-2 avec deux cristaux de 2 mm de diamètre, fabriqué par TRITON Technology, Inc., San Diego, Californie, Etats-Unis d'Amérique.

On peut également utiliser un ou plusieurs de ces capteurs piézo-électriques pour détecter les variations de propagation et de réflexion sonores ou ultrasonores ou rechercher des variations de résonance sonore ou ultrasonore ou encore d'absorption sonore et/ou ultrasonore de préférence liées aux cycles cardiaques, par exemple. On peut également utiliser des capteurs tels que des microphones pour détecter la qualité et le niveau sonore des bruits produits par le myocarde, l'hémodynamique et/ou les valves cardiaques.

En variante, de tels capteurs peuvent également être émetteurs de sons ou d'ultrasons dans le sang et/ou dans le myocarde afin de détecter une éventuelle anomalie fonctionnelle globale ou régionale pouvant être déterminée par une sténose ou obstruction ou une hypertension artérielle excessive. Dans une variante, l'onde ultrasonore peut être modulée par une ou des fréquences sonores. Simultanément, des modulations sonores peuvent être déclenchées par rapport au phonocardiogramme détecté. De préférence, les sons ou ultrasons sont émis avec une variation rapide de fréquence balayant un spectre programmé afin de détecter la résonance et/ou l'absorption sonore de la colonne sanguine artérielle ou veineuse, ou du myocarde, selon la position et l'orientation du capteur-émetteur. Ces émissions peuvent être permanentes ou très rapprochées de façon à balayer la totalité des cycles cardiaques, ou au contraire, on peut les produire pendant une ou plusieurs périodes déterminées du cycle cardiaque en calant temporellement chaque émission et réception sonore ou ultrasonore sur un phénomène périodique du cycle, de façon à provoquer l'émission et la mesure subséquente en un instant significatif pour les pressions et volumes des cavités cardiaques, des colonnes veineuses ou artérielles ou du myocarde. En particulier, on peut régler les émissions et mesures sur des phénomènes sonores sélectionnés, par exemple fermeture d'une valve cardiaque ou bruits accompagnant les mouvements du myocarde.

On peut aussi, par exemple, si l'on provoque l'émission et la mesure à l'aide d'un capteur-émetteur convenablement placé orienté vers l'arbre sanguin veineux ou artériel, selon le cas, à partir d'une sonde, par exemple endocavitaire, mesurer la résonance et l'absorption d'un arbre sanguin artériel ou veineux, ce qui donne une indication immédiate de l'état de la pression de la masse sanguine et de la rigidité de la paroi artérielle. A chaque maximum et minimum de la pression sanguine correspond respectivement une fréquence de résonance pour chaque sujet. Ceci permet, par exemple, de détecter l'apparition ou l'accroissement d'une hypertension susceptible de favoriser une thrombose, embolie ou hémorragie vasculaire de sorte que le dispositif peut alors immédiatement administrer une dose de médicament correcteur. On peut également, en choisissant convenablement le moment de la mesure, le disposition et l'orientation du capteur-émetteur, détecter et quantifier l'éventuel volume sanguin, notamment ventriculaire, post-systolique ou télé-diastolique. A titre d'exemple, la recherche et la mesure de la résonance de l'arbre sanguin ou artériel peuvent être effectuées à l'aide d'un capteur-émetteur ultrasonore ou sonore placé dans ou près de la cavité du ventricule gauche par un cathéter trans-septal provenant du ventricule droit.

En variante, au lieu d'un balayage temporel de fréquence sonore ou ultrasonore, par exemple en modulation d'amplitude d'une fréquence ultrasonore plus élevée, lors de l'émission, on peut également émettre simultanément plusieurs fréquences s'échelonnant dans le spectre voulu. Les dispositifs émetteur et récepteur sonore ou ultrasonore décrits peuvent également être placés au niveau d'un vaisseau périphérique, par exemple l'artère radiale au niveau du bras ou du poignet, en étant implantés de façon à faire vibrer en résonance la colonne artérielle correspondante à partir de cet emplacement périphérique, la détection du spectre sonore résultant pouvant se faire éventuellement à distance de l'émetteur.

Dans une variante de l'invention, on peut provoquer, par télécommande de l'extérieur, la mise en oeuvre, par les moyens programmés ou programmables implantés, de la mesure d'une substance particulière présente dans le sang, par exemple une substance toxique, le dispositif ajustant un moyen de mesure, par exemple moléculaire optique, pour devenir sensible à cette substance. Dans le cas d'un toxique, par exemple alimentaire, on peut prévoir un dispositif spectrométrique ou autre, en soi connu, de recherche du toxique et de sa concentration dans l'aliment, l'eau ou l'air et transmettre l'information par télécommande au dispositif implanté pour le rendre sensible à cette substance et, éventuellement en retour, informer l'appareil externe ou l'utilisateur, du degré de concentration intracorporelle de ce toxique et lui indiquer la possibilité ou non de tolérer une incorporation supplémentaire de ce toxique. On peut aussi implanter, par exemple dans la moelle osseuse, un détecteur de rayonnement α,β,γ ou isotopique.

Comme autre paramètre biologique, dans une forme de réalisation préférée, on peut également mesurer, dans une voie vasculaire ou lymphatique, de préférence en amont d' un ganglion, ou une cavité cardiaque, notamment oreillette ou ventricule, au moins un paramètre de la crase sanguine, par exemple en utilisant un ou des capteurs sensibles à un ou plusieurs facteurs de la coagulation, soit de façon plus générale en mesurant un paramètré lié à la coagulabilité du sang ou liquéfaction des coagulats tel que, par exemple, viscosité ou tendance au dépôt de la fibrine. Par exemple, on peut mesurer la teneur du sang en l'une au moins des substances suivantes : prothrombine, plasminogène, prothrombinase, plasmine, fibrinopeptide, protéine C, endothéline, sérotonine, catécholamines, neuropeptides Y, fibrinogène, activateurs du plasminogène, etc.

On peut avantageusement utiliser, à cet effet, une présentation artificielle d'un ou plusieurs facteurs de la coagulation ou de leurs précurseurs ou antagonistes, utilisée dans un test de provocation ou d'inhibition de la coagulabilité. Cette présentation permanente ou périodique peut être réalisée notamment :
- par fixation définitive de cette substance sur un support insoluble inaltérable dans le sang, laissant libres, en contact avec le sang, les sites actifs des facteurs, leur permettant de solliciter localement les facteurs correspondants de la crase sanguine ; cette fixation peut être effectuée de toute façon connue, par exemple adsorption sur le support, réticulation à l'aide d'un agent réticulant usuel, liaison chimique usuelle avec le support, par exemple du type avidine-biotine ; le support peut être un support habituel dans ces techniques, par exemple polymère, acétate de cellulose, nitrocellulose ; on peut aussi utiliser la technique de détection d'ions ou de molécules par la surface de transistors à effet de champ en fixant des molécules à la surface de la silice, ces molécules étant activées pour recevoir des molécules sensibles à la détection, et étant reliées entre elles pour former un film très solide sur la silice ; voir M. Sugi, "Langmuir-Blodgett Films ; a Course towards Molecular Electronics : a Review", J. of Molec. Electron. 1, 3 (1985) ; et A. Barraud, "Conducteurs organiques ultraminces, la méthode de Langmuir-Blodgett" Clefs CEA, 6, 42 (1987),
- par diffusion lente de la substance, par exemple, à travers une membrane poreuse,
- par microperfusion locale, de l'ordre de quelques mm³ par jour, permettant par exemple une recharge semestrielle du réservoir sous-cutané correspondant.

Dans une variante de l'invention, au moins deux facteurs biologiquement opposés dans l'équilibre de la crase sanguine peuvent être ainsi simultanément ou successivement surveillés (ou stimulés), par exemple les facteurs coagulants de la fibrinogénèse et/ou de la thrombogénèse, d'une part, et les facteurs anticoagulants tels qu'activateur de la plasmine, plasminogène, ou dérivés de l'héparine, d'autre part. Ainsi, il devient possible de connaître un mécanisme pathogénique en cas d'anomalie de la crase sanguine et d'intervenir de façon sélective. Par exemple, en cas de CIVD (coagulation intravasculaire diminuée), l'insuffisance de la thrombine détectée, associée à une chute du fibrinogène, permettra d'en faire le diagnostic.

Ces capteurs ou moyens d'analyse peuvent être, par exemple, portés par un cathéter. Ainsi, à titre d'exemple, on peut mesurer la tendance au dépôt de la fibrine à partir d'une fine couche ou d'un fin fil, pointe ou tube de fibrine dans un réceptacle ou sur un relief d'un cathéter ou au niveau d'un petit segment d'autogreffe veineuse, fixé, par exemple, sur le cathéter et entouré de préférence d'une grille ou butée de protection, par un moyen optique, par exemple sensible à la couleur et/ou absorption moléculaire et/ou épaisseur de la fibrine, ou tout autre moyen, par exemple par mesure de la résonance sonore ou ultrasonore qui dépend de la masse de fibrine déposée ou de sa masse moléculaire détectable, ou par la mesure d'au moins une dimension de la couche de fibrine par mesure optique ou échographie ultrasonore, par exemple en mode A. On peut aussi mesurer l'impédance électrique, par exemple ohmique, et/ou l'absorption d'un courant alternatif de fréquence donnée fixe ou variable ou d'ultrasons par la résonance de macromolécules intervenant dans la coagulation ou dans la thrombolyse. Ce réceptacle ou relief peut être agencé en forme de V ou évasé pour créer une tendance à un minime dépôt de fibrine normalement contenu par le lavage sanguin et la fibrinolyse spontanée et dont la dimension dépend de l'état de la crase sanguine. Il peut être entouré d'une digue ou grillage empêchant l'arrivée des éléments figurés du sang et l'extension de la couche de fibrine.

A titre d'exemple, le dispositif peut comporter, sur un cathéter, un court élément tubulaire ayant un diamètre par exemple de l'ordre de 2 à 3 millimètres, disposé latéralement sur le cathéter et ayant un axe sensiblement parallèle à celui-ci de façon à être traversé par une partie du flux sanguin. Le cathéter présente, à l'intérieur de cet élément tubulaire, ou dans une cavité, un capteur de détection de substances telles que fibrine ou fibrinopeptides, monomères de fibrine, prothrombines et prothrombinases, constitué d'une fente ou ouverture ou intervalle de faible épaisseur entre une première paroi solidaire du cathéter et une deuxième paroi également solidaire du cathéter. L'espace entre ces deux parois peut être déterminé expérimentalement en recherchant, dans un sang dont l'état de la crase est normale, l'épaisseur conduisant à un fin dépôt de fibrine. Sur l'une des deux parois débouche l'extrémité d'une fibre optique reliée à une source lumineuse et en face, sur l'autre paroi, débouche le début d'une deuxième fibre optique. Les deux fibres optiques sont renvoyées dans le cathéter et aboutissent, la première, à une source d'émission lumineuse, la seconde à un capteur lumineux. L'ensemble de ces deux parois ou reliefs est entouré d'un grillage ou butée empêchant le passage ou l'agglomération des éléments figurés du sang et empêchant la sortie d'éléments de fibrine. Au niveau de ce capteur, par exemple tout autour de l'ensemble desdites deux parois, se trouvent disposés des moyens d'amenée d'un thrombolytique permettant, si nécessaire, de dissoudre la fibrine susceptible de s'accumuler sur le capteur. Lorsqu'un mince dépôt de fibrine ou de ses précurseurs se forme entre les deux extrémités optiques sur lesdites parois, la transmission optique change et l'intensité lumineuse reçue est atténuée. En outre, la couleur est modifiée de sorte que les moyens sensibles à la réception optique peuvent déterminer, lorsqu'un certain seuil est franchi, qu'une microcouche de fibrine est en train de se former. Le dispositif peut même être sensible à la vitesse et au sens de variation de la micro-couche de fibrine en mesurant la variation du signal optique dans le temps.

A une certaine distance en aval, ou sur la même pièce de présentation peut avantageusement se trouver, sur le cathéter et dans le même élément tubulaire, un capteur de détection d'élément favorisant la liquéfaction sanguine telle que la détection d'héparine, de plasminogène, protéine C, etc.

On peut également détecter la présence et la concentration, et même la taille, d'éléments présents dans le sang, tels que particules, corpuscules ou molécules, notamment les substances ou facteurs en rapport avec la crase sanguine dont certains ont été énumérés ci-dessus, notamment la fibrine soluble ou ses prédécesseurs ou d'autres protéines, notamment fibrinogène, plasminogène, prothrombine, etc. Ceci peut être effectué, par exemple, à l'aide d'une source optique dont la longueur d'onde est variée selon un programme de microprocesseur, par exemple à l'aide d'un prisme tournant ou un système de prismes à réflexion multiple et/ou de réseaux optiques connus et/ou de sources monochromatiques multiples ou laser modulé ; la détection des spectres d'absorption des molécules recherchées peut se faire par exemple par des microthermocouples et/ou des cellules photoélectriques ou photovoltaïques très sensibles et/ou des transistors à effet de champ (FET) ou autres fonctionnant en thermistance ou en détecteurs des charges électriques locales. On peut également utiliser un laser ou maser et étudier ces absorptions par détection et étude des interférences. La fréquence d'émission peut soit être fixe et déterminée en fonction de l'élément recherché, soit variable en balayant certaines zones de fréquence, par exemple dans le cas d'éléments tels que la fibrine ou les facteurs ou précurseurs de la fibrino- ou thrombino-formation dont la formation s'effectue par polymérisations ou clivages successifs qui modifient la masse moléculaire.

En particulier, la mesure par tests de provocation de l'état de la coagulabilité et de l'incoagulabilité sanguines peut être effectuée non pas sur une mince couche solide ou semi-solide des précurseurs de la fibrine ou thrombine, d'une part, et des héparines et plasminogène, d'autre part, mais sur la concentration relative sous forme liquide de ces substances à proximité immédiate d'un foyer de présentation de substances coagulo-actives et/ou anti-coagulantes. Ces foyers de présentation peuvent être soit de type solide avec des substances insolubilisées sur un support, soit constitués d'une diffusion liquide comme décrit précédemment. On évite ainsi de provoquer des dépôts de fibrine et l'on évite également de prévoir des grilles ou butées de protection. Dans une variante, les molécules facteurs de la coagulation et de l'anticoagulation sont fixées sur une matière insoluble, sur une surface, par exemple une tige dans un petit tube, ralentissant la circulation sanguine locale, de manière que le rapport des détections locale et générale des ions calcium, magnésium ou H+, permette, à lui seul, d'évaluer automatiquement l'état de la crase sanguine.

Dans une variante de l'invention, une émission ultrasonore ou autre moyen, laser pulsé ou autre, peut être utilisé pour détruire ou disloquer l'amas dangereux qui peut se former sur le capteur dans le volume protégé, les débris étant détruits par le fibrinolytique délivré ou la fibrinolyse spontanée.

On peut également utiliser, comme paramètre, l'agrégation globulaire, notamment plaquettaire, par un moyen de détection convenable, par exemple de type optique.

On peut également prévoir des moyens pour mesurer l'hémoglobine et/ou les globules rouges sanguins dans un volume de quelques mm3 et déterminer ainsi l'hématocrite et l'hémoglobine par litre. Cette dernière analyse permet de détecter et suivre certaines hémorragies, polyglobulies et anomalies de la volhémie. Des capteurs de ce type sont connus et peuvent être miniaturisés.

Le dispositif selon l'invention, convenablement muni de moyens devenus maintenant classiques de transmission/réception, de programmation et de monitorage, peut être prévu pour programmer de nouvelles longueurs d'ondes ou de nouvelles modulations d'ondes pour les émissions de détection, destinées à rechercher de nouvelles molécules ou nouveaux rapports de concentration de celles-ci. Le dispositif peut également permettre la télécommunication automatique avec émission d' une alarme sélective ou de données biologiques mesurées, selon des modes connus.

Comme cela a été vu, les différents capteurs et/ou émetteurs décrits ci-dessus, qu'il s'agisse d'électrodes, de capteurs à ions, de capteurs piézo-électriques, de capteurs optiques avec emplacement de test de provocation ou d'inhibitionde la coagulation sanguine, etc., peuvent être disposés en divers emplacements dans le coeur ou les vaisseaux ou à proximité. L'une des localisations préférées est la localisation intracavitaire sur un ou plusieurs cathéters. Une autre localisation avantageuse peut être effectuée au niveau d'un pontage coronarien ou d'une prothèse valvulaire si elle doit être posée chez un patient; ainsi la prothèse valvulaire peut comporter un ou plusieurs des éléments tels qu'électrodes de mesure d'impédance du sang ou de la fibrine locale, capteur de pression, etc.

Lorsque le paramètre mesuré franchit un seuil déterminé à l'avance, le dispositif peut alors automatiquement et de façon régulière ou périodique provoquer l'envoi dans le sang, à partir d'une pompe et réservoir implantés, de quantités réduites, par exemple du type goutte à goutte, de fibrinolytique ou d'anticoagulant ou d'antihémorragique ou autre agent.

Au contraire, si, lors de la mesure, le paramètre tombe au-dessous du seuil déterminé, toute délivrance de fibrinolytique ou d'anticoagulant est arrêtée. La fréquence à laquelle le dispositif procède aux mesures est adaptée aux caractéristiques de l'agent thérapeutique et de ses effets, par exemple à la rapidité et durée d'efficacité, à sa demi-vie et, éventuellement, aux particularités connues du patient. A titre d'exemple, l'intervalle entre deux mesures successives peut aller d'une fraction de seconde à quelques heures ou la mesure peut même être continue, au moins par périodes.

Bien entendu, lorsque le dispositif est prévu pour délivrer sélectivement plusieurs agents par exemple complémentaires ou antagonistes, ou indépendants, il peut être prévu un ou plusieurs paramètres différents avec un ou plusieurs seuils différents associés aux détections respectives.

De façon particulièrement préférée, le dispositif comporte un ou des capteurs susceptibles de mesurer l'un au moins et de préférence plusieurs des paramètres suivants :
- apparition ou variation d'une mince couche ou masse de fibrine ou contenant de la fibrine ou un ou plusieurs de ses précurseurs sur un support convenablement disposé dans la circulation sanguine, par exemple dans l'oreillette ou le ventricule, de sorte que l'augmentation de la masse correspond à une tendance à l'hypercoagulabilité du sang alors que la diminution de la masse à une tendance à la thrombolyse ; on peut ainsi suivre très rapidement une variation de l'état de la crase sanguine alors que les procédés actuels exigent un prélèvement extra-corporel discontinu et long à interpréter. Cette couche peut avantageusement être mise sous une tension électrique ;
- mesure de la concentration en ions calcium et/ou magnésium au voisinage immédiat de, ou dans, cette fine couche ou pellicule, à l'aide d'un capteur à base d'une membrane sélective aux ions calcium en soi connue, permettant de suivre instantanément les variations de concentration locale en ion calcium, liées au degré de coagulabilité du sang par rapport à la concentration générale en ions calcium mesurée ailleurs par un autre capteur dans le sang.

La petite couche peut, par exemple, se former sur une surface, par exemple en verre à microrayures, portée par le cathéter et chargée d'électricité négative de voltage déterminé, par ailleurs connue, provoquant la formation locale du complexe activateur de la prothrombine et la tendance à la coagulation.

La valeur de ce voltage négatif peut être variée, soit dans le temps, soit spatialement, le long de ladite surface, ou d'une succession de telles surfaces, les moyens de mesure prenant alors en compte la valeur du voltage correspondant à l'accroissement le plus rapide du dépôt.

La surface peut, le cas échéant, présenter des précurseurs ou autres facteurs influençant la formation de fibrine ou précurseurs, convenablement insolubilisés ou fixés, par exemple, sur la surface électriquement négativée. La surface peut permettre le contact direct des plaquettes.

Les moyens pour déterminer le ou les seuils auxquels on compare la valeur mesurée du paramètre permettent de régler manuellement et de préférence d'ajuster automatiquement la valeur ou les valeurs de seuil. De préférence, ces moyens peuvent avantageusement être télécommandés depuis l'extérieur de l'organisme comme dans les télécommandes de stimulateurs cardiaques, et être visualisés sur un moniteur externe.

Le dispositif comporte, associés à ses moyens de détermination de seuil, des moyens de comparaison logiques ou analogiques, permettant de comparer les valeurs mesurées à la valeur programmée de seuil pour actionner, si le seuil est franchi, les moyens permettant de libérer la dose appropriée de substance médicamenteuse dans la circulation, de préférence après autorisation par des moyens de contrôle de la signification pathologique ou métabolique de ce franchissement.

Ces moyens de libération peuvent avantageusement comporter une ou plusieurs pompes et réservoirs implantés, contenant la ou les substances à administrer et susceptibles de délivrer, par au moins un conduit intracardiaque ou intracorporel, une quantité programmée de substance.

La détermination de la dose à libérer peut être effectuée par le médecin, de façon tout à fait usuelle, en tenant compte des facteurs classiques, tels qu'activité, clairance, demi-vie, etc., relatifs au(x) médicament(s) choisi(s). La pompe implantée possède, à cet effet, un dispositif de réglage de dose usuel, des moyens de télécommande, par exemple électromagnétiques, étant de préférence prévus pour permettre au médecin de régler la dose par commande hertzienne ou autre depuis l'extérieur de l'organisme.

Ces moyens peuvent, par exemple, être reliés au système vasculaire, veineux ou artériel général ou à un vaisseau local, déterminé, par exemple une artère coronaire ou carotide.

Dans le cas où l'invention s'applique à un dispositif de protection d'artères coronaires, il est préférable, pour un traitement in situ, de faire déboucher le conduit en provenance de la réserve de substance dans l'artère coronaire, le conduit étant alors de préférence un conduit contenu dans un cathéter endocavitaire ou accompagnant un tel cathéter, ledit conduit pouvant traverser le myocarde dans sa zone apicale par exemple, pour être relié, après sa sortie épicardique, à l'artère coronaire considérée. On peut cependant aussi délivrer la substance dans un autre vaisseau, ou dans une cavité cardiaque, oreillette ou ventricule ou même parfois dans un tissu.

De même, dans une telle réalisation, on préfère que les conducteurs reliés à des électrodes de mesure d'impédances d'artères coronaires, traversent le myocarde pour rejoindre, de préférence, le cathéter d'un stimulateur cardiaque dans la cavité ventriculaire droite.

Conformément à un perfectionnement important du dispositif selon l'invention, ce dispositif peut comporter des moyens sensibles aux cycles cardiaques et/ou pulsatoires du système artériel surveillé, ces moyens sensibles pilotant les moyens de mesure du paramètre de façon à provoquer les mesures en des instants déterminés ou connus du cycle.

On peut aussi, dans une forme particulièrement préférée de l'invention, prévoir de tels moyens sensibles aux cycles cardiaques, par exemple à la durée du cycle cardiaque, et éventuellement l'origine et la propagation de l'activation électrique qui le détermine, de façon à pouvoir juger, en cas de franchissement de seuil, du caractère pathologique ou non de ce fonctionnement, et décider, ou non l'intervention automatique.

Cette détection peut également être utilisée pour modifier automatiquement les seuils et/ou les traitements, en fonction d'une programmation déterminée.

Dans le cas d'artères périphériques, on peut utiliser des détecteurs implantés de pouls ou des capteurs de pression endo- ou périartérielle ou autres dispositifs connus.

Dans le cas de la protection contre les hémopathies, telles que les leucémies, on peut utiliser une cellule optique ou l'extrémité optique d'un endoscope fibroscopique associé à des moyens de mesure et/ou de comptage des cellules sanguines, associé à des moyens d'analyse des images détectées, l'optique d'acquisition étant disposée à l'intérieur d'un ganglion lymphatique ou d'une moelle osseuse, par exemple sternale. Des électrodes intra- ou périosseuses peuvent également être utilisées comme électrodes électrocardiographiques ou pour la mesure des variations d'impédances électriques.

Dans le cas de la protection au niveau du coeur et/ou des artères coronaires cardiaques, il est particulièrement utile de prévoir des moyens sensibles à la durée, et de préférence également à la qualité de propagation de l'activation électrique, au moins d'un cycle cardiaque précédent, et éventuellement de plusieurs cycles antérieurs, tels que décrits par exemple dans les brevets Zacouto US-A-3 857 399 et 4 052 991.

On peut ainsi, par exemple en cas d'extrasystoles, modifier automatiquement les seuils par rapport à la précocité de l'extrasystole et à la potentialisation extrasystolique résultant des paramètres mécaniques et métaboliques du myocarde.

Par exemple, également en cas de tachycardie, la diminution des amplitudes des phénomènes mécaniques et la modification du métabolisme, qui peuvent conduire à une diminution brusque, mais sans thrombose, du débit coronarien, ne seront pas considérées comme justifiant la libération d'une dose de thrombolytique pur ou d'un vasodilatateur coronarien, par exemple, sauf si une thrombose coronarienne est détectée simultanément par un autre moyen.

On peut également, et cette forme de réalisation est préférée dans le cas de la protection du coeur ou de ses artères coronaires, prévoir des moyens d'acquisition du phénomène électrique cardiaque tel que l'électrocardiogramme et/ou au moins un capteur sensible aux variations de pression et/ou de volume d'une cavité cardiaque, pour piloter les moyens de mesure de façon à réaliser la mesure en un moment déterminé ou connu du cycle, notamment systolique ou diastolique.

Dans un tel dispositif, la différence ou variation de paramètre tel que l'impédance électrique entre diastole et systole est comparée à un seuil ou fourchette de seuil qui peut être fixe ou, le cas échéant, ajusté automatiquement, notamment en fonction de la fréquence cardiaque et/ou des tensions artérielles et par exemple du niveau détecté des catécholamines sanguines ou intramyocardiaques.

En plus de, ou à la place de, la mesure de la différence d'impédance électrique entre diastole et systole, on peut également détecter les décalages temporels de cette impédance et/ou de la pression sanguine ou myocardique par rapport à l'électrogénèse, par exemple acquise par l'électrocardiogramme général ou local, et/ou par rapport aux cycles de pressions statiques ou dynamiques locales ou générales. Le dispositif peut alors être agencé pour être sensible aux variations des décalages. De préférence, le dispositif est agencé pour acquérir l'électrocardiogramme local à l'endroit où l'on mesure l'impédance, et de préférence simultanément la pression, pour s'affranchir des modifications de l'électrogénèse et du mécanogramme qui ne sont pas obligatoirement contrôlées ni significatives par rapport aux phénomènes localement mesurés.

Ce décalage est de préférence étudié par le dispositif selon l'invention dans le myocarde, par exemple apical. On peut également, en plus, mesurer les variations et décalage des mêmes paramètres dans une cavité cardiaque telle que par exemple le ventricule, de préférence à assez faible distance de l'emplacement où l'on effectue les détections des mêmes paramètres dans le myocarde de façon à procéder à une comparaison des détections dans ce tissu et dans le sang.

Dans une autre forme de réalisation, le dispositif comporte une pompe ou autre moyen implanté de délivrance rapide de médicament, de préférence par tube intravasculaire ou endocavitaire, ce moyen étant actionnable par télécommande de l'extérieur, notamment par le patient lui-même. En cas de douleur caractéristique, celui-ci actionne la télécommande extérieure, provoquant la délivrance immédiate d'une dose adaptée de mécidament, notamment thrombolytique et/ou antispasmodique coronaire. Ce dispositif, d'une grande simplicité, permet au patient de se protéger immédiatement d'une menace de spasme ou thrombose coronariens tout en diminuant significativement la dose de médicament et, par conséquent, en évitant pratiquement les effets secondaires des doses habituelles. De préférence, les doses délivrées peuvent être de l'ordre de 5 à 20 % des doses habituelles. Le dispositif peut être programmé pour comptabiliser la quantité totale délivrée par rapport au temps et éviter un cumul de doses dépassant un seuil tolérable.

Bien entendu, cette forme de réalisation peut être associée à d'autres formes de réalisation de l'invention, notamment le contrôle de facteurs de la coagulation.

Dans une forme de réalisation particulière, le dispositif selon l'invention peut comporter des moyens, fonctionnant de préférence par analyse optique comme décrit précédemment, à la fréquence d'absorption convenable, connue, sensibles à la concentration du sang en myoglobine (poids moléculaire 12599) et/ou myosine (poids moléculaire 5OO OOO). En cas d'augmentation, par exemple de 1O %, de la valeur en 3O minutes, le dispositif provoque la délivrance d'une dose de thrombolytique et/ou de vasodilatateur. La détection de la myoglobine et ou de la myosine s'effectue de préférence par l'extrémité d'une fibre optique placée au niveau du sinus coronaire.

Dans une autre forme de réalisation, le microprocesseur du dispositif, grâce à des électrodes disposées en au moins trois points (14, 14a, 15),peut détecter la déformation de la courbe de l'impédance vectorielle, normalement de forme arrondie, et être sensible à l'apparition d'une déformation rendant la courbe bifide, auquel cas il délivre une dose de médicament.

Le dispositif peut en plus comprendre, en combinaison, un stimulateur électrique portatif orthorythmique (POR) de préférence implanté mais pouvant également être externe, et possédant des électrodes permettant de détecter le rythme cardiaque et d'adresser des impulsions de stimulation au coeur, et un défibrillateur portatif, de préférence externe, comprenant une source d'énergie et des électrodes de défibrillation, et des moyens de détection d'une fibrillation ou tachycardie rapide, par exemple polymorphe ou mal tolérée, pour mettre en oeuvre ledit défibrillateur, des moyens, d'un type en soi connu, étant prévus pour protéger le circuit électronique du stimulateur cardiaque en cas de mise en oeuvre de la défibrillation, ainsi que des moyens de détection de l'hémodynamique centrale et/ou artérielle et susceptibles d'empêcher le fonctionnement du défibrillateur tant qu'un fonctionnement hémodynamique suffisant reste détecté.

Les moyens de détection de l'hémodynamique comportent de préférence au moins un capteur de pression cardiaque. Ce capteur peut être endocavitaire, de préférence intraventriculaire, de préférence gauche, ou intramyocardique ou, de préférence, on utilisera au moins deux capteurs, l'un intraventriculaire, l'autre intramyocardique.

Cependant, on peut également, à la place des capteurs de pression, ou en combinaison avec eux, prévoir sur un cathéter intraventriculaire, une pluralité d'électrodes longitudinalement espacées le long de la cavité, pour mesurer la variation volumétrique de la cavité et/ou débit par mesure de conductance électrique à chaque cycle comme décrit par John C. WOODARD dans Ventricular Volumetry by Catheter Measurement of Conductance, PACE, (Juillet-août 1987), vol. 10, N.-York, USA. La détection d'une chute importante de la variation volumétrique de la cavité autorise, en combinaison avec la détection par le POR d'une tachyarythmie très grave, le déclenchement d'une défibrillation
Dans une première forme de réalisation de l'invention, la totalité du dispositif est externe.

Dans une autre forme de réalisation, l'une au moins des éléments peut être implanté, qu'il s'agisse du stimulateur, du défibrillateur, ou des moyens de détection de l'hémodynamique, et/ou des moyens de détection de l'activité électrique du coeur et/ou de paramètres électriques artificiellement induits.

Dans le cas, préféré, où le défibrillateur est non implanté, le défibrillateur peut avantageusement posséder, de préférence sur une ceinture ou autre harnais thoracique, au moins deux électrodes de grande surface, de préférence souples, susceptibles d'être appliquées et maintenues sur la peau.

Ces deux électrodes de grande surface sont disposées sensiblement en opposition, c'est-à-dire de façon telle que le choc électrique circulant entre les deux électrodes, qui sont de polarités opposées au moment du choc, traverse la plus grande partie possible du muscle cardiaque. Il est préférable de prévoir, réparties sur la ceinture, plusieurs paires d'électrodes en opposition, en des positions angulaires différentes, ces électrodes étant disposées autour du thorax, deux à deux en opposition, et étant également chaque fois de polarités opposées.

Dans cette forme de réalisation, on peut avantageusement prévoir une mesure permanente, par exemple par microcourant, de l'impédance entre les électrodes et la peau de façon à vérifier le bon contact des électrodes avec la peau, des moyens étant prévus pour émettre un signal auditif ou visuel d'alarme en cas de perte de bon contact des électrodes. De préférence, les moyens électroniques sont alors agencés de façon à empêcher, en cas de contact insuffisant des électrodes avec la peau, l'envoi d'une impulsion de défibrillation.

Dans une autre forme de réalisation, on peut avantageusement réaliser le choc électrique de défibrillation entre, d'une part, une ou des électrodes intra-cardiaques ayant une polarité déterminée, et une ou, de préférence, plusieurs électrodes extra-cardiaques, de préférence réparties autour du coeur.

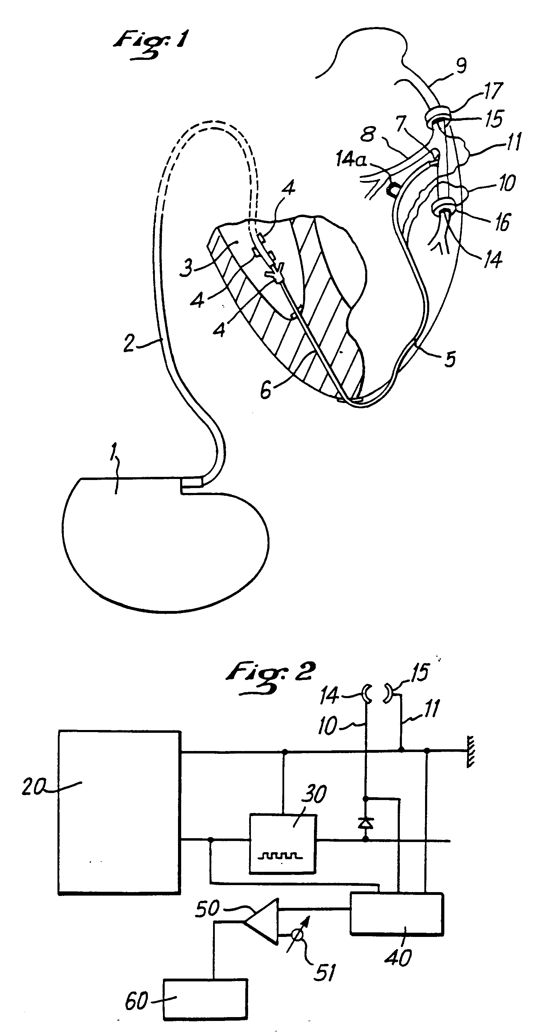

Dans un premier cas, on peut prévoir, sur une ceinture, une ou, de préférence, plusieurs électrodes cutanées, ayant, cette fois, la même polarité et qui coopèrent avec l'électrode intra-cardiaque ayant une polarité opposée, la fermeture du circuit électrique s'effectuant alors avantageusement par l'intermédiaire d'une liaison électromagnétique dont les deux bobinages sont respectivement extra- et sous-cutanés, comme décrit par exemple par F. Zacouto (Aperçu sur l'Expérimentation des pompes sanguines implantables, Réanimation et Organes artificiels, Tome 2 (1965) n^{o} 2, Masson et Cie, Editeur, Paris, p. 155-181 et plus spécialement fig. 1b).

L'électrode intra-cardiaque est de préférence, dans ces formes de réalisation, portée par le cathéter endocavitaire associé au stimulateur implanté. De préférence, cette électrode est, au moyen d'un mandrin contenu dans le cathéter, susceptible d'être introduite et maintenue dans le myocarde même, selon une technique en soi connue, cette électrode étant alors indépendante fonctionnellement de la ou des électrodes de stimulation et de détection du stimulateur lui-même, bien qu'on puisse, à la rigueur, utiliser la même électrode.

Il est ainsi possible, grâce à l'utilisation de l'électrode intra-cardiaque, utilisant de préférence le même cathéter du stimulateur, de diminuer considérablement l'énergie du choc de défibrillation efficace et, par conséquent, l'inconfort du sujet et la taille de la source d'énergie externe, cette source pouvant avantageusement être constituée d'un ou plusieurs condensateurs portatifs, par exemple portés par la ceinture qui porte les électrodes cutanées.

Dans une autre variante, dans laquelle on utilise également la ou les électrodes intra-cardiaques, par exemple sur le même cathéter que celui qui porte les électrodes du stimulateur, on peut cette fois-ci implanter une ou, de préférence, une pluralité d'électrodes d'assez grandes surfaces, de même polarité opposée à celle de la ou des électrodes intra-cardiaques, ces électrodes implantées pouvant être sous-cutanées ou, de préférence, insérées dans ou sous les muscles thoraciques, de préférence réparties autour de la circonférence thoracique. On préfère avoir au moins deux telles électrodes, écartées l'une de l'autre, et, de préférence, trois ou quatre. Ces électrodes sont toutes réunies, par des conducteurs convenables, à la source d'énergie délivrant l'énergie électrique nécessaire pour le choc de défibrillation. Cette énergie peut être contenue, par exemple, dans un ou plusieurs condensateurs implantés, mais, de façon avantageuse, elle peut être extérieure, portée par une ceinture ou harnais, le condensateur externe alimentant alors lesdites électrodes réparties autour du thorax, par le biais d'une liaison électromagnétique, par exemple la liaison électromagnétique précitée.

Dans une telle réalisation, l'énergie nécessaire pour un choc de défibrillation est encore davantage diminué et la partie externe encore réduite au minimum, à savoir source d'énergie telle que les condensateurs et l'élément externe de la liaison électromagnétique.

De préférence, afin d'obtenir un dispositif particulièrement allégé, on peut prévoir que les condensateurs du défibrillateur soient chargés à une valeur déterminée, une simple pile suffisant alors, par l'intermédiaire d'un générateur de haute tension classique, à maintenir les capacités à la charge choisie en compensant les courants de fuite. Dans ce cas, le sujet peut avantageusement, lorsqu'il est averti par un signal que la charge des condensateurs n'atteint plus le seuil choisi, procéder au complément de charge nécessaire par l'intermédiaire, par exemple, d'un appareil indépendant branché sur le réseau ou une autre source. Un tel dispositif portatif permet de délivrer, à coup sûr, au moins un choc électrique avant d'être rechargé à partir du réseau.

Dans une autre forme de réalisation, on peut prévoir que le dispositif externe comporte une source d'énergie suffisamment importante pour pouvoir procéder elle-même à la recharge rapide des condensateurs, une telle source pouvant être avantageusement de type accumulateur.

De préférence, le stimulateur comporte un circuit électronique relié aux électrodes du défibrillateur ou à des électrodes propres, afin d'analyser les signaux électriques du coeur détectés pour en déduire la présence, s'il y a lieu, d'une tachycardie ou fibrillation. De façon avantageuse, le défibrillateur est programmable, d'une façon en soi connue, pour permettre de déclencher l'envoi d'une impulsion de défibrillation en fonction de critères déterminés à l'avance. Ces moyens d'analyse peuvent également, le cas échéant, ajuster sélectivement l'amplitude et/ou la direction spatiale et/ou les durées différentes en fonction de la nature du trouble détecté et analysé ainsi qu'en fonction de la nature et disposition des électrodes et des particularités du sujet.

Dans le cas où l'on utilise, simultanément, un stimulateur externe, il peut être avantageux d'utiliser de grandes électrodes de stimulation, de préférence également souples, qui, éventuellement, peuvent être les mêmes que les électrodes du défibrillateur. Dans le cas d'un tel stimulateur externe, on peut avantageusement prévoir des électrodes de détection disposées en des emplacements particuliers sur le thorax, ou ailleurs, de façon à être peu influencées par la contraction musculaire qui résulte de la stimulation externe, ces électrodes étant aveuglées lors de l'émission des impulsions électriques.

Les moyens de détection d'un paramètre de l'hémodynamique sont de type connu, par exemple susceptibles d'une détection dans le proche infra-rouge et/ou le rouge; lorsqu'ils sont externes, les capteurs correspondants peuvent être, par exemple, disposés au niveau du lobe de l'oreille ou au niveau d'un doigt. Ces capteurs peuvent, le cas échéant, être associés à des capteurs d'autres paramètres tels que pression partielle en oxygène et/ou CO2 et/ou d'autres paramètres métaboliques tels que pH, température, réserves alcalines, Na, K, etc... Les capteurs de ces moyens de détection sont reliés par des conducteurs convenables aux moyens d'analyse associés au défibrillateur et/ou au stimulateur.

Ce ou ces capteurs peuvent être identiques ou associés au(x) capteur(s) sensible(s) à un paramètre de l'hémodynamique utilisé(s) pour la délivrance d'une dose, tels que décrits plus haut.

Selon un perfectionnement de l'invention, la ceinture thoracique peut comporter des moyens moteurs, de préférence purement électrostatiques ou électromagnétiques, capables de réaliser un massage cardiaque externe efficace en appuyant brusquement et rythmiquement sur la partie inférieure du thorax à raison de 20 à 60 fois/mn, soit directement par raccourcissement de la ceinture, soit par action sur une protubérance sternale sus-xyphoïdienne.

Selon un perfectionnement de l'invention, le dispositif peut encore comprendre une large sangle abdominale comportant des moyens moteurs, de type quelconque, par exemple par pulsions pneumatiques ou hydrauliques, pour assurer des élargissements et rétrécissements périodiques du pourtour abdominal de façon à assurer une assistance ventilatoire diaphragmatique en cas de besoin. Un tel dipositif, créant une pression positive à l'intérieur du corps, assure normalement la désobstruction des voies aériennes supérieures si nécessaire. Dans une forme de réalisation particulièrement préférée, cette sangle peut comporter des moyens moteurs purement électrostatiques ou électromagnétiques, supprimant la nécessité de tout organe moteur ou de pompe extérieurs à la ceinture. L'énergie électrique nécessaire est de préférence fournie par un câble branché sur une source convenable. De préférence, la mise en oeuvre de cette sangle d'assistance mécanique respiratoire peut être déclenchée par la constatation d'une défaillance hémodynamique par les moyens de détection de l'hémodynamique et/ou à partir des détections des gaz du sang, oxygène et/ou CO2, et/ou par détection du rythme et/ou de l'amplitude de la respiration spontanée

Dans une forme de réalisation préférée, comme il a été dit ci-dessus, le stimulateur cardiaque peut être implanté et comporte avantageusement des électrodes endocavitaires et/ou myo- et/ou épicardiques.

De façon particulièrement préférée, le stimulateur implanté peut comporter des moyens d'analyse des signaux cardiaques afin de détecter l'existence d'une tachycardie ou d'une fibrillation, le stimulateur comportant alors des moyens pour transmettre cette information à l'extérieur vers des moyens de réception, par exemple par le biais d'une antenne sous-cutanée ou proche, qui peut avantageusement être disposée sur le boîtier du stimulateur, les moyens électroniques associés au défibrillateur étant alors sensibles à cette information et agencés pour permettre l'envoi d'un choc de défibrillation. Dans une première variante, le défibrillateur externe ne possède pas de moyens de détection et d'analyse propres des signaux électriques du coeur et est actionné par un ordre provenant des moyens d'analyse du stimulateur interne.

Dans une autre variante, le défibrillateur externe possède ses propres moyens de détection et d'analyse de l'électrocardiogramme et éventuellement de paramètres de l'hémodynamique et ne se déclenche qu'en cas de coïncidence ou accord des analyses des signaux cardiaques détectés au niveau du stimulateur et des signaux externes détectés au niveau des électrodes cutanées ou sous-cutanées. On peut ainsi éliminer de fausses détections de fibrillation ou de tachycardie dues à des causes extra-cardiaques.

Dans une variante dans laquelle les signaux cardiaques détectés par les électrodes implantées ou les analyses de ces signaux, sont également transmis à l'extérieur, de préférence par le biais d'une telle antenne, des moyens peuvent avantageusement être prévus pour comparer les signaux des électrodes implantées, par exemple endocavitaires droits, et le signaux cutanés, sous-cutanés ou juxtacardiaques en provenance du coeur de façon à déterminer, en fonction de l'importance du décalage entre le début du signal recueilli à l'extérieur du coeur, et le début du signal détecté par les électrodes implantées, par exemple le bipole de la sonde endocavitaire du ventricule droit. On peut ainsi évaluer l'emplacement d'origine, et l'intervalle de retard, des signaux électriques spontanés et du circuit de rentrée de tachycardie par rapport aux électrodes et, modifier, en conséquence, des moyens antitachycardiques pouvant être mis en oeuvre par l'appareil.

Selon un perfectionnement de l'invention, on peut avantageusement disposer, dans les cavités cardiaques et/ou le myocarde, ou bien autour du thorax, ou bien de préférence par voie de fixation épicardique, au moins trois électrodes distantes entre elles, de préférence d'au moins 4 cm, lesdites électrodes étant agencées par groupe d'au moins trois électrodes, chaque fois, dans deux ou trois plans différents, par exemple le plan horizontal, le plan vertical, et de préférence également le plan sagittal du coeur, ces électrodes détectant l'activité ou réaction électrique du coeur et étant reliées à des moyens, par exemple par le biais d'une antenne de radio-fréquence, après amplification et modulation, pour émettre à l'extérieur, vers un dispositif récepteur, les signaux électriques respectivement recueillis au niveau des différentes électrodes, lesdits signaux étant ensuite traités par un ordinateur, de préférence vectoriel, ou de type scanner, pour reconstituer l'électrogramme spatial, c'est-à-dire le vectocardiogramme tridimensionnel, des moyens étant de préférence prévus pour en former l'image tridimensionnelle visible dans l'espace.

Ainsi, l'image peut être réalisée, par exemple, sur un écran à deux dimensions par un programme usuel de conception assistée par ordinateur, l'image étant superposée à la représentation d'un coeur, de préférence avec parties vues et cachées en perspective. On peut également, à l'aide d'un tel écran, prévoir des moyens de figuration tridimensionnelle d'un type également connu, par exemple utilisant des lunettes polarisées. On peut également utiliser les systèmes holographiques. Une autre possibilité consiste à réaliser, à l'intérieur d'un cube en matière plastique transparente, un modèle de coeur transparent et, par l'envoi de faisceaux lumineux de préférence monochromatiques ou à laser, reconstituer l'image par visualisation du point de croisement de deux faisceaux, selon une technique en elle-même également connue et développée, par exemple, par la société américaine General Electric.

Ainsi, les circuits d'activation cardiaque deviennent immédiatement visibles sur une telle projection lumineuse à l'intérieur d'un coeur transparent servant de modèle optique ; en cas de circuit ectopique générateur ou précurseur de tachycardie, l'influence d'une stimulation orthorythmique selon son intervalle temporel et sa localisation et/ou sa direction spatiale peut être contrôlée en direct et la stimulation modifiée pour interrompre le circuit de dépolarisation de la tachycardie. Ce dispositif permet également de visualiser en direct et en représentation spatiale les phénomènes qui mènent à la fibrillation ventriculaire ou à la fibrillation auriculaire et l'effet de stimulations multiples et/ou tournantes selon leurs paramètres temporels ou spatiaux, et peut par ailleurs guider une thérapie par électro-laser-micro-ondes ou ultrasonofulguration locale.

Les points de repère permettant d'acquérir et d'ajuster spatialement, sur le modèle, l'image des dépolarisation, activation et repolarisation cardiaques spontanées ou provoquées, ainsi que le déroulement et la propagation des stimulations artificielles cardiaques, sont constitués par lesdites électrodes fixes, endo-, intra et/ou extra-cardiaques, notamment épicardiques, qui permettent de cadrer la visualisation spatiale intra-cardiaque des origines et propagations de l'électrogénèse et de la stimulation artificielle, qu'elle soit efficace ou non, du coeur à l'intérieur d'un tel modèle d'imagerie tridimensionnel du coeur observable sous toutes les incidences. Ce modèle du coeur, par exemple un coeur transparent déformable à double paroi en matière plastique, peut être agencé pour reproduire les anomalies géométriques ou fonctionnelles du coeur naturel telles qu'elles ont pu être détectées par le cardiologue, par exemple en déformant localement, par fluide ou mécanisme, une ou des parois du modèle de coeur, ou par simulation par l'intermédiaire de l'ordinateur.

Dans une autre forme de réalisation de l'invention, on peut prévoir de disposer d'une pluralité d'électrodes intra-cardiaques et/ou épicardiques réparties au niveau ventriculaire, notamment gauche. Le dispositif, par exemple le microprocesseur du stimulateur, auquel ces électrodes sont subordonnées, provoque périodiquement, par exemple toutes les minutes, ou même à chaque cycle, l'acquisition des électrocardiogrammes régionaux entre différentes combinaisons de ces électrodes, d'une part, et la mesure de l'impédance myocardique locale par envoi de micro-courant (par exemple à 6 kHz) entre, de préférence, les mêmes combinaisons d'électrodes, mesurée de préférence aux intervalles suivants: dépolarisation et repolarisation détectées par l'électrocardiogramme local, et contractions mécaniques maximale et minimale détectées par capteur de pression ou conductance, d'autre part. Lorsqu'on détecte simultanément une modification importante (notamment diminution d'amplitude) de l'électrocardiogramme local et une diminution notable de l'impédance myocardique de la même région, on en déduit l'existence d'une accumulation locale de liquides et d'ions K+, traduisant une ischémie locale grave. Dans ce das, le dispositif déclenche une alarme extérieure et/ou une perfusion automatique de thrombolytiques ou autres médicaments.

Dans un mode de mise en oeuvre préféré de l'invention, on peut prévoir un détecteur permanent de pression statique et/ou dynamique, à l'intérieur d'un ventricule, de préférence le ventricule gauche, ou encore dans une des parois myocardiques en rapport avec le ventricule gauche et/ou droit.

Dans le cas où l'on utilise des électrodes endocavitaires dans le ventricule droit, on peut avantageusement associer au cathéter portant les électrodes, ou séparément de lui, des moyens permettant la mise en place d'un capteur de pression endocavitaire ventriculaire gauche ou intramyocardique ventriculaire gauche ou septale. De tels moyens sont par exemple décrits dans le brevet US-A-3.857.399. Les capteurs utilisés sont actuellement fabriqués par l'industrie spécialisée.

On peut également se contenter d'un capteur de pression dans le ventricule droit, fixé par exemple sur le cathéter porte-électrodes du stimulateur. On peut avantageusement placer, à la fois, un capteur de pression dans le ventricule droit et un autre dans ou près du ventricule gauche.

Dans un mode de mise en oeuvre particulièrement préféré, le stimulateur, qu'il soit externe ou de préférence implanté, peut être du type orthorythmique (POR), c'est-à-dire permettant un traitement automatique des tachycardies, tel que notamment décrit dans les susdits brevets US-A-3.857.399 et 4.052.991. Une telle combinaison présente, en outre, l'avantage d'assurer la défibrillation automatique de toute fibrillation qui serait induite par un traitement de stimulation automatique anti-tachycardique. De plus, cette combinaison permet, par la prévention qu'elle assure des tachycardies ventriculaires ou rapides, d'éliminer un grand nombre de risques de tachycardies dangereuses pouvant dégénérer en fibrillation, de sorte que le défibrillateur sera nettement moins sollicité, permettant l'utilisation, de préférence, d'un défibrillateur ne comportant qu'une petite pile d'entretien du courant de fuite des condensateurs.

Ces préventions de tachycardies peuvent être effectuées notamment par les moyens suivants :
- traitement sélectif des extra-systoles isolées ou pairées par déclenchement d'une stimulation consécutive à un rythme temporairement plus rapide, pilotée par l'extra-systole elle-même, pour supprimer l'arythmie extra-systolique souvent précurseur d'une tachycardie (voir notamment le brevet US-A-3.857.399),
- en cas d'apparition d'une tachycardie détectée sur un certain nombre de cycles consécutifs, le dispositif procède à une stimulation à une féquence déterminée, généralement supérieure, pour faire cesser la tachycardie (brevet US-A-4.052.991) ; la stimulation en rafale peut être, par exemple, à fréquence progressivement accrue ("ramp" en anglais),
- prévention d'une tachycardie non précédée d'extra-systoles (ou précédée d'extasystoles non utilisables pour la prévention) ; dans ce cas on procède à la détection périodique automatique du seuil d'excitabilité par envoi d'impulsions préférentiellement infra-liminaires de préférence à amplitude variable (brevet US n^{o} 4.552.150, ZACOUTO), de préférence en au moins deux points différents du coeur.

Le stimulateur peut également avantageusement comporter un dispositif destiné à maintenir une bonne hémodynamique par le myocarde en cas de tachycardie. Un tel dispositif comporte, outre les moyens de détection du rythme cardiaque et des moyens de stimulation, par exemple les moyens de stimulation du stimulateur, et une souce d'énergie, des moyens sensibles au rythme cardiaque pour détecter une tachycardie, des moyens pour adresser au muscle cardiaque une stimulation électrique pairée, ou couplée de façon similaire, à un évènement périodique du cycle cardiaque, des moyens de détection périodique (non obligatoirement à chaque cycle cardiaque) d'un phénomène cardiaque lié à la contraction maximale et/ou au métabolisme du muscle cardiaque et des moyens électroniques sensibles auxdits moyens de détection périodique du phénomène cardiaque et susceptibles de modifier le couplage de la stimulation et/ou d'arrêter ladite stimulation, en fonction de ladite détection.

Dans un autre mode de mise en oeuvre de l'invention, nécessitant cette fois-ci une intervention par thoracotomie large, dans le cas d'un muscle cardiaque présentant des déficiences mécaniques et/ou hémodynamiques importantes, on met en place autour du muscle cardiaque, au niveau des ventricules, une ou plusieurs bandes de préférence autocontractiles, de préférence à fonctionnement électrostatique, électromagnétique ou fluidique, afin d'empêcher, au moins dans un premier temps, une dilatation progressive des cavités cardiaques correspondant à un étirement des fibres myocardiques. A cet effet, une ou plusieurs bandes sont fixées ou cousues sur l'épicarde. Si le simple empêchement de la dilatation des cavités cardiaques reste insuffisant, on peut mettre en oeuvre des bandes contractiles de façon à provoquer, de préférence en synchronisme avec les contractions spontanées, une activité mécanique de contraction rythmique complémentaire de celle du coeur.

De préférence, la bande autocontractile peut présenter des segments introduits à l'intérieur du myocarde, et notamment du myocarde ventriculaire gauche lui-même, ces segments pouvant être des segments minces non-autocontractiles.

De préférence, les bandes portent, au moins, sur leur face cardiaque, une pluralité d'électrodes pouvant servir à la détection et/ou à la stimulation cardiaque, par exemple orientée à volonté, et/ou à la défibrillation, les électrodes pouvant être placées également au niveau des segments intramyocardiques non autocontractiles et disposées pour pouvoir fonctionner à volonté en champs fixes ou tournants, soumettant ainsi presque tout le myocarde ventriculaire à l'effet d'un champ électrique dépolarisant. Une bande circulaire au niveau de l'apex cardiaque, portant également une série d'électrodes sur toute sa circonférence, peut venir utilement compléter les actions électriques et/ou autocontractiles décrites. Les électrodes en question peuvent également avantageusement être utilisées pour fournir, comme décrit ci-dessus, les informations à un récepteur extérieur permettant de reconstituer une image tridimensionnelle de toute propagation électrique détectable dans le muscle cardiaque.

En outre, ces bandes, du fait qu'elles suivent la déformation du muscle cardiaque, peuvent être utilisées pour fournir à l'extérieur des renseignements permettant de reconstituer sensiblement la géométrie statique et dynamique du muscle cardiaque dans ces différents segments. On peut, en effet, utiliser les électrodes pour pratiquer des mesures, en soi connues, par exemple d'impédance du muscle cardiaque qui varient proportionnellement à leurs éloignements respectifs. On peut également prévoir, sur les bandes, des détecteurs quelconques, par exemple jauges de contrainte, permettant d'obtenir une image de la déformation géométrique des bandes. On peut également utiliser les éléments contractiles électrostatiques ou électromagnétiques de ces bandes, lorsqu'elles sont constituées de tels éléments, pour obtenir une vue de la déformation géométrique des bandes par détermination de l'éloignement des différents éléments les uns par rapport aux autres.

Si nécessaire, on peut en outre prévoir de mettre en place par un passage apical au niveau du ventricule gauche en tube, tel que décrit dans le brevet français FR-A-1.514.319 et sa première addition 97075, mis au point sur le chien par l'inventeur, des moyens pour améliorer, voire remplacer, la fonction hémodynamique du coeur. A cet effet, on peut par exemple brancher, sur ce tube transapical gauche, un réservoir contractile jouant le rôle d'un ventricule artificiel. Il peut ne comporter qu'un seul orifice pour aspirer et refouler à travers le ventricule gauche, ou bien au contraire constituer une dérivation ventriculo-aortique.

Dans une forme de réalisation avantageuse, le dispositif peut comporter un ou plusieurs microphones miniaturisés, de préférence endocavitaires, tels qu'ils sont maintenant couramment utilisés en cathétérisme. Un premier avantage est d'obtenir, d'une façon en soi connue, le phonocardiogramme statique et dynamique, pour contrôler automatiquement la fonction cardiaque, ou l'adresser à un moniteur externe. Cependant, il est particulièrement avantageux d'associer au microphone, passif, des moyens permettant de générer, soit par le microphone lui-même s'il est agencé à cet effet, soit par un émetteur sonore ou ultra-sonore convenable, des sons à des fréquences destinées à explorer la ou les fréquences de vibration de la masse sanguine circulant en systole ou diastole, et notamment au moment de l'éjection systolique du ventricule gauche, moment auquel le microphone placé dans ce ventricule ou à proximité se trouve en communication directe avec la totalité de la masse sanguine artérielle.

On peut prévoir, par exemple, de faire balayer par l'émetteur sonore un certain spectre sonore à la recherche de la fréquence de résonance maximale, qui est alors captée par le microphone et retransmise à l'extérieur ou au microprocesseur . Le spectre sonore ainsi obtenu, présente la fréquence de résonance artérielle dynamique qui est notamment proportionnelle à la pression sanguine maximale et à son décours. On peut avantageusement procéder à un étalonnage de ce spectre sonore par une sphygmomanométrie classique au brassard, faite soigneusement, et ceci pour plusieurs pressions différentes induites, par exemple par action de différentes drogues, en tenant compte des rythmes cardiaques fixés ou non par action du stimulateur.

On obtient ainsi des valeurs que l'on peut associer à des tensions artérielles, le fonctionnement du stimulateur, et, si présents, des bandes ou ventricules artificiels étant alors agencé de façon à maintenir automatiquement la tension et/ou le débit entre des limites supérieure et inférieure de tension et/ou de débit déterminées à l'avance.

La dose d'agent thérapeutique peut être libérée de façon à diffuser dans le sang mais on peut aussi fixer, de façon irréversible, l'agent thérapeutique sur un support solide, par des procédés connus, le support étant disposé dans la circulation ou près, par exemple dans un vaisseau sanguin.

Par exemple, le support peut être soumis à des champs électriques adaptés aux molécules actives en fonction de la valeur du paramètre détecté ou mesuré.

A titre d'exemple, dans le cas où le paramètre détecté renseigne automatiquement sur l'état de la coagulatibilité, le support peut présenter au voisinage ou à distance des facteurs anticoagulants (par exemple antiprotéines spécifiques, héparine sulfate, human leuserpin II, dermatane sulfate, antivitamines K, protéines S, facteur AT III, thromboglobuline, anti-inhibiteur spécifique de la protéine C) et/ou fibrinolytiques, notamment par stimulation de la libération de l'activation tissulaire du plasminogène naturel grâce à l'inactivation de son inhibiteur (TpAi), tel que la protéine C, des molécules TpA activées, des inhibiteurs des antiplasmines, facteur XII et kallicréine etc...

On peut, au lieu ou en plus de champs électriques, prévoir des moyens mécaniques permettant de replier ou déplier le support, lequel peut être agencé de façon à présenter par des moyens connus, un rapport surface/volume très élevé.

Des moyens sont prévus pour éviter le dépôt de substances colmatantes tels que fibrine ou pour dissiper périodiquement ces substances, par exemple par ultrasons.

Toutes sortes d'autres substances actives peuvent être fixées sur un tel support, par exemple immunomodulateurs, anticorps, vaccins, et d'une façon générale, toute activité thérapeutique orientée vers des cibles circulantes et agissant par des mécanismes ne nécessitant pas leur consommation, par exemple des mécanismes enzymatiques de catalyse ou de replication ou de traduction génétique. Les supports peuvent être, par exemple,réalisées en nitrocellulose ou en silice ou siloxanes, la fixation des molécules s'effectuant par des moyens connus, par exemple ceux utilisés pour les tests immunologiques ou la chromatographie d'affinité.

D'autres caractéristiques de l'invention apparaîtront à la lecture de la description suivante, faite à titre d'exemple non limitatif et se référant au dessin annexé dans lequel :
- la figure 1 représente une vue schématique d'un dispositif selon l'invention,
- la figure 2 représente une vue schématique des moyens implantés de mesure et de comparaison de ce dispositif,
- la figure 3 représente une vue schématique de l'électrocardiogramme et de la courbe de mesure des impédances électriques,
- la figure 4 représente une vue schématique de moyens de mesure de paramètres myocardiques,
- les figures 5 et 6 représentent des vues schématiques en coupe transversale et axiale d'un capteur sensible à l'état de la crase sanguine,
- la figure 7 représente schématiquement un dispositif selon un perfectionnement de l'invention,
- la figure 8 représente un schéma de la partie implantée,
- la figure 9 représente un schéma de la partie externe,
- la figure 10 représente un schéma d'une sangle abdominale,
- les figures 11 et 12 représentent des schémas de moyens électrostatiques ou autocontractiles,
- la figure 13 représente une vue de bandes épicardiques,
- la figure 14 représente une vue schématique d'un capteur de pression selon l'invention.

En se référant à la figure 1, on voit que le dispositif selon l'invention, totalement implanté dans l'organisme, comporte, dans un boîtier implantable 1, analogue à celui d'un stimulateur cardiaque implantable, un ensemble qui sera détaillé par la suite. A partir du boîtier 1, un cathéter veineux complexe 2 pénètre par le ventricule droit 3 dans l'apex. Dans le ventricule, et éventuellement dans l'oreillette, il possède un certain nombre d'électrodes de détection, respectivement de stimulation reliées par des conducteurs électriques dans le cathéter 2 à un stimulateur cardiaque contenu dans le boîtier 1. A partir de la dernière électrode 4, le cathéter 2 se poursuit par un tube 5 qui, par voie transmyocardique sur le trajet 6, rejoint la surface du muscle cardiaque pour se terminer par une canule 7 pénétrant dans une artère coronaire 9. Le tube 5 est contenu dans une gaine qui contient de préférence un certain nombre de conducteurs, 10,11 qui émergent de la gaine et qui rejoignent respectivement des électrodes 14,15 portées intérieurement par des bagues 16,17 de préférence élastiques qui ont été mises en place autour de l'artère coronaire 9 par abord chirurgical thoracique ou épigastrique, le diamètre interne des bagues 16,17 avec leurs électrodes internes étant suffisamment grand pour ne pas risquer de comprimer ou sténoser l'artère. La mise en place de la canule 7 dans la coronaire 9 s'effectue lors de la même intervention.

On peut avantageusement implanter une troisième électrode 14a située à l'écart de la ligne joignant les électrodes 14, 15, les distances entre les électrodes étant, par exemple, de l'ordre de 5 mm. On peut alors mesurer successivement les impédances entre chacun des trois couples d'électrodes 14, 15, 14a, les mesures étant de préférence adressées aux moyens d'exploitation du boîtier 1 par multiplexage. Le boîtier 1 fournit aux électrodes les courants de mesure d'impédance, par exemple un courant continu de 25 microampères.

On comprend donc que le cathéter 2, qui est un cathéter complexe, comporte tous les électrodes et conducteurs d'un cathéter classique pour la détection et la stimulation par voie endocardique droite et, en plus, le tube 5 avec les conducteurs qui lui sont associés et qui vont vers les moyens d'électrodes 14,15. L'extrémité du tube 5 est raccordée, par une microvanne convenable, à une pompe miniaturisée susceptible de débiter des doses déterminées d'un médicament tel qu'un thrombolytique, par exemple un dérivé de l'urokinase ou de la streptomicine.

Dans une autre variante de l'invention ne nécessitant pas d'intervention chirurgicale cardiaque, mais simplement une intervention endoartérielle, le tube 5 peut être omis, de même que les bagues 16,17, le cathéter 4 étant un cathéter classique de stimulateur. Dans cette forme de réalisation, on vient introduire, en un ou plusieurs emplacements de l'artère coronaire choisie, à l'aide d'un cathéter artériel introduit, par exemple, par l'artère fémorale, une prothèse endocoronaire, selon le procédé existant qui vient se plaquer contre la paroi interne de l'artère coronaire, cette prothèse, par exemple de forme circulaire ou tubulaire, présentant des éléments conducteurs formant électrodes de façon à pouvoir déterminer au moins deux électrodes entre lesquelles il sera possible de mesurer l'impédance du sang du segment d'artère disposé entre les deux électrodes. On peut prévoir, le cas échéant, plus de deux électrodes espacées le long de l'artère et mesurer les impédances en plusieurs segments le long de l'artère. Les électrodes, ainsi disposées sur les anneaux de prothèses endocoronariennes, sont reliées par des fils conducteurs respectifs très fins et souples, de préférence regroupés, et s'étendant le long d'un trajet artériel jusqu'à un point de sortie, par exemple au niveau d'une branche de l'artère fémorale, pour être reliés, toujours en restant implantés par voie sous-cutanée, jusqu'aux moyens électroniques disposés dans le boîtier 1.

Dans une variante préférée, les électrodes artérielles, telles que 14, 15, peuvent être alimentées par une source d'énergie autonome ou alimentées par transmission haute fréquence ou hertzienne depuis un émetteur intracorporel, par exemple situé au niveau du boîtier du stimulateur cardiaque. De même, les valeurs significatives de la mesure d'impédance seront adressées à un récepteur intracorporel selon des procédés connus.

En se référant à la figure 2, on voit très schématiquement la partie du dispositif contenue dans le boîtier 1. Celui-ci comporte un stimulateur orthorythmique 20 antitachycardique qui peut être avantageusement du type décrit dans les brevets US-A-3 857 399 et 4 052 991. A ce stimulateur 20 peut, le cas échéant, être associé un défibrillateur entièrement automatique tel que décrit dans le brevet français Zacouto du 11 juillet 1953 n^{o} 1 237 702 et implantable tel que décrit dans le brevet français Zacouto n^{o} 74 01383.

Le boîtier 1 incorpore également une source d'énergie électrique, par exemple des piles au lithium, et/ou des moyens de transmission périodique transcutanée d'énergie pour recharger des piles ou accumulateurs ou condensateurs. Les piles modernes utilisées dans les stimulateurs et défibrillateurs implantables permettent de faire face pendant un certain temps, suffisant pour que le patient ait une indépendance entre les périodes de recharge ou de remplacement, même dans le cas où la consommation électrique est assez forte, par exemple pour l'actionnement d'émetteurs ultrasonores. On peut aussi utiliser des piles isotopiques implantables connues.

Ce stimulateur 20 est articulé, d'une façon connue, autour d'un organe logique tel qu'un microprocesseur, associé à des moyens de détection et des moyens de stimulation selon le procédé décrit dans les brevets US précités. Ces moyens permettent, en particulier, d'acquérir l'électrocardiogramme au niveau des électrodes endocavitaires droites 4 et d'une électrode extracardique, par exemple sur le boîtier 1. L'un des éléments de l'électrocardiogramme (ECG) ainsi obtenu est le rythme instantané de l'électrocardiogramme, c'est-à-dire la période séparant deux complexes QRS.

Le stimulateur 20 comporte les moyens antitachyarythmiques automatiques décrits dans lesdits brevets Zacouto et est sensible aux troubles du rythme, en détectant leur fréquence et, de préférence, la morphologie des signaux électriques spontanés recueillis dans au moins une cavité ou paroi cardiaque. En cas de détection de signaux reconnus dangereux, le stimulateur 20 délivre automatiquement une stimulation électrique conformément auxdits brevets, notamment en augmentant le rythme de la stimulation.

Selon un perfectionnement de l'invention, le cathéter 2, en dehors du tube 5 et des conducteurs qui lui sont associés et qui sont destinés aux électrodes juxta- ou endocoronaires, peut être conçu comme celui décrit dans le brevet US-A-4 754 753, avec, dans le boîtier 1, les moyens décrits dans ce brevet permettant d'acquérir, à chaque pulsation cardiaque, l'électrovectocardiogramme.

D'une façon schématique, le dispositif contenu dans le boîtier 1 présente encore un générateur ou source 30 d'impulsions carrées ou sinusoïdales ou autres, voire une source de courant continu, susceptible d'adresser dans les circuits constitués par les paires de conducteurs 10,11, une succession d'impulsions carrées. La durée d'un train d'impulsions est, par exemple, de 30 ms, la fréquence de 5 kHz à 25 MHz et l'intensité de l'ordre de 10 à 30 ou 40 microampères. Un circuit de mesure d'impédance (ou conductance) 40 est électriquement associé à la paire de conducteurs en provenance du couple d'électrodes coronaires 14,15 (et éventuellement des couples 14, 14a et 15, 15a) de façon à mesurer l'impédance au cours des émissions d'impulsion de courant en provenance de la source 30, le circuit 40 étant accordé à la fréquence correspondante.

Le générateur 30 et le circuit 40 peuvent éventuellement être pilotés à partir du stimulateur cardiaque 20, en fonction de la fréquence de l'électrocardiogramme.

Le circuit 40 comporte des moyens, pouvant d'ailleurs utiliser un microprocesseur, agencés de façon à calculer la différence des impédances maximale et minimale d'un cycle et à adresser le résultat de cette mesure à un moyen de comparaison, microprocesseur ou comparateur 50 à la seconde entrée duquel est affichée une valeur ou fourchette de seuil à partir d'un potentiomètre de seuil réglable 31. La sortie du comparateur est adressée à un circuit de commande de pompe 60 qui, lorsqu'une impulsion provient du comparateur 50, actionne le moteur de la pompe contenue dans le boîtier 1 et provoque la délivrance par le tube 5 d'une dose de médicament dans la coronaire concernée.

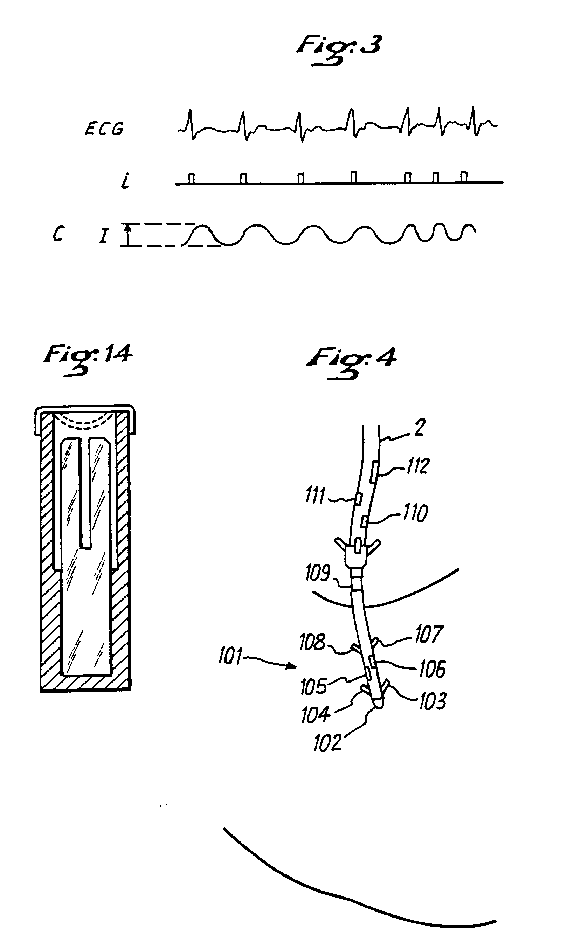

En fonctionnement normal, le fonctionnement est le suivant et l'on se reportera à la figure 3.

La source adresse aux électrodes 14, 15, des impulsions et le circuit 40 correspondant mesure l'impédance entre les électrodes 14, 15. On obtient ainsi une courbe C représentée sur la figure et montrant le décours de la vatiation d'impédance. Le circuit 40 est en outre sensible aux cycles électriques cardiaques tels qu'il sont détectés par le stimulateur cardiaque, ces cycles étant matérialisés par la succession des complexes QRS de l'électrocardiogramme qui sont transformés en impulsions i par le stimulateur et adressés sous cette forme au dispositif 40. Le circuit 40 est en même temps sensible à la différence I entre la valeur la plus grande et la valeur la plus petite de l'impédance mesurée pendant un cycle. La différence I entre ces deux valeurs dans un même cycle est adressée au moyen de comparaison 50 qui la compare aux seuils programmés et, en cas de franchissement du ou des seuils, le circuit 60 de commande de la pompe est actionné.

La valeur de seuil à laquelle on compare la différence d'impédance I peut être fixe ou, le cas échéant, programmée pour tenir compte de la durée et de l'origine d'un ou de plusieurs cycles précédents. En remplacement, si l'on maintient un seuil fixe ou en tout cas indépendant des cycles, on peut avantageusement prévoir un circuit d'inhibition empêchant l'actionnement du circuit 60 lorsque, par exemple, le cycle précédant le cycle en cours était anormalement court.

Ainsi, par exemple, lorsque le dispositif de détection du stimulateur détecte une tachycardie dépassant un certain seuil de fréquence, par exemple 150 pm, le microprocesseur du stimulateur inhibe le dispositif de commande 60 de la pompe de façon à éviter l'envoi programmé de médicament même si la différence entre impédance maximale et minimale par cycle est descendue au-dessous du seuil.

De même, ces moyens d'inhibition peuvent être agencés pour inhiber le dispositif pendant la durée d'un cycle normal précédé d'un cycle court et/ou ectopique.

En variante, le dispositif peut être perfectionné de façon à ne pas commander le circuit de commande 60 de la pompe lors de la première détection d'une diminution de la différence I au-dessous de la valeur de seuil mais au contraire il peut être agencé de façon à n'activer ces moyens que si cet abaissement est confirmé sur un ou plusieurs cycles consécutifs ou successifs.

L'invention permet également de distinguer et, si on le désire, de traiter différemment les spasmes coronariens et les thromboses coronariennes. Ceci est obtenu en prévoyant des moyens de contrôle de l'électrocardiogramme, en soi connus, sensibles aux modifications de l'ECG induites par l'ischémie. L'invention a permis, en effet, de découvrir qu'en cas d'ischémie par spasme, l'ECG peut être modifié avant la modification de l'impédance (modification de I) alors qu'en cas de thrombose, l'impédance est souvent modifiée avant l'ECG. Le dispositif est alors agencé de façon à être sensible à l'ordre de survenue de ces phénomènes ; par exemple en cas de modification de l'ECG sans modification de la mesure d'impédance le dispositif surveille si une modification d'impédance survient dans les 10 cycles suivants. Dans ce cas, le dispositif peut être agencé pour délivrer une dose de trinitrine ou autre vaso-dilatateur immédiat. Si au contraire le dispositif détecte une modification de l'impédance sans modification préalable de l'ECG, il surveille si l'ECG est suivi d'une modification tardive de l'impédance (par exemple après les 10 cycles suivants), il délivre une dose de thrombolytique.

L'acquisition des cycles, au lieu d'être obtenue par détection de l'électrocardiogramme, peut également être obtenue par d'autres moyens, par exemple par capteur de pression intracavitaire et/ou intramyocardique et/ou artérielle.

De préférence, le dispositif peut être agencé de façon à pouvoir être réglé par le médecin qui pourra choisir soit entre l'envoi d'une dose à chaque détection de franchissement de seuil sous réserve des conditions d'inhibition précitées, ce qui présente l'avantage d'une médication instantanée, d'action particulièrement rapide, soit après l'acquisition d'un certain nombre d'abaissements successifs au-dessous du seuil, ce qui présente l'avantage de n'intervenir qu'en cas d'anomalie pratiquement certaine du débit coronaire.

Le dispositif selon l'invention peut également comporter des moyens, réalisés par le microprocesseur programmé de façon convenable, pour être sensibles non seulement à la différence entre les valeurs maximum et minimum d'impédance mesurées et à leur décours au cours d'un cycle mais également au décalage des maximum et minimum par rapport au début du cycle. Il peut également être sensible à des paramètres liés à ce décalage tels que l'intégration ou la dérivation mathématique de la courbe de mesure d'impédance. Dans cette forme de réalisation, le dispositif peut avantageusement comporter un seuil de décalage qui, s'il est franchi, c'est-à-dire si l'intervalle temporel entre le début du cycle et le maximum et/ou le minimum d'impédance dépasse cette valeur de seuil, actionne le circuit de contrôle de pompe 60, à moins que celui-ci ne soit inhibé pour une autre raison. Le début du cycle cardiaque peut être déterminé par un phénomène électrique, par exemple complexe QRS, ou par un ature phénomène cardiaque tel que bruits du coeur ou un point précis du mécanogramme de pression du coeur.

Dans une autre forme de réalisation de l'invention, de préférence combinée à celle qui vient d'être décrite, on peut également introduire, dans la coronaire, des capteurs piézo-électriques miniaturisés, à savoir au moins un capteur, et de préférence plusieurs capteurs, par exemple, placés sur une électrode de type anneau, répartis le long de la coronaire lorsque cela est possible. On peut ainsi obtenir, par cycle, la courbe de pression en un ou plusieurs endroits de la coronaire et, en association avec la mesure d'impédance si elle est prévue, obtenir, en effectuant le produit de l'impédance et de la pression, une grandeur représentative du rapport débit/pression dans les coronaires.

La courbe de pression dans l'artère coronaire peut être exploitée par le microprocesseur d'une façon analogue à celle de la courbe d'impédance. En particulier, le dispositif peut être sensible à des changements du décours de la courbe de pression ou à des décalages temporels du décours de la courbe de pression par rapport au cycle électrique ainsi qu'au croisement des courbes de pression de flux et de reflux sanguin dans les artères coronaires.

La mesure de pression peut être effectuée par quartz par exemple d'horlogerie dont la fréquence de vibration propre est modulée par la pression. Le quartz est enfermé dans une enceinte fermée proche et rigide comportant une partie déformable de façon à transmettre au gaz contenu dans l'enceinte, les variations de pression extérieures (sang ou myocarde). Ces variations de pression ainsi provoquées se traduisent par des variations, qui sont détectées, de la fréquence propre du quartz modifié par la résistance mécanique opposée par le gaz (voir figure 14).

En se référant maintenant à la figure 4, on voit une autre forme de réalisation de l'invention dans laquelle la partie du dispositif contenue dans l'enveloppe 1 est sensiblement identique à celle décrite ci-dessus. Par contre, le cathéter 2 ne se prolonge pas par un tube traversant le myocarde pour rejoindre une ou plusieurs artères coronaires.

L'extrémité 101 du cathéter 2 pénètre sur une courte distance dans le myocarde au fond du ventricule droit vers l'apex du coeur. La distance d'enfoncement peut être de l'ordre, par exemple, de 4 à 8 mm. Cette extrémité 101 comporte successivement, depuis la pointe, un capteur de pression 102 puis, de part et d'autre du cathéter, deux électrodes 103, 104, venant légèrement s'écarter dans le myocarde pour former un point d'ancrage en forme de V. Le cathéter présente ensuite des capteurs à ions potassium et ions H+ 105, 106 puis à nouveau deux électrodes 107, 108 s'ouvrant en V pour l'ancrage.

Dans le ventricule, le cathéter peut avantageusement comporter, outre différentes électrodes, un capteur de pression 109 ainsi que d'autres capteurs 110, 111, 112 par exemple pour détecter, dans le sang, les ions potassium, les ions H+ et au moins un paramètre relatif à la coagulabilité sanguine, notamment du plasma sanguin et/ou des éléments figurés du sang.

Les deux électrodes 103 et 107, séparées entre elles d'une distance de quelques millimètres, sont reliées à un générateur d'impulsions tel que le générateur 30 et un moyen de mesure d'impédance entre ces électrodes 103 et 107 tel que le circuit 40. Les électrodes 104 et 108 sont destinées à détecter entre elles l'électrocardiogramme local dans cette zone du myocarde. On peut ainsi obtenir localement à la fois l'impédance électrique, l'électrocardiogramme et le mécanogramme de pression myocardique.

Dans la partie du cathéter pénétrant dans le myocarde, on peut également placer un détecteur optique sélectif discriminant l'hémoglobine de la myoglobine et/ou la myosine, et NADH pour détecter une éventuelle ischémie.

Le dispositif peut alors être agencé, comme précédemment, de façon à comparer aux seuils programmés la différence entre les impédances minimum et maximum au cours d'un cycle cardiaque déterminé par l'électrocardiogramme bipolaire local, et/ou la forme de la courbe d'impédance correspondante et/ou le décalage temporel du pic ou du minimum d'impédance. Il peut également prévoir des seuils auxquels il compare les valeurs maximales et minimales de pression pendant un cycle cardiaque, le déroulement de la courbe de pression ainsi que l'éventuel décalage d'un pic ou d'un minimum de pression par rapport au cycle cardiaque.

Il peut, en outre, être sensible à la diffusion anormale d'ions potassium ou à une variation anormale du pH.

L'invention peut, bien entendu, faire l'objet d'un certain nombre de variantes. En particulier, les moyens permettant au niveau d'une artère, telle que par exemple une coronaire ou une collatérale coranarienne, de détecter le passage ou la stagnation du débit sanguin peuvent être différents.

En restant dans le domaine des mesures d'impédance électrique, on peut utiliser des électrodes intra-artérielles au lieu d'être disposées autour de l'artère. Ces électrodes peuvent être, par exemple, constituées de simples fils conducteurs introduits dans l'artère, par exemple au moment de la réalisation d'un pontage coronarien ou par sonde endartérielle.

En cas d'implantation d'un coeur artificiel, (réalisée pour la première fois avec succès chez l' animal par l'inventeur en 1954/55), la protection des artères, notamment carotides et cérébrales peut être particulièrement importante et les tests de provocation de la coagulabilité et de l'incoagulabilité du sang ainsi que les tests d'hémorragie vasculaire et de formule sanguine seront surveillés par le dispositif.

On peut également utiliser des moyens électriques sensibles au flux sanguin autres que la mesure d'impédance et, de façon non limitative, on pourrait par exemple prévoir un émetteur et un capteur à ultrasons fonctionnant par exemple sur effet Doppler ou des moyens de mesure de durée de transit ultrasonore, ou bien encore un capteur optique avec une source lumineuse.

Au lieu d'utiliser un moyen de détection sensible au flux sanguin immédiatement en place, on peut également utiliser des moyens de détection du vectocardiogramme comme décrit dans le brevet US 4 754 753 de façon à acquérir l'origine et les vecteurs de la propagation électrique tant en dépolarisation qu'en repolarisation. On sait que l'étude de la variation de ces vecteurs permet de déterminer, au moins de façon approximative, la zone myocardique dans laquelle une ischémie se produit. En cas de variations des vecteurs relatifs à cette zone au-delà d'un certain seuil de variation, le dispositif produit une libération d'une dose de médicament dans la circulation endocavitaire, ou directement dans un vaisseau coronaire ou dans les trois coronaires principales pouvant être reliées pour un envoi sélectif de médicament.

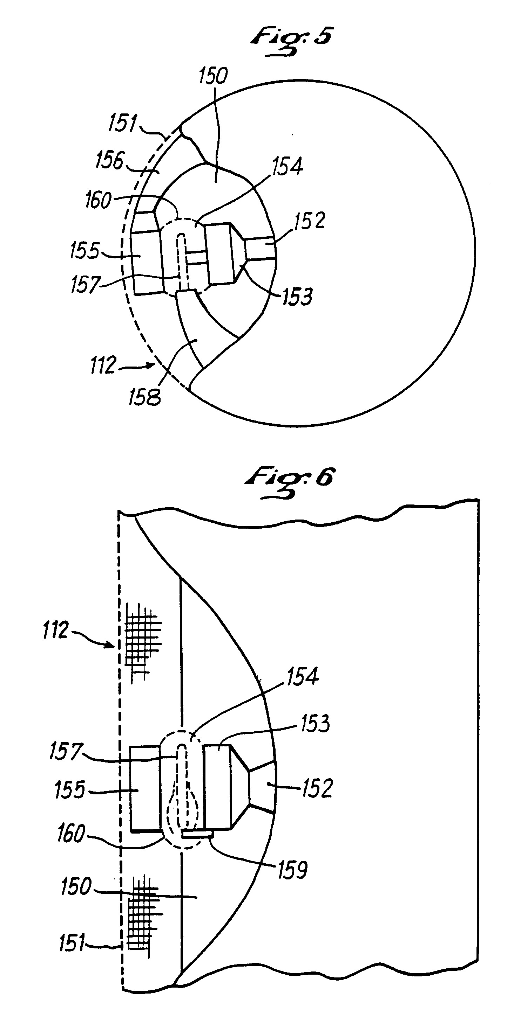

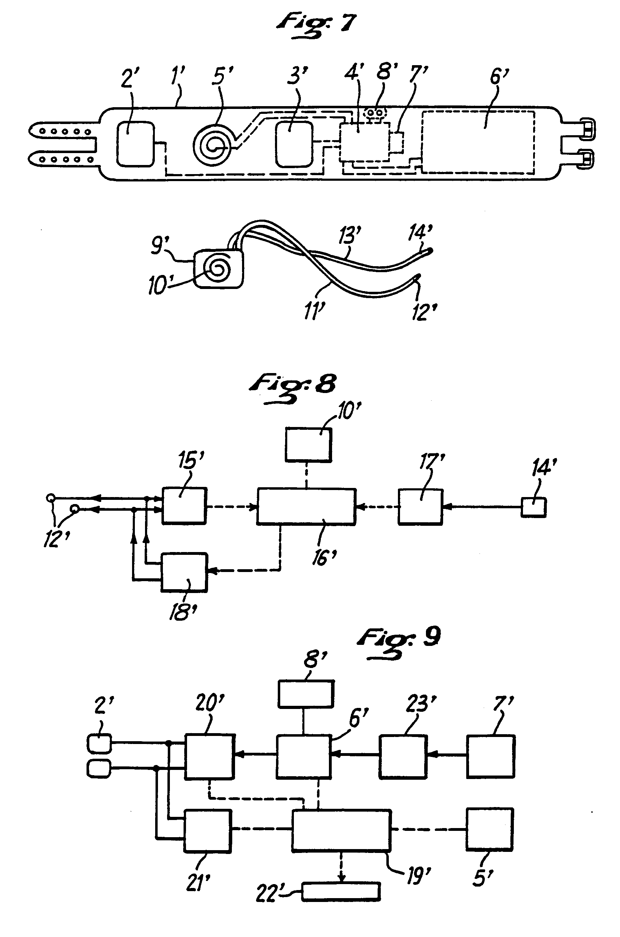

En se référant aux figures 5 et 6, on voit une forme de réalisation d'un capteur tel que le capteur 112, destiné à mesurer par voie optique les variations d'épaisseur d'un dépôt de fibrine. Le capteur 112 est disposé dans une cavité 150 du cathéter 2. Sur le dessin, le cathéter 2 présente un diamètre de l'ordre de 3,5 mm. La cavité 150, convenablement profilée, est obturée à l'extérieur par une grille en acier perforée d'une épaisseur, par exemple de 3/10 mm et dont les trous de mailles ont un diamètre de préférence supérieur à 1/10 mm et inférieur à 1 ou 2 mm. Une bonne taille peut être, par exemple de l'ordre de 0,8 mm. Cette grille est soumise périodiquement à un faisceau ultrasonore permettant de la libérer des éléments du sang qui tendraient à la colmater. Dans la partie centrale de la cavité 150, le fond de la cavité présente un bras 152 contenant un faisceau de fibres optiques aboutissant à un dispositif optique usuel terminant un faisceau de fibres 153 allongé dans le sens de la longueur du cathéter. En face, et séparée d'un espace 154, se trouve une optique 155 aboutissant à un faisceau de fibres contenu dans un bras 156. Le faisceau optique passant par 152 et rejoignant l'optique 153 est relié à une source lumineuse susceptible d'adresser dans l'espace 154, un rayonnement optique qui sera capté par l'optique 155 et retransmis par les fibres optiques du faisceau 156 vers des moyens de mesure. Dans l'espace 154, se trouve disposée à mi-distance entre les optiques 153 et 155, une grille 157, de préférence électriquement conductrice et qui peut être soumise périodiquement à des vibrations ultrasonores pour éliminer les éléments colmatants. Cette grille est supportée dans cet espace par un bras 158. A la partie inférieure de la grille 157, se trouve un transistor à effet de champ 159 de préférence associé à une petite électrode (non représentée) susceptible d'être amenée à une tension négative. L'ensemble de la grille et du transistor 159 est contenu dans un très fin grillage 160 dont les mailles ont une dimension d'ouverture de 3 microns, cette grille pouvant être par exemple réalisée à partir de fils en fibre telle que fibre de carbone ayant un diamètre, par exemple, de 1 à 3 microns.

Lorsque le cathéter est disposé dans le sang, celui-ci, dans son écoulement, traverse la grille 151 et circule dans une certaine mesure dans la cavité 150. Le sang pénètre à travers le fin grillage 160 dans l'espace 154, les éléments figurés du sang étant cependant incapables de pénétrer dans cet espace en raison du diamètre des mailles du grillage 160 qui peut, le cas échéant, être également amené à un potentiel négatif repoussant les éléments figurés. La fibrine qui peut éventuellement se former se déposera préférentiellement sur la grille 157 laquelle peut, à cet effet, être préalablement pourvue d'une couche de fibrine ou présentée en association avec la fibrine ou séparément, des produits susceptibles d'induire la formation de fibrine, tels que notamment des précurseurs ou facteurs insolubilisés sur la grille. La lumière traversant l'espace 154 verra son intensité décroître, et son spectre, le cas échéant, se modifier, en fonction de la présence ou de l'absence de fibrine sur le grillage 157, et de l'épaisseur de la couche de fibrine. Le transistor 159 est sensible à la concentration en ions calcium. De façon avantageuse, la partie inférieure de la grille 160 peut être amenée à un potentiel négatif facilitant la formation de la fibrine de sorte que l'on peut, le cas échéant, observer un dépôt d'épaisseur décroissante vers le haut, les variations spatiales d'épaisseur pourra alors être également analysées par analyse des différents pixels reçus dans les différentes fibres du faisceau 156. De façon avantageuse, un ou plusieurs conduits peuvent être prévus dans le bras 158 pour amener dans l'espace 154, s'il y a lieu, un agent fibrinolytique, par exemple plasminogène activé, , héparine, etc., et limiter la quantité de fibrine susceptible de s'y former. La grille 151, pour sa part, empêche, en cas d'effraction du grillage 160, le passage dans la circulation d'éléments de fibrine ayant une taille dangereuse.

Mesure et modification de la coagulabilité sanguine.

Le dispositif peut avantageusement provoquer et/ou inhiber localement sur une petite bandelette la coagulabilité sanguine, la bandelette étant couverte de films moléculaires (obtenus par exemple par la méthode de Langmuir-Blodgett) et plongée dans le sang. Les films moléculaires sont dopés par des lignes ou points superposées des différentes molécules coagulantes (telles que prothrombine, thrombine, fibrinogène avec ponts de Ca⁺⁺, fibrine monomère) et anticoagulantes (tels que héparine sulfate, activateur du plasminogène, + plasminogène, plasmine, protéine C,). Ces groupes de molécules fixés et répartis sur le support attirent des molécules spécifiques qui, d'une part, tendent à compléter la chaîne fonctionnelle de la macro-molécule en question et, d'autre part, des molécules spécifiques qui tendent à s'opposer à l'action de ces macromolécules. Le micro-brouillard ou micro-dépôt local qui se forme dans le sang peut être dosé, par exemple par voie optique, quantitativement et qualitativement. Deux groupes de molécules coaguloactives, par exemple antagonistes, peuvent avantageusement être placés sur deux pointes géométriquement opposées et faiblement éloignées l'une de l'autre, de préférence 1 mm ou moins. Différents groupes antagonistes ou au contraite complémentaires, peuvent être étagés le long d'un cathéter. Les deux pointes d'un groupe peuvent être réalisées en matériau optiquement conducteur de sorte que le nuage qui se forme entre les pointes agisse sur la transmission de lumière d'une pointe à l'autre. Ainsi, une anomalie de la coagulation peut révéler immédiatement les molécules ou chaînes de réactions en manque ou en excès. A titre d'exemple, l'une des pointes de chaque groupe présente des couches de Langmuir-Blodgett dopées par de la fibrine monomère, les deuxièmes pointes de chaque groupe présentant respectivement des couches dopées par thrombine, prothrombine, héparine, TPA, plasminogène, plasmine, protéine C. Le dispositif thrombolitique peut alors éventuellement automatiquement corriger certains défauts de la coagulation par injection de substances thrombolytiques par exemple ou alerter par signaux sur une télécommande le sujet de son trouble sanguin afin qu'il prenne la médication adéquate.

Les différents moyens de mesure de paramètre, tels que des émetteurs et/ou récepteurs laser et maser, éventuellement modulés, ultra-sonore, sonore, de mesure d'impédance électrique ou d'électrocardiogramme peuvent, de préférence être orientés spatialement dans l'organisme, par télécommande depuis l'extérieur. Dans ce cas, l'énergie nécessaire au mouvement mécanique peut être fournie par télétransmission, par exemple haute fréquence, la source d'énergie du dispositif selon l'invention pouvant, par exemple, comporter, d'une part, une pile, nucléaire ou non, et d'autre part, un récepteur implanté d'énergie depuis l'extérieur, soit pour des surconsommations liées à des manoeuvres ou interventions particulières du dispositif, soit pour recharger tout ou partie de la source.

Ainsi, par exemple, le détecteur récepteur ultrasonore intra- ou juxtacardiaque peut comporter une tête émettrice-réceptrice mobile ou tournante motorisée par télécommande, permettant d'enregistrer sur une mémoire du dispositif implanté, un échocardio- ou échomédiastinogramme d'origine centrale, capable d'être retransmis à l'extérieur par télécommande.

Les moyens optiques endoscopiques peuvent également être orientables, par exemple pour explorer les structures ou cellules osseuses et médullaires.

Dans le cas où la dose thérapeutique est obtenue par expression génétique de moyens situés sur un support solide dans le sang ou la lymphe, l'expression d'une molécule thérapeutique, par exemple thrombolytique, peut être obtenue par traduction de ses molécules génétiques codantes à longues chaînes opposées ARN.m et ARN.t activées, entourées de ses ribosomes et ses nombreuses enzymes aminoacyl-ARN.t synthétases maintenues à proximité fonctionnelle selon des modes connus (par exemple : section transcrite d'un gène isolé inclus dans un film moléculaire artificiel permettant le passage des molécules plus petites telles qu'acides aminés, tPA, plasminogène).

Sur la figure 7, on voit un dispositif selon l'invention. Celui-ci comporte une ceinture thoracique 1′ susceptible d'être fixée autour du thorax du sujet. Cette ceinture est, de préférence, maintenue par des bretelles scapulaires (non représentées) qui ont, en plus, l'avantage d'indexer la position angulaire de la ceinture. Cette ceinture thoracique comporte deux larges électrodes métalliques souples 2′, 3′ continues ou en spirale qui, lorsque la ceinture est mise en place sur le sujet, sont disposées l'une, 3′, sensiblement sternale basse, l'autre, 2′, légèrement rétroaxillaire pour l'envoi d'une impulsion de choc de défibrillation. Les deux électrodes 2′, 3′ sont reliées, par des voies conductrices représentées en traits interrompus, à une unité centrale 4′. Cette unité centrale est reliée par des voies conductrices à une antenne réceptrice 5′ ainsi que par d'autres voies conductrices à un condensateur, ou un ensemble de condensateurs, 6′, de grande capacité, par exemple 300 joules à 3000 volts. Une pile 7′ est associée à l'unité centrale 4′. Une prise de courant, d'un format spécial, 8 permet la charge du condensateur 6′.

Dans le boîtier 1′ se trouve aussi un stimulateur implantable, de type orthorythmique POR selon les brevets US-A-3.857.399 et 4.052.991, comprenant une antenne 10′ d'émission en spirale. Lorsque la ceinture 1 est convenablement disposée autour du thorax, l'antenne réceptrice se trouve au regard ou à proximité de l'antenne émettrice 10 du dispositif implanté. Le stimulateur comporte un cathéter intracardiaque 11′ se terminant par des électrodes de préférence bi-polaires 12′, destinées à être mises en place dans la cavité ventriculaire droite. Le dispositif comporte encore un cathéter 13′ se terminant par un capteur de pression 14. Le cathéter 13′ est également destiné à pénétrer dans la cavité ventriculaire droite puis, de là, à franchir le septum interventriculaire de façon que le capteur de pression 14′ puisse pénétrer dans le myocarde ou ventricule gauche. En variante, le capteur 14′ peut être agencé sur le cathéter 11′ lui-même.

Le stimulateur implanté, qui ne sera pas décrit autrement en détail, est d'un type en soi connu, par exemple conforme aux deux brevets US précités. Il détecte les signaux électriques spontanés du coeur par ses électrodes bipolaires 12′ de façon à acquérir l'électrocardiogramme. Si la fréquence spontanée du coeur descend au-dessous d'une certaine limite, par exemple au-dessous de 50 (cycles par minute), le stimulateur stimule le coeur, par les électrodes 12′, à un rythme de base, par exemple 60, et ce tant qu'un signal électrique spontané (QRS) ne se sera pas manifesté. Si au contraire, la fréquence dépasse brusquement une certaine valeur, réglable, par exemple de 160/mn, correspondant à une tachycardie, le stimulateur adresse au coeur une succession d'impulsions de stimulation à une fréquence accrue de 190/mn, ce qui permet dans de nombreux cas de réduire la tachycardie. Un stimulateur de ce genre, conforme aux brevets US précités, est, par exemple, vendu sous licence de l'inventeur par la société Medtronic sous la dénomination DDD 7008, dans le cas d'une stimulation auriculaire.

Enfin, ce stimulateur comporte en outre des moyens électroniques permettant de déceler, par le biais des électrodes 12′, une fibrillation ventriculaire. Ces moyens, qui permettent d'analyser l'électrocardiogramme pour en déduire s'il s'agit d'une fibrillation ventriculaire, sont également d'un type connu et ont par exemple été décrits dans le brevet US-Re-27.757, ou dans tout autre moniteur usuel permettant la détection d'une fibrillation.

Enfin, le stimulateur comporte un circuit sensible à la pression captée par le capteur 14′.

Les moyens électroniques du stimulateur sont agencés, conformément à l'invention, de façon que, lorsque le stimulateur détecte l'apparition de signaux électriques d'une fibrillation strictement ventriculaire (par exemple tachyarythmie supérieure à 200), il vérifie pendant une durée, par exemple, de 4 s, s'il existe une activité de pression satisfaisante détectée par le capteur 14′. L'activité de pression peut être, par exemple, obtenue par acquisition d'un au moins des paramètres suivants : effondrement du maximum de la dérivée pression/temps ; effondrement de l'intégration de la pression systolique au temps ; allongement de la période de contraction isométrique du coeur ("preejection period") ; disparition des bruits valvulaires ; effondrement de toute variation de pression, par exemple au-dessous de 3 cm Hg. Si cette activité hémodynamique minimale reste présente, le stimulateur réagit selon sa programmation normale. Si cette activité hémodynamique minimale n'est plus présente, le stimulateur adresse, par le biais de son antenne émettrice 10′, un signal de déclenchement qui est capté par l'antenne réceptrice 5′ de la ceinture thoracique 1′. Les moyens électroniques 4′ de la ceinture, sensibles à cette réception, après vérification du bon contact des électrodes 2′ et 3′ avec la peau thoracique et vérification de l'état de charge du condensateur 6′, provoquent l'envoi d'une impulsion de défibrillation par les électrodes 2′ et 3′.

Le dispositif peut avantageusement être prévu pour envoyer une nouvelle impulsion si, au bout d'un certain temps, par exemple de l'ordre de 8 à 20 s, les moyens électroniques du stimulateur 8′ et/ou le capteur 14′ ne détectent pas de reprise d'une activité cardiaque satisfaisante.

On a représenté, sur la figure 8, un schéma du stimulateur qui comporte, relié aux électrodes 12′, un circuit électronique usuel de filtrage, d'amplification et de mise en forme 15′ permettant l'acquisition de l'électrocardiogramme, à savoir notamment l'acquisition du rythme cardiaque spontanée et éventuellement de sa morphologie, adressant les informations, et notamment la fréquence cardiaque spontané à un microprocesseur 16′. Le capteur de pression 14′ adresse ses signaux à un moyen de mise en forme et d'amplification 17′ transmettant les informations de valeur de pression au microprocesseur 16′. Le microprocesseur peut, selon le cas, inhiber ou un contraire piloter un générateur d'impulsions de stimulation 18′ à une fréquence choisie par le microprocesseur 16. Le microprocesseur 16′, en cas de détection de fibrillation, par exemple lorsque les moyens 15′ lui envoient un signal de fréquence irrégulière très élevée, par exemple supérieure à 200/mn, correspondant à la détection de la fibrillation, vérifie s'il reçoit des informations de pression satisfaisantes depuis les moyens 17′ et, dans la négative, il ordonne à l'antenne 10′ et aux moyens qui lui sont associés, d'émettre un signal de commande de défibrillation.

On a représenté sur la figure 9 un schéma des moyens électriques et électroniques de la ceinture 1′. Ces moyens comportent, dans le circuit 4′, un microprocesseur 19′ susceptible de piloter un circuit d'interruption de puissance, par exemple un thyristor 20′ qui, normalement, interrompt la liaison entre le condensateur 6′ et les électrodes de défibrillation 2′, 3′. Le circuit 4′ contient encore un circuit de mesure d'impédance 21′, relié aux électrodes 2′ et 3′, qui, par microcourant, mesure l'impédance entre les électrodes et adresse le résultat de sa mesure au microprocesseur 19′. Au cas où l'impédance dépasse une valeur de seuil réglable pour chaque sujet, le microprocesseur déclenche une alarme par un moyen d'alarme de type audiovisuel 22′. Par ailleurs, le microprocesseur reçoit les informations en provenance de l'antenne 5′.

Le condensateur peut être chargé à partir d'une prise de connexion 8′, que le sujet peut relier à un appareil de charge d'un type usuel permettant d'adresser au condensateur un courant de charge. En outre, le dispositif 4′ comporte un générateur haute tension continue 23′, alimenté par la pile 7′ de façon à compenser la perte de charge du condensateur due aux petits courants de fuite qui sont toujours présents. Le microprocesseur est sensible à la charge du condensateur, dont la valeur, réglable, est déterminée par le médecin et génère une alarme si cette charge devient insuffisante. Lorsque le microprocesseur 19′ reçoit, par l'antenne, le signal provenant du stimulateur 9′ et indiquant qu'une fibrillation avec perte de l'hémodynamique est en cours, après avoir fait les vérifications précitées, il actionne l'interrupteur de puissance 20′ et autorise par conséquent la décharge brutale du condensateur 6 pendant la durée choisie à l'avance, par exemple 1 à 10 ms, pour assurer le choc de défibrillation. Bien entendu, dans les différents schémas des figures précitées, on n'a pas représenté les divers moyens accessoires usuels de tels circuits électroniques tels qu'horloges, alimentations des différents composants, etc.

On comprend également qu'en variante, le cathéter 13′ peut être omis et la détection du fonctionnement de l'hémodynamique peut être assurée par un capteur externe, par exemple fixé au lobe de l'oreille ou autour d'un doigt, et qui est susceptible d'indiquer la présence ou l'absence du flux hémodynamique. Dans ce cas, le stimulateur adresse, par son antenne, une impulsion de commande en direction de la ceinture 1′ dès lors qu'il détecte un phénomène électrique cardiaque de nature fibrillatoire ou tachycardique grave. C'est alors le circuit électronique 4′ qui vérifie s'il existe une hémodynamique satisfaisante et, dans la négative, provoque l'envoi du choc de défibrillation.

On comprend, par ailleurs, que la ceinture thoracique 1′ peut encore comporter, sur sa surface orientée vers le thorax, une pluralité d'électrodes de détection et/ou de stimulation, à la manière de la ceinture décrite dans le brevet FR-A-1.237.702 (1953). Ces électrodes peuvent également servir à l'envoi d'un choc de défibrillation si elles ont une surface suffisante. Ces électrodes, par ailleurs, peuvent permettre de détecter l'électrocardiogramme à la place du stimulateur interne 9′, ou bien encore servir à une stimulation par l'extérieur, à la façon décrite par exemple dans le brevet français précité. L'avantage de l'utilisation d'un grand nombre d'électrodes disséminées sur la ceinture, en choisissant celles des électrodes que l'on utilise, est d'assurer des stimulations ou des défibrillations ayant des orientations spatiales particulières.

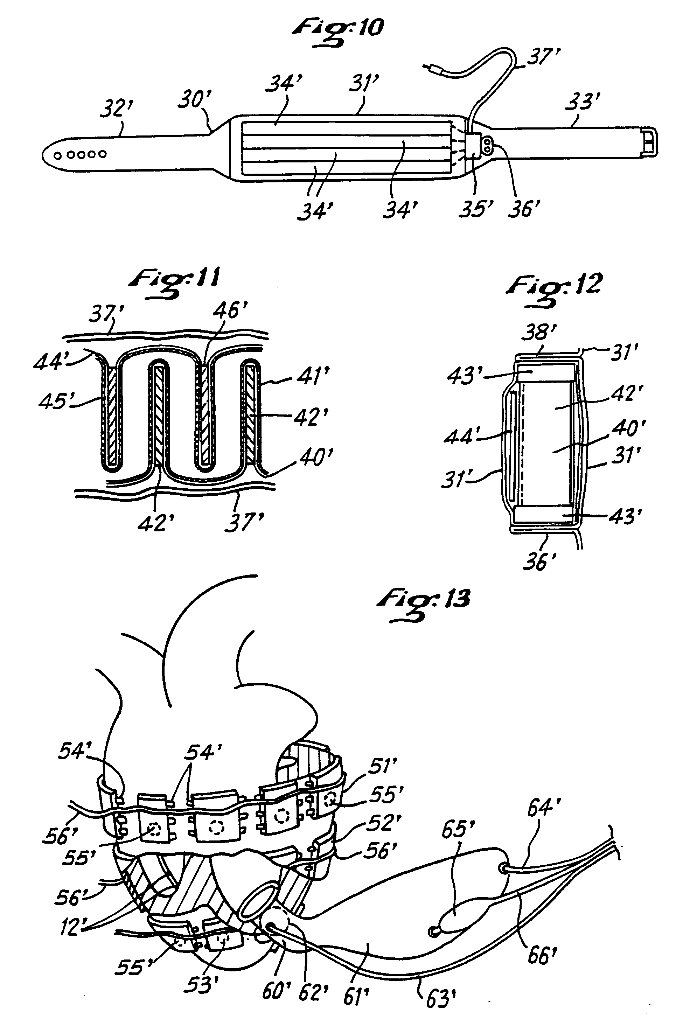

En se référant à la figure 10, on voit une vue d'une sangle abdominale complémentaire 30′ présentant une partie ventrale élargie 31 et des parties 32′, 33′ destinées à être réunis dans le dos. Dans sa partie 31′, la matière textile ou plastique est extrêmement fine et déformable tout en présentant une grande résistance à la traction. Sur cette partie 31 se trouve appliqués une pluralité d'éléments moteurs longitudinaux 34′ respectivement alimentés par des conducteurs en provenance d'un circuit de connexion 35′ et susceptibles d'être alimentés à partir d'une prise de courant 36 fixée sur la ceinture. Un cordon conducteur 37′ permet une liaison électrique entre la boîte de connexion 35′ et les moyens électroniques 4′ de la ceinture thoracique 1′.

En se référant aux figures 11, 12, on voit de façon schématique un exemple de la structure interne d'un élément longitudinal autocontractile 34′, selon une coupe horizontale (fig. 11), lorsque la ceinture est dans sa position verticale comme représenté sur la figure 10.

Chaque élément 34 comporte une gaine souple déformable électriquement isolante 37 ayant une section transversale par exemple rectangulaire, et dont les petits côtés sont fixés à des replis correspondantes 38 de la paroi de ceinture 31. Dans cette gaine sont disposés deux éléments électrostatiques. L'un de ces éléments se présente sous forme d'une bande métallique souple conductrice 40 revêtue d'une couche isolante électrique extrêmement mince à très haut pouvoir d'isolation et susceptible de suivre étroitement les déformations de la bande. Cette couche d'isolant est représentée en trait fort 41 sur les figures 5 et 6. Périodiquement, la bande 40 chevauche des éléments minces plus rigides isolants 42 de forme généralement rectangulaire et dont les extrémités 43, qui dépassent de la bande, sont rendues solidaires de la gaine 31 et du repli 38 au même emplacement. Une deuxième bande 44, identique à la première et comme elle revêtue d'un isolant 45, entoure également, en les chevauchant, des éléments rectangulaires rigides 46 identiques aux éléments 42, les deux bandes étant assemblées de façon à réaliser une imbrication. Au repos, les éléments transversaux consécutifs des deux bandes, formés chacun par deux épaisseurs de bande et un élément rigide situé entre elles, se trouvent espacés d'une distance inférieure à 1 mm. Si l'on applique une tension élevée positive à l'une des bandes et une tension élevée négative à l'autre bande, les différents éléments transversaux vont violemment s'attirer les uns les autres jusqu'à ce que les couches diélectriques des différents éléments soient amenées en contact. On provoque ainsi un raccourcissement de l'élément contractile 34. Si, au contraire, on applique une tension élevée de même signe aux deux bandes cette fois rapprochées, les bandes auront tendance à se repousser violemment. Le circuit de commutation 35 est relié électriquement aux différentes bandes afin de les mettre alternativement aux tensions voulues, l'énergie motrice pouvant être amenée à la ceinture par l'intermédiaire de la prise 36.

Les bandes peuvent être extrêmement minces, voire non métalliques, de l'ordre du micron ou inférieur, la continuité électrique pouvant être assurée par métallisation. La couche isolante peut être de l'ordre d'une fraction de micron à 50 microns. La plaque rigide 42′ ou 46′ est aussi mince que possible.

En variante, les éléments rigides peuvent être omis ou remplacés par une isolation relativement rigide, extérieure au repli de la bande et remplaçant l'isolant 41′, 45′.

La bande abdominale peut également être contractée par des moyens électromagnétiques. Par exemple, une réalisation simple consiste à disposer, le long de la bande, une succession d'électro-aimants, le noyau mobile attiré par un électro-aimant donné étant fixé à la bobine de l'électro-aimant consécutif, et ainsi de suite, de façon que l'actionnement de la totalité des aimants provoque une diminution de longueur de la ceinture égale à la somme des courses des noyaux mobiles.

Le circuit 35′ est relié par le cordon conducteur 37′ au circuit électronique 4 de la ceinture thoracique pour être mis en oeuvre lorsque la ceinture thoracique a, comme décrit ci-dessus, constaté, soit directement par le biais d'un capteur hémodynamique externe, soit par l'intermédiaire du capteur de pression associé au stimulateur interne, l'absence durable d'une hémodynamique convenable, auquel cas le circuit 35′ actionne alternativement les éléments électrostatiques ou électromagnétiques des différentes bandes contractiles 34′, à une fréquence par exemple de l'ordre de 20 par minute, provoquant ainsi des contractions rythmiques de l'épigastre et des côtes sus-jacentes, qui assurent une respiration abdominale auxiliaire.

On conçoit également que l'on pourrait rendre autocontractile, de la même façon, la ceinture thoracique afin de réaliser, par ce biais, un massage cardiaque externe.

On comprend également que l'on pourrait provoquer les contractions de la sangle abdominale et/ou de la ceinture thoracique par d'autres moyens moteurs, notamment hydrauliques ou pneumatiques, mais ceux-ci nécessitent alors la présence, par exemple, d'une pompe, par exemple piézo-élecrique, ou d'une autre source de fluide comprimé.

En se référant à la figure 13, on a représenté trois bandes épicardiques autocontractiles 51′, 52′, 53′ disposées autour de la zone ventriculaire du muscle cardiaque. Ces bandes autocontractiles sont susceptible de se contracter, de préférence péristaltiquement comme dans la contraction naturelle et automatiquement ajustables pour un rendement hémodynamique élevé, par les mêmes moyens électrostatiques ou électromagnétiques que la bande abdominale précitée, ces moyens étant aussi miniaturisés que possible. Chaque bande peut présenter en fait des zones contractiles séparées par des zones non-contractiles constituées de bandelettes 54′ pénétrant, après incision et suture, sur une certaine profondeur dans le muscle cardiaque, les bandes pouvant être, par ailleurs, cousues sur l'épicarde. La bande peut aussi porter des électrodes 55′ sur sa face épicardique, continue entre les bandelettes. Ces bandelettes 54′ peuvent être, en outre, conductrices et former ainsi des électrodes intramyocardiques réparties autour du myocarde ventriculaire, une pluralité de conducteurs 56′ desservant les différentes électrodes qui sont convenablement repérées dans l'espace au moment de la pose. L'alimentation en tension des différents éléments électrostatiques ou électromagnétiques des bandes est assurée par des conducteurs, non représentés, qui aboutissent à une source électrique de tension convenable qui peut être implantée ou externe. Dans ce dernier cas, un conducteur transcutané est préféré, le passage à travers la peau s'effectuant selon une technique déjà connue, mais un passage non transcutané est possible, soit par accouplement magnétique cylindrique, soit par couplage électromagnétique (F. Zacouto, Aperçu sur l'Expérimentation des Pompes Sanguines Implantables, Réanimation et Organes artificiels. Tome 2 (1965) n^{o} 2, Editeur Masson et Cie, Paris, p. 155-181).

Bien entendu, les bandes présentent, aux emplacements nécessaires, des ponts assez rigides passant par dessus les coronaires sans les comprimer.

La pose de ces bandes nécessite une thoracotomie et une adaptation exacte des bandes sur l'épicarde.

De façon avantageuse, les contractions des bandes contractiles peuvent être déclenchées par la détection des ondes de pression du mécanogramme par le capteur de pression 14, de sorte que la contraction des bandes sert uniquement à amplifier et prolonger la contraction naturelle régionale ou générale du muscle cardiaque. En variante, en cas de muscle cardiaque complètement défaillant, la contraction des bandes autocontractiles peut être pilotée, par exemple, par une base de temps convenable du stimulateur implanté, de préférence sous le contrôle d'une autorégulation hémodynamique, par exemple par détection des pressions et débits sanguins intraventriculaires, intramyocardiques ou artériels.

Les bandes présentent également l'avantage de contenir le muscle cardiaque et d'éviter une dilatation de celui-ci.

Si nécessaire, on peut réaliser, par les techniques décrites par exemple dans le brevet français FR-A-1.515.319 et son addiion, la mise en place d'un tube transmyocardique 60 au niveau apical de façon à faire communiquer le ventricule gauche avec un dispositif contractile auxiliaire ou supplétif destiné, soit à faire refluer vers le ventricule, à chaque systole, du sang sous pression préalablement recueilli, pour augmenter le débit cardiaque, soit à réaliser une dérivation ventriculo-aortique comme décrit par exemple par F. ZACOUTO "Sur l'évolution des circulations assistées, Extraits de la Biologie Médicale" (1971) 26, av. de l'Observatoire, Paris, Vol. LX, n^{o} 1, p. 14-34.

Un tel ventricule artificiel (pour lequel les moyens auto-contractiles n'ont pas été représentés) est désigné par 61 sur le dessin. A ce ventricule artificiel 61′, on peut avantageusement associer un ballonnet 62′ disposé dans le tube transmyocardique, ce ballonnet pouvant être gonflé ou dégonflé, pour la fermeture ou l'ouverture du ventricule, automatiquement ou manuellement à l'aide d'un fluide amené par un conduit souple extrapleural 63′. De façon avantageuse, un drain 64 aboutit dans le ventricule artificiel afin de pouvoir assurer le drainage et le lavage du ventricule, ainsi qu'éventuellement une perfusion médicamenteuse, à l'intérieur de la cavité. Une pompe à médicaments 65′ peut également aboutir dans le ventricule en étant reliée par un conduit 66′ de façon à pouvoir injecter sélectivement une ou plusieurs drogues, telles que par exemple de l'héparine, manuellement ou automatiquement. Un autre conduit peut être prévu pour aboutir au niveau de la surface d'un anneau autocontractile cardiaque afin de pouvoir introduire un fibroscope orientable pour explorer visuellement les surfaces cardiaques et juxtacardiaques.

Les différents conduits référencés sont amenés soit vers une extrémité sous-cutanée, soit par voie transcutanée, à des dispositifs externes permettant leur mise en oeuvre.

La présente invention propose de fournir un autre dispositif pour réduire les tachycardies, réalisé sous forme d'un stimulateur électrique et susceptible d'être utilisé soit seul, soit en combinaison, par exemple, avec un dispositif du type décrit ci-dessus avec son défibrillateur implanté ou non.

On a déjà essayé de réduire des tachycardies par pilotage manuel d'un stimulateur externe en adressant des impulsions de stimulation pairées, c'est-à-dire suivant chaque complexe ORS, en faisant varier la durée de couplage par tâtonnement jusqu'à obtenir une division par deux, apparente et stable, de la fréquence cardiaque et une modification correspondante du ventriculogramme avec une amélioration sensible des conditions hémodynamiques. Cette amélioration peut être expliquée par le fait que, lorsque la stimulation pairée intervient opportunément pendant l'intervalle systolique dans lequel le muscle cardiaque est contracté au maximum, une dépolarisation artificielle est réalisée, empêchant la survenue du prochain complexe électrique naturel de sorte que la durée diastolique s'accroît. Voir par exemple von E. Domanig et al., Verhandlungen Der Deutschen Gesellschaft Für Kreislaufforschung, Dr. Dietrich Steinkopff Verlag, Darmstadt (1969) 294-299.

De tels procédés n'ont cependant pas pu aboutir à une utilisation clinique du fait de l'apparition de troubles graves du rythme parfois avec fibrillation ventriculaire.

La présente invention a donc pour objectif de remédier à ces inconvénients et de fournir un dispositif de stimulation cardiaque pour le traitement et la réduction des extrasystoles automatiquement sélectionnées et des tachycardies, susceptible d'envoyer des impulsions électriques de stimulation pendant un intervalle systolique correspondant à la contraction du muscle cardiaque, de façon à réduire la tachycardie, et ceci sans risquer de dégrader le bilan énergétique du muscle cardiaque, les myocardes ventriculaires gauche et droit étant de préférence traités séparément lorsque leur contraction mécanique est décalée de plus de 30 ms.

L'invention a pour objet un dispositif de réduction des tachycardies comprenant :
- des moyens de détection du rythme cardiaque et de l'électrocardiogramme , tels que par exemple des électrodes implantées ;
- des moyens de stimulation pour adresser une impulsion ou un groupe d'impulsions de stimulation au muscle cardiaque, par exemple par le moyen d'électrodes implantées ;
- et une source d'énergie, par exemple implantée ; caractérisé en ce qu'il comporte :
- des moyens pour adresser au muscle cardiaque une stimulation électrique pairée, ou couplée de façon similaire, à un évènement périodique du cycle cardiaque ;
- des moyens de détection périodique d'un phénomène cardiaque lié à la contraction maximale et/ou au métabolisme du muscle cardiaque ;
- des moyens électroniques sensibles auxdits moyens de détection périodique du phénomène cardiaque et susceptibles automatiquement de modifier le couplage de la stimulation et/ou d'arrêter ladite stimulation, en fonction de ladite détection.

Les moyens sensibles au rythme cardiaque pour détecter une tachycardie peuvent être de tous types connus. Ces moyens peuvent notamment, d'une façon connue, exploiter les signaux électriques spontanés, et notamment les complexes ORS, détectés par les électrodes de détection du rythme cardiaque, et en déduire la fréquence cardiaque, soit instantanément, sur un seul cycle cardiaque, soit en établissant une moyenne de fréquence sur plusieurs cycles successifs, ou encore en comptant le nombre de cycles dans un intervalle de temps donné (voir par exemple le brevet US-A-4.052.991).

Tous autres moyens y compris ceux utilisant d'autres phénomènes, tels que par exemple les pulsations hémodynamiques, peuvent être utilisés.

Les moyens pour adresser au muscle cardiaque une stimulation électrique pairée, c'est-à-dire couplée à un évènement périodique du cycle cardiaque, peuvent également inclure des moyens électroniques usuels, ces moyens étant agencés pour adresser une impulsion de stimulation, ou un train d'impulsions de stimulation, par le biais d'électrodes implantées, après une durée déterminée et qui suit l'acquisition de l'évènement périodique choisi du muscle cardiaque. De préférence, on utilise les électrodes 12 du stimulateur.

Cet évènement peut être un événement tel qu'un signal électrique, par exemple le complexe QRS ou plus précisément l'onde R, ou bien au contraire le moment d'envoi de la stimulation précédente, c'est-à-dire un signal non spontané.

Cet évènement périodique peut également être mécanique ou hémodynamique, et notamment lié à l'onde systolique du mécanogramme. Il peut s'agir d'un évènement lié à l'onde systolique du dernier cycle achevé ou encore même du cycle en cours, auquel cas la durée déterminée de couplage est, bien entendu, extrêmement courte. Il peut s'agir, notamment, d'une valeur de pression, déterminée par un capteur de pression intraventriculaire par exemple, ou bien d'un point particulier du mécanogramme de pression, par exemple un point de transition, ou même d'une autre manifestation liée au mécanogramme telle que par exemple le bruit d'ouverture d'une valve cardiaque détectée par microphone.

Les moyens de détection d'un phénomène lié à la contraction maximale et/ou au métabolisme du muscle cardiaque peuvent être notamment l'un des suivants, plusieurs de ces moyens pouvant avantageusement être associés :
- des moyens d'acquisition des mécanogrammes ventriculaires droit et/ou gauche, tels que par exemple un capteur de pression, relié à des moyens susceptibles d'analyser les mécanogrammes et de détecter une anomalie de la forme des mécanogrammes et de leur intervalle ou d'une partie de celui-ci (par exemple le capteur 14);
- des moyens comprenant un ou des capteurs de paramètres biochimiques, notamment concentration en catécholamines, la pression d'oxygène ou CO2, ou le pH, ou la température, de préférence dans une cavité cardiaque telle que le ventricule droit ;
- des moyens d'acquisition du débit cardiaque.

Ces moyens d'acquisition peuvent avantageusement comprendre des moyens d'analyse, tels que par exemple un microprocesseur permettant de comparer le mécanogramme ou la partie du mécanogramme dont l'acquisition vient d'être faite, à un ensemble de référence ou, s'il s'agit de mesure de paramètres biochimiques, à une ou des valeurs de référence.

Les moyens électroniques, sensibles aux moyens de détection périodique du phénomène cardiaque, comportent de préférence un circuit ou un microprocesseur, qui peut être également utilisé pour l'acquisition de la détection du phénomène cardiaque en provenance des capteurs.

Ces moyens électroniques sont agencés, par exemple, de façon à pouvoir modifier la durée de couplage déterminée, c'est-à-dire la durée séparant l'évènement périodique du cycle cardiaque qui a été choisi et le moment de l'envoi de l'impulsion, en fonction de la qualité détectée des demande et dépense énergétiques du muscle cardiaque, de préférence des ventricules gauche et droit à chaque cycle.

Dans une forme de réalisation, tant que le dispositif estime que la contraction et/ou le métabolisme est normal, il ne modifie pas ladite durée. En cas d'anomalie, il modifie la durée, soit suivant un balayage systématique, par exemple un balayage en diminution de ladite durée, soit en fonction d'instructions pré-établies.

Dans une autre forme de réalisation, en cas d'apparition d'une baisse de la qualité de la disponibilité énergétique, lesdits moyens électroniques suppriment les stimulations, laissant alors le coeur fonctionner à son rythme propre ou stimulé.

On préférera combiner les deux formes de réalisation de sorte que le dispositif, en cas de baisse de la qualité de fonctionnement du muscle cardiaque, tentera d'abord, en modifiant ladite durée déterminée de couplage, de rétablir la qualité et, si au bout d'un nombre prédéterminé, par exemple, de cycles, cette qualité n'a pas pu être rétablie, il abandonnera la stimulation.

Le dispositif selon l'invention peut être réalisé sous forme d'une unité susceptible d'être implantée telle quelle. Cependant, il peut également être associé à un stimulateur cardiaque usuel à la demande, d'un type quelconque, par exemple, du type DDD ou non, ou encore à un stimulateur anti-tachycardique tel que décrit, par exemple dans les brevets US précités.

Lorsque le dispositif est ainsi combiné à d'autres moyens de stimulation, on comprend qu'il peut avantageusement partager avec ceux-ci les électrodes de détection et les électrodes de stimulation du muscle, la source d'énergie implantée et, le cas échéant, les moyens sensibles au rythme cardiaque pour détecter la tachycardie et les moyens pour adresser au muscle cardiaque la stimulation. Il peut également partager des moyens électroniques tels que par exemple un microprocesseur. Le dispositif peut également êre associé à un défibrillateur automatique externe ou implanté et, dans ce cas, il peut avantageusement être sensible à la survenue d'une impulsion de défibrillation. Par exemple, il peut être agencé pour être rendu inopérant après la survenue d'une impulsion de défibrillation, ou bien dans le cas où plusieurs impulsions de défibrillation se produisent à l'intérieur d'un intervalle de temps déterminé, ou après un nombre de cycles déterminé.

## Revendications

1. Dispositif de protection contre les affections liées à la coagulation sanguine, et notamment thromboses, embolies, coagulopathies, hémorragies, hémopathies et présence d'éléments cellulaires anormaux dans le sang, caractérisé en ce qu'il comprend :
- des premiers moyens implantables pour mesurer, en continu ou périodiquement, au moins un paramètre biologique susceptible de précéder ou d'accompagner une affection lieé à la coagulation sanguine comprenant au moins un moyen de présentation susceptible d'être placé dans une cavité ou un vaisseau sanguin ou lymphathique, pour présenter au sang, ou à la lymphe, un élément (157) agencé pour faciliter une variation locale dudit au moins un paramètre biologique, et des moyens de capteur (153,155) associés audit élément et sensibles à ladite variation,
- des seconds moyens implantables (40, 50, 51) déterminant un ou plusieurs seuils auquel ledit paramètre est comparé,
- et des troisième moyens implantables (60) sensibles aux dépassements de ce(s) seuil(s) pour délivrer, dans la circulation, une dose d'une ou plusieurs substances actives sur la coagulation.

2. Dispositif selon la revendication 1 caractérisé en ce que lesdits premiers moyens implantables sont portés par un cathéter (2).

3. Dispositif selon la revendication 2, caractérisé en ce que ledit élément (157) comporte au moins un facteur de la coagulation sanguine, agencé pour être entouré par le sang ou la lymphe.

4. Dispositif selon la revendication 3, caractérisé en ce que ledit facteur est la fibrine ou un de ses précurseurs.

5. Dispositif selon la revendication 2, caractérisé en ce qu'il comporte des moyens de présentation disposés sur des pointes faiblement distantes l'une de l'autre.

6. Dispositif selon la revendication 5, caractérisé en ce que les éléments présentés sur lesdites pointes sont choisis parmi les suivants : fibrine, ses dérivés ou précurseurs, prothrombine, prothrombinase, plasminogène, plasmine, protéine C, endothéline, sérotonine, catécholamines, neuropeptides Y, activateurs du plasminogène ou ses inhibiteurs.

7. Dispositif selon l'une des revendications 2 à 5, caractérisé en ce que lesdits éléments sont fixés sur un support insoluble.

8. Dispositif selon l'une des revendications 1 à 7, caractérisé en ce que lesdits moyens comportent un capteur sensible au calcium et/ou aux ions H⁺.

9. Dispositif selon l'une des revendications 1 à 7, caractérisé en ce que lesdits premiers moyens comportent un capteur optique.

10. Dispositif selon l'une des revendications 1 à 7, caractérisé en ce que lesdits premiers moyens comportent un capteur sensible à une impédance électrique.

11. Dispositif selon l'une des revendications 2 à 7, caractérisé en ce que lesdits premiers moyens sont sensibles à des variations de facteurs locaux de la coagulation variant en fonction des champs ou courants électriques locaux.

12. Dispositif selon l'une des revendications 1 à 11, caractérisé en ce qu'il comporte des moyens (4) de détection du cycle cardiaque ou du pouls et commandant l'acquisition des mesures dudit paramètre biologique en des instants prédéterminés du cycle cardiaque.

13. Dispositif selon l'une des revendications 2 à 4, caractérisé en ce que lesdits premiers moyens comportent un capteur sensible à l'épaisseur d'un dépôt dudit élément (157).

14. Dispositif selon l'une des revendications 1 à 13, caractérisé en ce qu'il comporte, en outre, des moyens sensibles à d'autres paramètres biologiques, généraux ou locaux.

15. Dispositif selon la revendication 14, caractérisé en ce qu'il comporte des moyens (14, 15) pour mesurer l'impédance d'une voie vasculaire.

16. Dispositif selon la revendication 14, caractérisé en ce que lesdits moyens (14, 15) sont disposés dans ou autour d'une artère, notamment coronaire.

17. Dispositif selon la revendication 15, caractérisé en ce que lesdits moyens sont disposés dans le myocarde.

18. Dispositif selon l'une quelconque des revendications 15 à 17, caractérisé en ce qu'il comporte une source électrique (30) reliée à des moyens d'électrodes (14, 15) pour une émission destinée à la mesure d'une impédance électrique, à au moins une fréquence comprise entre quelques kHz et un ou quelques MHz.

19. Dispositif selon l'une quelconque des revendications 15 à 18, caractérisé en ce que le dispositif comporte des moyens (40) sensibles à la différence des impédances mesurées.

20. Dispositif selon la revendication 19, caractérisé en ce que lesdits moyens (40) sont sensibles à la différence entre l'impédance maximale et l'impédance minimale mesurées dans un même cycle cardiaque.

21. Dispositif selon l'une quelconque des revendications 15 à 20, caractérisé en ce qu'il comporte des moyens (40) sensibles au décalage du décours de la courbe de mesure d'impédance susceptible de se produire entre deux cycles.

22. Dispositif selon l'une quelconque des revendications 15 à 21, caractérisé en ce qu'il comporte des moyens d'électrodes disposés dans le et/ou à distance du muscle cardiaque pour former des axes de mesure distincts et acquérir le vectogramme des variations d'impédance électrique du coeur.

23. Dispositif selon la revendication 14, caractérisé en ce qu'il comporte au moins un capteur de pression sensible à la pression sanguine ou myocardique.

24. Dispositif selon la revendication 23, caractérisé en ce qu'il comporte au moins deux capteurs de pression espacés le long d'une voie vasculaire ou cardiaque et des moyens sensibles à la valeur des pressions captées et/ou au décalage entre l'établissement des pressions captées par lesdits capteurs.

25. Dispositif selon la revendication 14, caractérisé en ce qu'il comporte un ou plusieurs capteurs sensibles à la déformation géométrique d'une voie cardiaque ou vasculaire.

26. Dispositif selon la revendication 25, caractérisé en ce que ledit capteur est une jauge de contrainte disposée sur un vaisseau.

27. Dispositif selon la revendication 25, caractérisé en ce que ledit capteur comporte un émetteur ultrasonore.

28. Dispositif selon la revendication 27, caractérisé en ce qu'il comporte des moyens de capteurs susceptibles de mesurer la durée du transit ultrasonore dans la voie vasculaire ou cardiaque.

29. Dispositif selon l'une quelconque des revendications 25 à 28, caractérisé en ce qu'il comporte des moyens reliés auxdits capteurs et sensibles à l'amplitude de la déformation géométrique pendant un cycle.

30. Dispositif selon l'une quelconque des revendications 25 à 29, caractérisé en ce qu'il comporte des moyens sensibles au décalage du décours de la courbe de déformation géométrique entre deux cycles.

31. Dispositif selon la revendication 14, caractérisé en ce qu'il comporte au moins un capteur sensible à une concentration ionique et disposé au voisinage d'une zone à risque.

32. Dispositif selon la revendication 31, caractérisé en ce que les ions de ladite concentration ionique sont choisis parmi les ions potassium, H+, sodium, chlore, calcium, magnésium, phosphore.

33. Dispositif selon la revendication 14, caractérisé en ce qu'il comporte au moins un capteur sensible à un paramètre tel que teneur en oxygène, CO₂, CO, hémoglobine ou dérivés, myoglobine, myosine, disposé au voisinage d'une zone à risque.

34. Dispositif selon la revendication 14, caractérisé en ce qu'il comporte au moins un capteur de température sensible à la température différentielle au voisinage d'une zone à risque.

35. Dispositif selon l'une quelconque des revendications 1 à 34, caractérisé en ce qu'il comporte des moyens d'acquisition de l'électrocardiogramme.

36. Dispositif selon la revendication 35, caractérisé en ce qu'il comporte des moyens sensibles au rythme cardiaque.

37. Dispositif selon l'une quelconque des revendications 35 et 36, caractérisé en ce qu'il comporte des moyens sensibles à la forme de l'électrogenèse cardiaque et notamment aux décalages des segments ST.

38. Dispositif selon l'une quelconque des revendications 35 à 37, caractérisé en ce qu'il comporte des moyens d'électrodes endocavitaires et/ou intramyocardiques pour l'acquisition de l'électrocardiogramme.

39. Dispositif selon l'une quelconque des revendications 35 à 38, caractérisé en ce qu'il comporte des moyens sensibles à une anomalie électrique cardiaque dépassant une valeur de seuil pour provoquer la délivrance d'un médicament.

40. Dispositif selon l'une quelconque des revendications 35 à 39, caractérisé en ce qu'il comporte des électrodes de détection électrique disposées sur plusieurs axes géométriques pour l'acquisition du vectocardiogramme et des moyens sensibles à une modification du vectocardiogramme au-delà d'un seuil pour provoquer la libération d'une dose de médicament.

41. Dispositif selon la revendication 14, caractérisé en ce qu'il comporte au moins un capteur sensible aux bruits spontanés des valves cardiaques et/ou du myocarde.

42. Dispositif selon la revendication 14, caractérisé en ce qu'il comporte des moyens pour générer des sons ou ultrasons dans le coeur ou dans une voie vasculaire, ainsi que des moyens sensibles à la propagation ou l'absorption desdits sons ou ultrasons.

43. Dispositif selon la revendication 42, caractérisé en ce qu'il comporte des moyens générant les sons ou ultrasons avec une variation rapide de fréquence sur une plage de fréquences.

44. Dispositif selon la revendication 42, caractérisé en ce qu'il comporte des moyens sensibles à la fréquence de résonance.

45. Dispositif selon la revendication 12, caractérisé en ce qu'il comporte, au voisinage dudit support, surface ou cavité, des moyens d'électrodes alimentés en un courant pour mesurer l'impédance de la couche ou masse du dépôt.

46. Dispositif selon la revendication 13, caractérisé en ce qu'il comporte, au voisinage dudit support, surface ou cavité, des moyens d'émission et de réception lumineuse sensibles à l'épaisseur de la masse du dépôt.

47. Dispositif selon la revendication 13, caractérisé en ce qu'il comporte, au voisinage du support ou cavité, des moyens d'émission sonore ou ultrasonore et des moyens de capteurs sonores ou ultrasonores sensibles à la résonance de la couche ou masse de dépôt.

48. Dispositif selon la revendication 13, caractérisé en ce qu'il comporte des moyens d'émission ultrasonore et de réception ultrasonore sensibles à la résonance correspondant au facteur dans le sang.

49. Dispositif selon la revendication 14, caractérisé en ce qu'il comporte des moyens d'émission sonore ou ultrasonore et un capteur sonore ou ultrasonore, orientés dans une voie vasculaire ou cardiaque pour émettre des sons ou ultrasons avec une variation de fréquence rapide et détecter la résonance de la colonne sanguine.

50. Dispositif selon la revendication 49, caractérisé en ce qu'il comporte des moyens pour provoquer l'émission et la mesure en des moments déterminés du cycle cardiovasculaire, notamment pendant une systole ou une diastole.

51. Dispositif selon la revendication 14, caractérisé en ce qu'il comporte des moyens optiques d'acquisition d'une couleur ou image dans le sang circulant ou une paroi cardiaque ou artérielle ou la moelle osseuse.

52. Dispositif selon la revendication 51, caractérisé en ce qu'il comporte des moyens permettant le comptage d'éléments figurés du sang.

53. Dispositif selon la revendication 14, caractérisé en ce que l'on effectue la mesure desdits autres paramètres de façon périodique en association avec les cycles cardiaques ou artériels.

54. Dispositif selon la revendication 12, caractérisé en ce que l'on mesure les paramètres plusieurs fois pendant un cycle et que l'on compare auxdits moyens de seuil la variation de paramètre pendant le cycle.

55. Dispositif selon l'une quelconque des revendications 1 à 13, caractérisé en ce qu'il comporte des moyens sensibles au rythme et/ou à l'électrogenèse cardiaque, lesdits moyens étant reliés à des moyens de détermination de seuil pour modifier les seuils.

56. Dispositif selon l'une quelconque des revendications 1 à 55, caractérisé en ce qu'il comporte des moyens sensibles au rythme et/ou à l'électrogenèse cardiaque, reliés aux moyens de libération de dose pour autoriser ou inhiber ladite libération.

57. Dispositif selon l'une quelconque des revendications 1 à 56, caractérisé en ce qu'il comporte un stimulateur et/ou défibrillateur cardiaque.

58. Dispositif selon la revendication 57, caractérisé en ce que ledit stimulateur est muni de moyens antitachyarythmiques.

59. Dispositif selon l'une quelconque des revendications 1 à 58, caractérisé en ce qu'il comprend, en combinaison, un stimulateur électrique orthorythmique portatif avec des électrodes (12') permettant de détecter le rythme cardiaque et d'adresser des impulsions de stimulation au coeur, et un défibrillateur portatif (4') comprenant une source d'énergie (6') et des électrodes de défibrillation (2', 3') et des moyens de détection d'une fibrillation ou tachycardie rapide, pour mettre en oeuvre ledit défibrillateur, le circuit électronique du stimulateur cardiaque étant protégé en cas de mise en oeuvre de la défibrillation, ainsi que des moyens de détection (14') de l'hémodynamique centrale et/ou artérielle et susceptibles d'empêcher le fonctionnement du défibrillateur tant qu'un fonctionnement hémodynamique suffisant reste détecté.

60. Dispositif selon la revendication 59, caractérisé en ce qu'il comporte un défibrillateur externe comportant, sur une ceinture (1') ou autre harnais thoracique, au moins deux électrodes cutanées de défibrillation (2', 3') disposées en opposition sur le thorax, les électrodes en opposition étant de polarités opposées.

61. Dispositif selon l'une quelconque des revendications 59 et 60, caractérisé en ce que le défibrillateur comporte, à l'extérieur, une ou plusieurs électrodes cutanées de même polarité, et au moins une électrode intra-cardiaque de polarité opposée, ainsi que des moyens de liaison électromagnétique disposés de part et d'autre de la peau pour compléter le circuit entre électrodes externe et interne.

62. Dispositif selon l'une quelconque des revendications 60 et 61, caractérisé en ce qu'il comporte une source d'énergie pour les chocs de défibrillation et comprenant un ou plusieurs condensateurs (6') fixés sur une ceinture ou harnais thoracique.

63. Dispositif selon l'une quelconque des revendications 59 à 62, caractérisé en ce qu'il comporte, pour fournir l'énergie nécessaire au choc de défibrillation, un ou plusieurs condensateurs (6'), une pile (7') susceptible de suppléer au courant de fuite des condensateurs ainsi qu'un moyen ou prise (8') de recharge de condensateur.

64. Dispositif selon l'une quelconque des revendications 59 à 63, caractérisé en ce que les moyens de détection de l'hémodynamique centrale et/ou artérielle comportent au moins un capteur de pression intra-cardiaque.

65. Dispositif selon l'une quelconque des revendications 59 à 64, caractérisé en ce qu'il comporte une ceinture thoracique comprenant des moyens autocontractiles ou moteurs susceptibles de réduire sa circonférence et de pratiquer un massage cardiaque à une fréquence de l'ordre de 20 à 60/mn, lesdits moyens étant sensibles aux moyens de détection de l'hémodynamique centrale et/ou artérielle et/ou de gaz du sang.

66. Dispositif selon l'une quelconque des revendications 59 à 65, caractérisé en ce qu'il comporte une sangle abdominale (31') munie de moyens contractiles (34') ou moteurs susceptibles d'en réduire la circonférence pour assurer une assistance respiratoire.

67. Dispositif selon l'une des revendications 65 et 66, caractérisé en ce que lesdits moyens autocontractiles ou moteurs sont de type électrostatique et/ou électromagnétique.

68. Dispositif selon l'une quelconque des revendications 59 à 67, caractérisé en ce qu'il comporte, autour du muscle cardiaque au niveau des ventricules, une ou plusieurs bandes (51') susceptibles de s'opposer à une dilatation progressive des cavités cardiaques.

69. Dispositif selon la revendication 68, caractérisé en ce que lesdites bandes (51') sont autocontractiles (54') et comportent des segments non-autocontractiles insérés dans le muscle cardiaque.

70. Dispositif selon l'une quelconque des revendications 1 à 69, caractérisé en ce qu'il comporte un tube transapical (60) du ventricule gauche reliable à une pompe sanguine aboutissant à l'aorte.

71. Dispositif selon l'une des revendications 59 à 70, caractérisé en ce que les moyens anti-tachycardiques comprennent des moyens pour le traitement sélectif des extra-systoles isolées ou pairées, par déclenchement d'une stimulation consécutive à rythme temporairement plus rapide, pilotée par l'extra-systole elle-même.

72. Dispositif selon l'une quelconque des revendications 59 à 71, caractérisé en ce que lesdits moyens antitachycardiques comportent des moyens susceptibles de générer une stimulation à une fréquence déterminée, notamment supérieure, en réponse à une détection d'une activité tachycardique.

73. Dispositif selon l'une quelconque des revendications 59 à 72, caractérisé en ce qu'il comporte des moyens pour la détection périodique automatique du seuil d'excitabilité du myocarde par envoi d'impulsions préférentiellement infra-liminaires.

74. Dispositif selon l'une quelconque des revendications 59 à 73, caractérisé en ce qu'il comporte des moyens sensibles au rythme cardiaque pour détecter une tachycardie, des moyens pour adresser au muscle cardiaque une stimulation électrique pairée, ou couplée de façon similaire, à un événement périodique du cycle cardiaque, des moyens de détection périodique d'un phénomène cardiaque lié à la contraction maximale et/ou au métabolisme du muscle cardiaque, et des moyens électroniques sensibles auxdits moyens de détection périodique du phénomène cardiaque et susceptibles de modifier le couplage de la stimulation et/ou d'arrêter ladite stimulation, en fonction de ladite détection.

75. Dispositif selon la revendication 74, caractérisé en ce qu'il comporte des moyens pour détecter un événement périodique tel qu'un signal électrique, notamment le complexe QRS ou le moment d'envoi de la stimulation précédente, ou un événement mécanique ou hémodynamique lié à l'onde systolique du mécanogramme.

76. Dispositif selon l'une quelconque des revendications 1 à 27, caractérisé en ce qu'il comporte, sur un support implanté, des moyens d'expression ou traduction génétique d'une dose d'agent thérapeutique.

## Claims

1. Device for protection against blood coagulation related ailments, in particular thrombosis, embolisms, coagulopathies, haemorrhages, hemopathies and presence of abnormal cell elements in the blood, characterized by the fact that it comprises:
- first implantable means to measure, continuously or periodically, at least one biological parameter likely to precede or accompany a blood coagulation related ailment comprising at least one presentation means which can be arranged in lymphatic or blood vessel or cavity, in order to present to the blood or lymph, an element (157) arranged to facilitate a local variation of said at least one biological parameter, and sensor means (153, 155) associated with said element and sensitive to said variation,
- second implantable means (40, 50, 51) determining one or several thresholds to which said parameter is compared,
- and third implantable means (60) which are sensitive to crossing of this(these) threshold(s) to release into circulation, a dose of one or several substances which are active on coagulation.

2. A device according to claim 1, characterized in that said first implantable means are carried by a catheter (2).

3. A device according to claim 2, characterized in that said element (157) includes at least one blood coagulation factor, arranged so as to be surrounded with blood or lymph.

4. A device according to claim 3, characterized in that said factor is fibrin or one of its precursors.

5. A device according to claim 2, characterized in that it comprises presentation means arranged on points which are arranged at a small distance from each other.

6. A device according to claim 5, characterized in that the elements presented on said points are selected from among the following substances: fibrin, its derivatives or precursors, prothrombin, prothrombinase, plasminogen, plasmin, protein C, endothelin, serotonin, cathecholamines, neuropeptides Y, plasminogen activators or its inhibitors.

7. A device according to one of claims 2 to 5, characterized in that said elements are attached on to an insoluble carrier.

8. A device according to one of claims 1 to 7, characterized in that said means include a sensor which is sensitive to calcium and/or H⁺ ions.

9. A device according to one of claims 1 to 7, characterized in that said first means include an optical sensor.

10. A device according to one of claims 1 to 7, characterized in that said first means include a sensor which is sensitive to an electrical impedance.

11. A device according to one of claims 2 to 7, characterized in that said first means are sensitive to variations of local factors of the coagulation varying as a function of local electrical currents or fields.

12. A device according to one of claims 1 to 11, characterized in that it includes means (4) for the detection of the cardiac cycle or heart pulse and controlling the acquisition of measurements of said biological parameter at predetermined moments of the cardiac cycle.

13. A device according to one of claims 2 to 4, characterized in that said first means include a sensor which is sensitive to the thickness of a deposit of said element (157).

14. A device according to one of claims 1 to 13, characterized in that it further comprises means which are sensitive to other general or local biological parameters.

15. A device according to claim 14, characterized in that it comprises means (14, 15) to measure the impedance of a vascular vessel.

16. A device according to claim 14, characterized in that said means (14, 15) are arranged inside or arround an artery, in particular coronary artery.

17. A device according to claim 15, characterized in that said means are arranged in myocardium.

18. A device according to any one of claims 15 to 17, characterized in that it comprises an electrical source (30) connected to electrode means (14, 15) for an emission to measure an electrical impedance, at least at a frequency between a few kHz and one or several MHz.

19. A device according to any one of claims 15 to 18, characterized in that said device includes means (40) which are sensitive to the difference in measured impedances.

20. A device according to claim 19, characterized in that said means (40) are sensitive to the difference between the maximum impedance and the minimum impedance measured in the same cardiac cycle.

21. A device according to any one of claims 15 to 20, characterized in that it includes means (40) which are sensitive to the time lag in the evolution pattern in the impedance measurement curve which may happen between two cycles.

22. A device according to any one of claims 15 to 21, characterized in that it includes electrode means arranged in and/or at a distance from the cardiac muscle so as to form distinct measurement axes and acquire the vectorgram of the heart electrical impedance variations.

23. A device according to claim 14, characterized in that it includes at least one pressure sensor which is sensitive to the blood or myocardial pressure.

24. A device according to claim 23, characterized in that it includes at least two pressure sensors spaced along a vascular or cardiac vessel, and means which are sensitive to the picked tip pressure value and/or time lag between the setting of pressures picked up by said sensors.

25. A device according to Claim 14, characterized in that it includes one or several sensors which are sensitive to the geometrical distortion of a cardiac or vascular vessel.

26. A device according to claim 25, characterised in that said sensor is a stress gauge arranged on a vessel.

27. A device according to claim 25, characterized in that said sensor includes an ultrasound emitter.

28. A device according to claim 27, characterized in that it includes sensor means which can measure the length of the ultrasound transit in the vascular or cardiac vessel.

29. A device according to any one of claims 25 to 28, characterized in that it comprises means connected to said sensors, which are sensitive to the geometrical distorsion amplitude during a cycle.

30. A device according to any one of claims 25 to 29, characterized in that it comprises means which are sensitive to the time lag in the evolution pattern of the geometrical distorsion curve between two cycles.

31. A device according to claim 14, characterized in that it contains at least one sensor which is sensitive to ion concentration and placed near a zone at risk.

32. A device according to claim 31, characterized in that said ions of said ion concentration are selected from among potassium, H⁺, sodium, chlorine, calcium, magnesium, phosphorus ions.

33. A device according to claim 14, characterized in that it contains at least one sensor which is sensitive to a parameter such as the concentration of oxygen, CO₂, CO, hemoglobin or derivatives, myoglobin, myosin, placed near a zone at risk.

34. A device according to claim 14, characterized in that it includes at least one temperature sensor which is sensitive to the differential temperature near a zone at risk.

35. A device according to any one of claims 1 to 34, characterized in that it contains electrocardiogram acquisition means .

36. A device according to claim 35, characterized in that it contains means which are sensitive to cardiac rhythm.

37. A device according to any one of claims 35 and 36, characterized in that it includes means which are sensitive to the shape of the cardiac electrogenesis and in particular, to the time lag of ST segments.

38. A device according to any one of claims 35 to 37, characterized in that it includes endocavitary and/or intramyocardial electrode means for the electrocardiogram acquisition.

39. A device according to any one of claims 35 to 38, characterized in that it contains means which are sensitive to a cardiac electric abnormality which exceeds a threshold value to trigger the delivery of a drug.

40. A device according to any one of claims 35 to 39, characterized in that it includes electrical detecting electrodes arranged on several geometrical axes for the acquisition of the vectorcardiogram, and means which are sensitive to a change in the vectorcardiogram beyond a threshold to trigger the delivery of a dose of a drug.

41. A device according to claim 14, characterized in that it includes at least one sensor which is sensitive to the spontaneous sounds of the cardiac valves and/or the myocardium.

42. A device according to claim 14, characterized in that it includes means for generating sounds or ultrasounds in the heart or in a vascular vessel, as well as means which are sensitive to the propagation or absorption of said sounds or ultrasounds.

43. A device according to claim 42, characterized in that it includes means generating sounds or ultrasounds with a quick frequency variation on a frequency interval.

44. A device according to claim 42, characterized in that it includes means which are sensitive to the resonance frequency.

45. A device according to claim 13, characterized in that it includes, near said carrier, surface or cavity, electrode means supplied with a current to measure the impedance of the deposit mass or layer.

46. A device according to claim 13, characterized in that it comprises, near said carrier, surface or cavity, light receiving and transmitting means which are sensitive to the thickness of the deposit mass.

47. A device according to claim 13, characterized in that it includes, near the carrier or cavity, sound or ultrasound emitting means and sound or ultrasound sensor means which are sensitive to the resonance of the deposit mass or layer.

48. A device according to claim 13, characterized in that it includes ultrasonic emitting means and ultrasonic receiving means which are sensitive to the resonance corresponding to the blood factor.

49. A device according to claim 14, characterized in that it includes sound or ultrasound transmitting means and a sound or ultrasound sensor, oriented in a vascular or cardiac vessel to transmit sounds or ultrasounds with quick frequency variation and to detect the resonance of the blood column.

50. A device according to claim 49, characterized in that it includes means for triggering the transmission and the measurement at determined moments in the cardiovascular cycle, notably during a systole or a diastole.

51. A device according to claim 14, characterized in that it includes optical means for acquisition of a color or image in the circulating blood or a cardiac or arterial wall or the bone marrow.

52. A device according to claim 51, characterized in that it includes means allowing to count discrete bodies of blood.

53. A device according to claim 14, characterized in that the measurement of said other parameters is made in a periodic manner in association with the cardiac or arterial cycles.

54. A device according to claim 12, characterized in that the parameters are measured several times during a cycle and in that the parameter variation is compared to said threshold means during the cycle.

55. A device according to any one of claims 1 to 13, characterized in that it includes means which are sensitive to the cardiac electrogenesis and/or rhythm, said means being connected to threshold determining means to change the thresholds.

56. A device according to any one of claims 1 to 55, characterized in that it includes means which are sensitive to cardiac electogenesis and/or rhythm, connected to the means for releasing a dose, in order to permit or inhibit said delivery.

57. A device according to any one of claims 1 to 56, characterized in that it includes a cardiac stimulator and/or defibrillator.

58. A device according to claim 57, characterized in that said stimulator is provided with antitachyarrythmic means.

59. A device according to any one of claims 1 to 58, characterised in that it includes, in combination, a portable orthorhythmic electrical stimulator with electrodes (12') allowing the detection of the cardiac rhythm and the transmission of stimulation impulses to the heart, and a portable defibrillator (4') including an energy source (6') and defibrillating electrodes (2', 3'), and means for detecting a quick tachycardia or fibrillation, to actuate said defibrillator, the electronic circuit of the cardiac stimulator being protected in the case of actuation of the defibrillation, as well as detecting means (14') for central and/or arterial hemodynamics which can inhibit the operation of the defibrillator as long as a sufficient hemodynamic operation is detected.

60. A device according to claim 59, characterized in that it includes an external defibrillator including, on a belt (1') or other thoracic harness, at least two defibrillating cutaneous electrodes (2', 3') arranged at opposite ends on the thorax, the opposed electrodes having opposed polarities.

61. A device according to claims 59 and 60, characterized in that the defibrillator includes, outside, one or several cutaneous electrodes having the same polarity, and at least one intra-cardiac electrode having opposed polarity, as well as means for electromagnetic link arranged on each side of the skin to complete the circuit between external and internal electrodes.

62. A device according to any one of claims 60 and 61, characterized in that it includes an energy source for the defibrillation shocks, and comprising one or several capacitors (6') attached to a thoracic harness or belt.

63. A device according to any one of claims 59 to 62, characterized in that it comprises, to provide the energy required for the defibrillation shock, one or several capacitors (6'), a battery (7') which can compensate for the leakage currents of the capacitors as well as a capacitor recharging means or plug (8').

64. A device according to any one of claims 59 to 63, characterized in that arterial and/or central hemodynamic detecting means include at least one intra-cardiac pressure sensor.

65. A device according to any one of claims 59 to 64, characterized in that it includes a thoracic belt comprising self-contracting or motor means which can reduce its circumference and realize a cardiac massage at a frequency in the order of 20 to 60 times/mn, said means being sensitive to the means for detecting the central and/or arterial hemodynamics and/or the gas in the blood.

66. A device according to any one of claims 59 to 65, characterized in that it includes an abdominal strap (31') equipped with contracting means (34') or motor means which can reduce its circumference in order to ensure respiratory assistance.

67. A device according to one of claims 65 and 66, characterized in that said self-contracting or motor means are of an electrostatic and/or electromagnetic type.

68. A device according to any one of claims 59 to 67, characterized in that it includes, around the cardiac muscle near the ventricles, one or several bands (51') which can oppose a progressive expansion of the cardiac cavities.

69. A device according to claim 68, characterized in that said bands (51') are self-contracting (54') and include non self-contrating segments inserted in the cardiac muscle.

70. A device according to any one of claims 1 to 69, characterized in that it includes a left ventricle transapical tube (60) which can be connected to a blood pump leading to aorta.

71. A device according to one of claims 59 to 70, characterized in that the anti-tachycardic means comprise means for selective treatment of isolated or paired extrasystoles by triggering a stimulation following a temporarily quicker rhythm, controlled by the extra-systole itself.

72. A device according to any one of claims 59 to 71, characterized in that said anti-tachycardic means comprise means which can generate a stimulation at a determined frequency, in particular a superior one, in response to a detection of a tachycardic activity.

73. A device according to any one of claims 59 to 72, characterized in that it includes means for the automatic periodic detection of the myocardial excitability threshold by sending preferentially infra-liminal impulses.

74. A device according to any one of claims 59 to 73, characterized in that it includes means which are sensitive to the cardiac rhythm in order to detect a tachycardia, means for sending to the cardiac muscle a paired electrical stimulation, or similarly couple, to a periodic event in the cardiac cycle, means for periodic detection of a cardiac phenomenom linked with the maximum contraction and/or metabolism of the cardiac muscle, and electronic means which are sensitive to said periodic detection means of the cardiac phenomenon and can change the coupling of the stimulation and/or stop said stimulation, as a function of said detection.

75. A device according to claim 74, characterized in that it includes means for detecting a periodic event such as an electrical signal, notably QRS complex or the moment at which the preceding stimulation is sent, or a mechanical or hemodynamic event linked with a systole wave of the mechanogram.

76. A device according to any one of claims 1 to 27, characterized in that it comprises, on an implanted carrier, genetic expressing or translating means of a dose of a therapeutic agent.

## Patentansprüche

1. Vorrichtung zum Schutz vor mit der Blutgerinnung zusammenhängenden Erkrankungen bzw. Störungen wie Thrombose, Embolie, Koagulopathien, Hämorrhagien, Hämopathien und Vorhandensein von anormalen Blutkörperchen, dadurch gekennzeichnet, daß sie
- erste, implantierbare Mittel zum durchgehenden oder periodischen Messen mindestens eines biologischen Parameters besitzt, der vor oder während einer mit der Blutgerinnung zusammenhängenden Störungen auftreten kann, das mindestens ein Mittel besitzt, das in einer Herzkammer oder einem Blut- bzw. Lymphgefäß angebracht werden kann, um dem Blut bzw. der Lymphe ein Element (157) auszusetzen, das so ausgelegt ist, daß es eine örtliche Schwankung des o.a. mindestens einen biologischen Parameters erleichtert, sowie dem o.a. Element zugeordnete Mittel (153, 155) zum Erfassen der Schwankung,
- zweite, implantierbare Mittel (40, 50, 51) besitzt, die einen oder mehrere Schwellenwerte bestimmen, mit denen der o.a. biologische Parameter verglichen wird,
- sowie dritte, implantierbare Mittel (60) besitzt, mit denen ein Überschreiten des bzw. der o.a. Schwellenwerte erfaßt werden kann, um im Blutkreislauf eine Dosis mit einem oder mehreren antikoagulierende Bestandteilen freizusetzen.

2. Vorrichtung nach Patentanspruch 1, dadurch gekennzeichnet, daß die ersten implantierbaren Mittel von einem Katheter (2) gehalten werden.

3. Vorrichtung nach Patentanspruch 2, dadurch gekennzeichnet, daß das o.a. Element (157) mindestens einen Faktor der Blutgerinnung enthält und so ausgelegt ist, daß es von Blut oder Lymphe umgeben ist.

4. Vorrichtung nach Patentanspruch 3, dadurch gekennzeichnet, daß der o.a. Faktor ein Fibrin oder ein Fibrinvorläufer ist.

5. Vorrichtung nach Patentanspruch 2, dadurch gekennzeichnet, daß sie Mittel zum Aussetzen besitzt, die auf leicht voneinander angeordneten Spitzen angebracht sind.

6. Vorrichtung nach Patentanspruch 5, dadurch gekennzeichnet, daß die auf den o.a. Spitzen angebrachten Elemente aus den nachfolgenden ausgewählt sind: Fibrin, Fibrinderivate und - vorläufer, Prothrombin, Prothrombinase, Plasminogen, Plasmin, Protein C, Endothelin, Serotonin, Katecholamine, Neuropeptide Y, Plasminogenaktivatoren oder -inhibitoren.

7. Vorrichtung nach Patentanspruch 2 bis 5, dadurch gekennzeichnet, daß die o.a. Elemente auf einem unlöslichen Träger angebracht sind.

8. Vorrichtung nach Patentanspruch 1 bis 7, dadurch gekennzeichnet, daß die o.a. Mittel einen auf Calcium und/oder H⁺-Ionen ansprechenden Meßfühler besitzen.

9. Vorrichtung nach Patentanspruch 1 bis 7, dadurch gekennzeichnet, daß die o.a. ersten Mittel einen optischen Detektor besitzen.

10. Vorrichtung nach Patentanspruch 1 bis 7, dadurch gekennzeichnet, daß die o.a. Mittel einen auf elektrische Impedanz ansprechenden Meßfühler besitzen.

11. Vorrichtung nach Patentanspruch 2 bis 7, dadurch gekennzeichnet, daß die o.a. ersten Mittel auf die Schwankung von lokalen Koagulationsfaktoren ansprechen, die sich je nach den lokalen elektrischen Feldern oder Strömen ändern.

12. Vorrichtung nach Patentanspruch 1 bis 11, dadurch gekennzeichnet, daß sie Mittel (4) zur Erfassung des Herzrhythmus oder des Pulses besitzt, die das Erfassen der Meßwerte des biologischen Parameters an vorgegebenen Zeitpunkten des Herzzyklus befehlen.

13. Vorrichtung nach Patentanspruch 2 bis 4, dadurch gekennzeichnet, daß die o.a. ersten Mittel einen Meßfühler besitzen, der auf die Stärke der Ablagerungen auf dem o.a. Element (157) ansprechen.

14. Vorrichtung nach Patentanspruch 1 bis 13, dadurch gekennzeichnet, daß sie weiterhin Mittel besitzt, die auf andere generelle oder lokale biologische Parameter ansprechen.

15. Vorrichtung nach Patentanspruch 14, dadurch gekennzeichnet, daß sie Mittel (14, 15) zum Messen der Impedanz in einem Blutgefäß besitzt.

16. Vorrichtung nach Patentanspruch 14, dadurch gekennzeichnet, daß die Mittel (14, 15) in einer Arterie, insbesondere einer Koronararterie oder darum herum angebracht sind.

17. Vorrichtung nach Patentanspruch 15, dadurch gekennzeichnet, daß die o.a. Mittel im Myokard angebracht sind.

18. Vorrichtung nach Patentanspruch 15 bis 17, dadurch gekennzeichnet, daß sie eine elektrischen Stromquelle (30) besitzt, die an Elektroden (14, 15) angeschlossen ist, die zur Messung einer elektrischen Impedanz bei mindestens einer Frequenz im Bereich zwischen einigen kHz und mehreren MHz dienen.

19. Vorrichtung nach Patentanspruch 15 bis 18, dadurch gekennzeichnet, daß sie Mittel (40) besitzt, die auf Unterschiede in der gemessenen Impedanz ansprechen.

20. Vorrichtung nach Patentanspruch 19, dadurch gekennzeichnet, daß sie Mittel (40) auf den Unterschied zwischen maximaler und minimaler Impedanz ansprechen, die bei ein und demselben Herzzyklus gemessen werden.

21. Vorrichtung nach Patentanspruch 15 bis 20, dadurch gekennzeichnet, daß sie Mittel (40) besitzt, die auf die Verschiebung bei Abweichungen der Impedanzmeßkurve ansprechen, die zwischen zwei aufeinanderfolgenden Zyklen entstehen kann.

22. Vorrichtung nach Patentanspruch 15 bis 21, dadurch gekennzeichnet, daß sie Elektroden besitzt, die im Herzmuskel oder abgelegen davon angebracht sind, um unterschiedliche Meßachsen zu bilden und das Vektogramm der elektrischen Impedanzschwankungen des Herzens zu erfassen.

23. Vorrichtung nach Patentanspruch 14, dadurch gekennzeichnet, daß sie mindestens einen auf den Blut- oder Myokarddruck ansprechenden Druckfühler besitzt.

24. Vorrichtung nach Patentanspruch 23, dadurch gekennzeichnet, daß sie mindestens zwei Druckfühler besitzt, die einem Blutgefäß oder einer Herzkammer entlang angebracht sind, sowie Mittel, die auf die erfaßten Druckwerte und/oder die Verschiebung zwischen der von diesen Druckfühlern erfaßten Druckwerten ansprechen.

25. Vorrichtung nach Patentanspruch 14, dadurch gekennzeichnet, daß sie einen oder mehrere Meßfühler besitzt, die auf die geometrische Verformung eines Blutgefäßes oder einer Herzkammer ansprechen.

26. Vorrichtung nach Patentanspruch 25, dadurch gekennzeichnet, daß der o.a. Meßfühler ein in einem Blutgefäß angebrachter Dehnungsmesser ist.

27. Vorrichtung nach Patentanspruch 15, dadurch gekennzeichnet, daß der o.a. Meßfühler einen Ultraschallsender besitzt.

28. Vorrichtung nach Patentanspruch 27, dadurch gekennzeichnet, daß sie Meßfühler besitzt, die in der Lage sind, die Dauer des Durchdringens von Ultraschallwellen in Blutgefäßen oder Herzkammern zu messen.

29. Vorrichtung nach Patentanspruch 25 bis 28, dadurch gekennzeichnet, daß sie mit den o.a. Meßfühlern verbundene Mittel besitzt, die auf die Amplitude der geometrischen Verformung während eines Zyklus anspricht.

30. Vorrichtung nach Patentanspruch 25 bis 29, dadurch gekennzeichnet, daß sie Mittel besitzt, die auf die Verschiebung bei Abweichungen der geometrischen Verformungskurve zwischen zwei Zyklen anspricht.

31. Vorrichtung nach Patentanspruch 14, dadurch gekennzeichnet, daß sie mindestens einen Meßfühler besitzt, der auf die Ionenkonzentration anspricht und der in der Nähe einer Risikozone angebracht ist.

32. Vorrichtung nach Patentanspruch 31, dadurch gekennzeichnet, daß die Ionen der o.a. Ionenkonzentration Kalium-, H⁺-, Natrium-, Chlor-, Calcium-, Magnesium- und/oder Phosphorionen sein können.

33. Vorrichtung nach Patentanspruch 14, dadurch gekennzeichnet, daß sie mindestens einen Meßfühler besitzt, der auf einen der Parameter Sauerstoff-, CO₂-, CO-Gehalt, Hämoglobin oder Hämoglobinderivate, Myoglobin, Myosin anspricht und der in der Nähe einer Risikozone angebracht ist.

34. Vorrichtung nach Patentanspruch 14, dadurch gekennzeichnet, daß sie mindestens einen Temperaturfühler besitzt, der auf die Differentialtemperatur in der Nähe einer Risikozone anspricht.

35. Vorrichtung nach Patentanspruch 34, dadurch gekennzeichnet, daß sie Mittel zur Aufnahme eines Elektrokardiogramms besitzt.

36. Vorrichtung nach Patentanspruch 35, dadurch gekennzeichnet, daß sie Mittel besitzt, die auf den Herzrhythmus ansprechen.

37. Vorrichtung nach Patentanspruch 35 und 36, dadurch gekennzeichnet, daß sie Mittel besitzt, die auf die Form der kardialen Elektrogenese und insbesondere auf die Verschiebung der ST-Segmente ansprechen.

38. Vorrichtung nach Patentanspruch 35 bis 37, dadurch gekennzeichnet, daß sie endokavitäre und/oder intramyokardiale Elektroden zum Erfassen eines Elektrokardiogramms besitzt.

39. Vorrichtung nach Patentanspruch 35 bis 38, dadurch gekennzeichnet, daß sie Mittel besitzt, die auf eine elektrische Herzanomalie ansprechen, die einen bestimmten Schwellenwert überschreitet, um die Freisetzung eines Medikamentes auszulösen.

40. Vorrichtung nach Patentanspruch 35 bis 39, dadurch gekennzeichnet, daß sie Elektroden zur elektrischen Erfassung besitzt, die zur Erfassung des Vektokardiogramms auf mehreren geometrischen Achsen angeordnet sind, sowie Mittel, die auf eine Veränderung des Vektokardiogramms ansprechen, die einen bestimmten Schwellenwert überschreitet, um die Freisetzung eines Medikamentes auszulösen.

41. Vorrichtung nach Patentanspruch 14, dadurch gekennzeichnet, daß sie mindestens einen Meßfühler besitzt, der auf die spontanen Geräusche der Herzklappen und/oder des Myokards anspricht.

42. Vorrichtung nach Patentanspruch 14, dadurch gekennzeichnet, daß sie Mittel zum Erzeugen von Schall- oder Ultraschallwellen im Herz oder in einem Blutgefäß sowie Mittel zur Fortpflanzung oder Absorption der Schall- bzw. Ultraschallwellen besitzt.

43. Vorrichtung nach Patentanspruch 42, dadurch gekennzeichnet, daß sie Mittel zum Erzeugen von Schall- bzw. Ultraschallwellen unter schneller Frequenzänderung in einem bestimmten Frequenzbereich besitzt.

44. Vorrichtung nach Patentanspruch 42, dadurch gekennzeichnet, daß sie Mittel besitzt, die auf die Resonanzfrequenz ansprechen.

45. Vorrichtung nach Patentanspruch 13, dadurch gekennzeichnet, daß sie in der Nähe des Trägers, der Unterlage oder der Aushöhlung mit Spannung versorgte Elektroden zum Messen der Impedanz der Masse bzw. Schicht der Ablagerung besitzt.

46. Vorrichtung nach Patentanspruch 13, dadurch gekennzeichnet, daß sie in der Nähe des Trägers, der Unterlage oder der Aushöhlung Mittel zum Senden und Empfangen von Lichtwellen besitzt, die auf die Stärke der Ablagerungsschicht ansprechen.

47. Vorrichtung nach Patentanspruch 13, dadurch gekennzeichnet, daß sie in der Nähe des Trägers, der Unterlage oder der Aushöhlung Mittel zum Senden und Mittel zum Erfassen von Schall- bzw. Ultraschallwellen besitzt, die auf die Resonanz der Schicht oder der Masse der Ablagerung ansprechen.

48. Vorrichtung nach Patentanspruch 13, dadurch gekennzeichnet, daß sie Mittel zum Senden und Empfangen von Ultraschallwellen besitzt, die auf die Resonanzfrequenz ansprechen, die einem Faktor in dem Blut entspricht.

49. Vorrichtung nach Patentanspruch 14, dadurch gekennzeichnet, daß sie Mittel zum Senden und Mittel zum Erfassen von Schall- bzw. Ultraschallwellen besitzt, die in einem Blutgefäß oder in einer Herzkammer so ausgerichtet sind, daß Schall- bzw. Ultraschallwellen mit hoher Frequenzänderung ausgestrahlt werden, daß eine Erfassung der Resonanzfrequenz im Blutgefäßbaum möglich ist.

50. Vorrichtung nach Patentanspruch 49, dadurch gekennzeichnet, daß sie Mittel zum Auslösen des Sendens und Messens zu bestimmten Zeitpunkten des kardiovaskulären Zyklus, insbesondere während einer Systole oder Diastole besitzt.

51. Vorrichtung nach Patentanspruch 14, dadurch gekennzeichnet, daß sie optische Mittel zum Erfassen einer Farbe oder eines Bildes im zirkulierenden Blut, in einer Herz- oder Blutgefäßwand oder im Knochenmark besitzt.

52. Vorrichtung nach Patentanspruch 51, dadurch gekennzeichnet, daß sie Mittel zum Zählen von Blutkörperchen besitzt.

53. Vorrichtung nach Patentanspruch 14, dadurch gekennzeichnet, daß die Messung der übrigen Parameter periodisch entsprechend den kardialen bzw. arteriellen Zyklen erfolgt.

54. Vorrichtung nach Patentanspruch 12, dadurch gekennzeichnet, daß die Parameter mehrmals während eines Zyklus gemessen werden und die Schwankung der Parameterwerte während eines Zyklus mit vorgegebenen Schwellenwerten verglichen werden.

55. Vorrichtung nach Patentanspruch 13, dadurch gekennzeichnet, daß sie Mittel besitzt, die auf den Herzrhythmus und/oder die Herzelektrogenese ansprechen und mit Mitteln zum Bestimmen von Schwellenwerten verbunden sind, um diese Schwellenwerte zu verändern.

56. Vorrichtung nach Patentanspruch 55, dadurch gekennzeichnet, daß sie Mittel besitzt, die auf den Herzrhythmus und/oder die Herzelektrogenese ansprechen und mit Mitteln zum Freisetzen einer Dosis verbunden sind, um diese Freisetzung zu genehmigen oder zu verhindern.

57. Vorrichtung nach Patentanspruch 1 bis 56, dadurch gekennzeichnet, daß sie einen Herzschrittmacher und/oder einen Defibrillator besitzt.

58. Vorrichtung nach Patentanspruch 57, dadurch gekennzeichnet, daß der Herzschrittmacher antitachyarhythmische Mittel besitzt.

59. Vorrichtung nach Patentanspruch 1 bis 58, dadurch gekennzeichnet, daß sie in kombinierter Form einen tragbaren orthorhythmischen elektrischen Schrittmacher mit Elektroden (12'), mit denen der Herzrhythmus gemessen und Stimulationsimpulse an den Herzmuskel übertragen werden können, einen tragbaren Defibrillator (4') mit einer Energiequelle (6') und Defibrillationselektroden (2', 3') und Mittel zum Erkennen von Kammerflimmern oder schneller Tachykardie besitzt, um den Defibrillator auszulösen, wobei der Herzschrittmacher beim Auslösen des Defibrillators geschützt wird, sowie Mittel (14') zum Erkennen der zentralen und/oder arteriellen Hämodynamik, mit denen die Möglichkeit besteht, den Defibrillator ausgeschaltet zu lassen, solange eine ausreichende Hämodynamik festgestellt wird.

60. Vorrichtung nach Patentanspruch 59, dadurch gekennzeichnet, daß sie einen externen Defibrillator besitzt, der in einem Brustgurt (1') oder Brustgeschirr mindestens zwei kutane Defibrillationselektroden (2', 3') aufweist, die sich jeweils am Brustkorb gegenüberliegen und jeweils eine entgegengesetzte Polarität besitzen.

61. Vorrichtung nach Patentanspruch 59 und 60, dadurch gekennzeichnet, daß der Defibrillator außen eine oder mehrere kutane Elektroden gleicher Polarität und mindestens eine intrakardiale Elektrode entgegengesetzter Polarität sowie beidseitig der Haut angebrachte Mittel zur elektromagnetischen Verbindung besitzt, mit denen der Stromkreis zwischen den äußeren und inneren Elektroden geschlossen wird.

62. Vorrichtung nach Patentanspruch 60 und 61, dadurch gekennzeichnet, daß sie eine Stromquelle für die Defibrillationsschocks besitzt, die aus einem oder mehreren an einem Brustgurt oder Brustgeschirr angebrachten Kondensatoren (6') besteht.

63. Vorrichtung nach Patentanspruch 59 bis 62, dadurch gekennzeichnet, daß sie zum Aufbringen der für den Defibrillationsschock erforderlichen Energie einen oder mehrere Kondensatoren (6'), eine Batterie (7') zum Ausgleichen des Kriechstroms des bzw. der Kondensatoren sowie ein Mittel oder einen Steckverbinder (8') zum Nachladen des bzw. der Kondensatoren besitzt.

64. Vorrichtung nach Patentanspruch 59 bis 63, dadurch gekennzeichnet, daß die Mittel zum Erkennen der zentralen und/oder arteriellen Hämodynamik mindestens einen intrakardialen Druckfühler besitzen.

65. Vorrichtung nach Patentanspruch 59 bis 64, dadurch gekennzeichnet, daß sie einen Brustgurt kontraktilen Elementen oder Antriebselementen besitzt, durch die Länge des Gurtes verringert werden und eine Herzmassage bei einer Frequenz von 20 - 60 Impulsen pro Minute vorgenommen werden kann, wobei diese Mittel auf die Erfassung der zentralen und/oder arteriellen Hämodynamik und/oder von Blutgasen ansprechen.

66. Vorrichtung nach Patentanspruch 59 bis 65, dadurch gekennzeichnet, daß sie einen Bauchgurt (31') mit kontraktilen Mitteln (34') oder Antriebselementen besitzt, durch die Länge des Gurtes verringert werden kann, um die Atmung des Patienten zu unterstützen.

67. Vorrichtung nach Patentanspruch 65 und 66, dadurch gekennzeichnet, daß die kontraktilen Mittel oder Antriebselemente elektrostatischer oder elektromagnetischer Art sind.

68. Vorrichtung nach Patentanspruch 59 bis 67, dadurch gekennzeichnet, daß sie in Höhe der Ventrikel um den Herzmuskel herum ein oder mehrere Bänder (51') besitzt, die einer fortschreitenden Ausdehnung der Herzkammern entgegenwirken.

69. Vorrichtung nach Patentanspruch 68, dadurch gekennzeichnet, daß die Bänder (51') kontraktil sind und nicht kontraktile Teile besitzen, die in den Herzmuskel eingesetzt sind.

70. Vorrichtung nach Patentanspruch 1 bis 69, dadurch gekennzeichnet, daß sie einen transapikalen Tubus (60) im linken Ventrikel besitzt, der an eine in die Aorta einmündende Blutpumpe angeschlossen ist.

71. Vorrichtung nach Patentanspruch 59 bis 70, dadurch gekennzeichnet, daß die antitachyrhythmischen Mittel Mittel zum selektiven Behandeln von einzelnen und gepaarten Extrasystolen durch Auslösen nachfolgenden Stimulation mit einem zeitweilig schnelleren Rhythmus besitzen, wobei der Stimulationsimpuls von der Extrasystole selbst gesteuert wird.

72. Vorrichtung nach Patentanspruch 59 bis 71, dadurch gekennzeichnet, daß die antitachyrhythmischen Mittel Mittel zum Erzeugen einer Stimulation bei vorgegebener, vorzugsweise höherer Frequenz als Reaktion auf die Erkennung einer tachykardialen Aktivität besitzen.

73. Vorrichtung nach Patentanspruch 59 bis 72, dadurch gekennzeichnet, daß sie Mittel zur periodische automatische Erfassung der Erregbarkeitsschwelle des Myokardes durch Senden von vorzugsweise unter der Schwelle liegenden Impulsen besitzt.

74. Vorrichtung nach Patentanspruch 59 bis 73, dadurch gekennzeichnet, daß sie Mittel zur Erkennung einer Tachykardie und Mittel zum Beschicken des Herzmuskels mit einem gepaarten oder auf ähnliche Art mit einem periodisch auftretenden Ereignis des Herzzyklus gekoppelten elektrischen Stimulationsimpuls, weiterhin Mittel zum periodischen Erkennen eines kardialen Ereignisses in Verbindung mit der maximalen Kontraktion und/oder dem Metabolismus des Herzmuskels, sowie elektronische Mittel, die auf die o.a. Mittel zur periodischen Erfassung des kardialen Ereignisses reagieren und je nach Funktion der o.a. Erfassung die Kopplung der Stimulation verändern und/oder diese unterbrechen können, besitzt.

75. Vorrichtung nach Patentanspruch 74, dadurch gekennzeichnet, daß sie Mittel zum Erkennen eines periodischen Ereignisses wie zum Beispiel eines elektrischen Signals und insbesondere des QRS-Komplexes oder des Zeitpunktes der Übertragung der vorhergehenden Stimulation oder eines mit der systolischen Kurve des Mechanogramms zusammenhängenden mechanischen oder hämodynamischen Ereignisses besitzt.

76. Vorrichtung nach Patentanspruch 1 bis 27, dadurch gekennzeichnet, daß sie auf einem implantierten Träger Mittel zur Expression oder genetischen Umsetzung einer Dosis eines therapeutischen Mittels besitzt.
